(19) Europäisches Patentamt
European Patent Office
Office européen des brevets

(11) EP 1 395 550 B1

(12) **EUROPEAN PATENT SPECIFICATION**

(45) Date of publication and mention of the grant of the patent:
**29.06.2005 Bulletin 2005/26**

(21) Application number: **02733989.4**

(22) Date of filing: **17.05.2002**

(51) Int Cl.[7]: **C07C 311/07**, C07C 311/08, C07C 307/08, A61K 31/18, A61P 25/00

(86) International application number:
**PCT/US2002/011895**

(87) International publication number:
**WO 2002/098847 (12.12.2002 Gazette 2002/50)**

# (54) CYCLOALKENYLSULFONAMIDE DERIVATIVES

ZYKLOALKENYLSULFONAMIDDERIVATE

DERIVES DE CYCLOALCENYLSULFONAMIDE

(84) Designated Contracting States:
**AT BE CH CY DE DK ES FI FR GB GR IE IT LI LU MC NL PT SE TR**

(30) Priority: **30.05.2001 US 294428 P**

(43) Date of publication of application:
**10.03.2004 Bulletin 2004/11**

(73) Proprietor: **ELI LILLY AND COMPANY**
**Indianapolis, Indiana 46285 (US)**

(72) Inventors:
- **CANTRELL, Buddy, Eugene**
  **Zionsville, IN 46077 (US)**
- **ZIMMERMAN, Dennis, Michael**
  **Zionsville, IN 46077 (US)**

(74) Representative: **Kingsbury, Oliver William**
**Eli Lilly and Company Limited,**
**Lilly Research Centre, Erl Wood Manor,**
**Sunninghill Road**
**Windlesham, Surrey GU20 6PH (GB)**

(56) References cited:
**EP-A- 0 860 428**
**WO-A-00/06158**
**WO-A-00/06539**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to the potentiation of glutamate receptor function using certain cyclalkenylsulfonamide derivatives. It also relates to novel sulfonamide derivatives, to processes for their preparation and to pharmaceutical compositions containing them.

In the mammalian central nervous system (CNS), the transmission of nerve impulses is controlled by the interaction between a neurotransmitter, that is released by a sending neuron, and a surface receptor on a receiving neuron, which causes excitation of this receiving neuron. L-Glutamate, which is the most abundant neurotransmitter in the CNS, mediates the major excitatory pathway in mammals, and is referred to as an excitatory amino acid (EAA). The receptors that respond to glutamate are called excitatory amino acid receptors (EAA receptors). See Watkins & Evans, *Ann. Rev. Pharmacol. Toxicol.,* 21, 165 (1981); Monaghan, Bridges, and Cotman, *Ann. Rev. Pharmacol. Toxicol.*, 29, 365 (1989); Watkins, Krogsgaard-Larsen, and Honore, *Trans. Pharm. Sci.,* 11, 25 (1990). The excitatory amino acids are of great physiological importance, playing a role in a variety of physiological processes, such as long-term potentiation (learning and memory), the development of synaptic plasticity, motor control, respiration, cardiovascular regulation, and sensory perception.

Excitatory amino acid receptors are classified into two general types. Receptors that are directly coupled to the opening of cation channels in the cell membrane of the neurons are termed "ionotropic". This type of receptor has been subdivided into at least three subtypes, which are defined by the depolarizing actions of the selective agonists *N*-methyl-D-aspartate (NMDA), alpha-amino-3-hydroxy-5-methylisoxazole-4-propionic acid (AMPA), and kainic acid (KA). The second general type of receptor is the G-protein or second messenger-linked "metabotropic" excitatory amino acid receptor. This second type is coupled to multiple second messenger systems that lead to enhanced phosphoinositide hydrolysis, activation of phospholipase D, increases or decreases in c-AMP formation, and changes in ion channel function. Schoepp and Conn, *Trends in Pharmacol. Sci.,* 14, 13 (1993). Both types of receptors appear not only to mediate normal synaptic transmission along excitatory pathways, but also participate in the modification of synaptic connections during development and throughout life. Schoepp, Bockaert, and Sladeczek, *Trends in Pharmacol. Sci.,* 11, 508 (1990); McDonald and Johnson, *Brain Research Reviews,* 15, 41 (1990).

AMPA receptors are assembled from four protein sub-units known as GluR1 to GluR4, while kainic acid receptors are assembled from the sub-units GluR5 to GluR7, and KA-1 and KA-2. Wong and Mayer, *Molecular Pharmacology* 44: 505-510, 1993. It is not yet known how these sub-units are combined in the natural state. However, the structures of certain human variants of each sub-unit have been elucidated, and cell lines expressing individual sub-unit variants have been cloned and incorporated into test systems designed to identify compounds which bind to or interact with them, and hence which may modulate their function. Thus, European patent application, publication number EP-A2-0574257 discloses the human sub-unit variants GluR1B, GluR2B, GluR3A and GluR3B. European patent application, publication number EP-A1-0583917 discloses the human sub-unit variant GluR4B.

One distinctive property of AMPA and kainic acid receptors is their rapid deactivation and desensitization to glutamate. Yamada and Tang, *The Journal of Neuroscience*, September 1993, 13(9): 3904-3915 and Kathryn M. Partin, *J. Neuroscience*, November 1, 1996, 16(21): 6634-6647. The physiological implications of rapid desensitization, and deactivation if any, are not fully understood.

It is known that the rapid desensitization and deactivation of AMPA and/or kainic acid receptors to glutamate may be inhibited using certain compounds.
This action of these compounds is often referred to in the alternative as "potentiation" of the receptors. One such compound, which selectively potentiates AMPA receptor function, is cyclothiazide. Partin et al., *Neuron.* Vol. 11, 1069-1082, 1993.

AMPA receptor potentiators have been shown to improve memory in a variety of animal tests. Staubli *et al.*, *Proc. Natl. Acad. Sci.,* Vol. 91, pp 777-781, 1994, *Neurobiology,* and Arai *et al., The Journal of Pharmacology and Experimental Therapeutics,* 278: 627-638, 1996.

In addition, certain sulfonamide derivatives which potentiate glutamate receptor function in a mammal have been disclosed in the following International Patent Application Publications: WO 98/33496 published August 6, 1998; WO 99/43285 published September 2, 1999; WO 00/06539; WO 00/06537, WO 00/06176, WO 00/06159, WO 00/06158, WO 00/06157, WO 00/06156, WO 00/06149, WO 00/06148, and WO 00/06083, all published February 10, 2000; WO 00/66546 published November 9, 2000, WO 01/00056, published November 29, 2001, and WO 01/42203, published June 14, 2001

The present invention provides compounds of formula I:

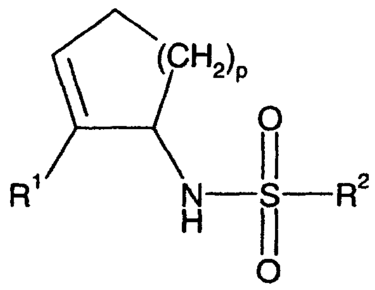

formula I

wherein

p represents integer 1 or 2;

$R^1$ represents an unsubstituted or substituted aromatic group, or an unsubstituted or substituted heteroaromatic group; and

$R^2$ represents (1-6C)alkyl, (3-6C)cycloalkyl, fluoro(1-6C)alkyl, chloro(1-6C)alkyl, (2-6C)alkenyl, phenyl which is unsubstituted or substituted by halogen, (1-4C)alkyl or (1-4C)alkoxy, or a group of formula $R^3R^4N$ in which $R^3$ and R4 each independently represents (1-4C)alkyl or, together with the nitrogen atom to which they are attached form an azetidinyl, pyrrolidinyl, piperidinyl, morpholino, piperazinyl, hexahydroazepinyl or octahydroazocinyl group;

or a pharmaceutically acceptable salt thereof.

According to another aspect, the present invention provides the use of a compound of formula I, or a pharmaceutically acceptable salt thereof for the manufacture of a medicament for treating cognitive disorders.

According to another aspect, the present invention provides the use of a compound of formula I, or a pharmaceutically acceptable salt thereof for the manufacture of a medicament for treating depression.

According to another aspect, the present invention provides the use of a compound of formula I, or a pharmaceutically acceptable salt thereof for the manufacture of a medicament for treating Alzheimer's disease.

According to another aspect, the present invention provides the use of a compound of formula I, or a pharmaceutically acceptable salt thereof for the manufacture of a medicament for treating psychosis or cognitive deficits associated with psychosis.

In addition, the present invention provides the use of a compound of formula I or a pharmaceutically acceptable salt thereof for potentiating glutamate receptor function.

The invention further provides pharmaceutical compositions comprising, a compound of formula I and a pharmaceutically acceptable diluent or carrier.

This invention also encompasses novel intermediates and processes for the synthesis of the compounds of formula I.

In this specification, the term "potentiating glutamate receptor function" refers to any increased responsiveness of glutamate receptors, for example AMPA receptors, to glutamate or an agonist, and includes but is not limited to inhibition of rapid desensitization or deactivation of AMPA receptors to glutamate.

A wide variety of conditions may be treated or prevented by compounds of formula I and their pharmaceutically acceptable salts through their action as potentiators of glutamate receptor function. Such conditions include those associated with glutamate hypofunction, such as psychiatric and neurological disorders, for example cognitive disorders and neuro-degenerative disorders such as Alzheimer's disease; age-related dementias; age-induced memory impairment; cognitive deficits due to autism, Down's syndrome and other central nervous system disorders with childhood onset, cognitive deficits post electroconvulsive therapy, movement disorders such as tardive dyskinesia, Huntington's chorea, myoclonus, dystonia, spasticity, and Parkinson's disease; reversal of drug-induced states (such as cocaine, amphetamines, alcohol-induced states); depression; attention deficit disorder; attention deficit hyperactivity disorder; psychosis; cognitive deficits associated with psychosis, drug-induced psychosis, chemotherapy-induced cognitive deficits, obesity, stroke, and sexual dysfunction. Compounds of formula I may also be useful, for improving memory (both short term and long term) and learning ability. The present invention provides compounds of formula I for use in the treatment of each of these conditions.

The present invention includes the pharmaceutically acceptable salts of the compounds defined by formula I. A compound of this invention can possess a sufficiently acidic group, a sufficiently basic group, or both functional groups, and accordingly react with any of a number of organic and inorganic bases, and inorganic and organic acids, to form a pharmaceutically acceptable salt.

The term "pharmaceutically acceptable salt" as used herein, refers to salts of the compounds of the above formula which are substantially non-toxic to living organisms. Typical pharmaceutically acceptable salts include those salts prepared by reaction of the compounds of the present invention with a pharmaceutically acceptable mineral or organic acid or an organic or inorganic base. Such salts are known as acid addition and base addition salts. Such salts include

the pharmaceutically acceptable salts listed in Journal of Pharmaceutical Science, 66, 2-19 (1977), which are known to the skilled artisan.

Acids commonly employed to form acid addition salts are inorganic acids such as hydrochloric acid, hydrobromic acid, hydroiodic acid, sulfuric acid, phosphoric acid, and the like, and organic acids such as *p*-toluenesulfonic, methanesulfonic acid, benzenesulfonic acid, oxalic acid, *p*-bromophenylsulfonic acid, carbonic acid, succinic acid, citric acid, benzoic acid, acetic acid, and the like. Examples of such pharmaceutically acceptable salts are the sulfate, pyrosulfate, bisulfate, sulfite, bisulfite, phosphate, monohydrogenphosphate, dihydrogenphosphate, metaphosphate, pyrophosphate, bromide, iodide, acetate, propionate, decanoate, caprate, caprylate, acrylate, ascorbate, formate, hydrochloride, dihydrochloride, isobutyrate, caproate, heptanoate, propiotate, propionate, phenylpropionate, salicylate, oxalate, malonate, succinate, suberate, sebacate, fumarate, malate, maleate, hydroxymaleate, mandelate, nicotinate, isonicotinate, cinnamate, hippurate, nitrate, phthalate, teraphthalate, butyne-1,4-dioate, butyne-1,4-dicarboxylate, hexyne-1,4-dicarboxylate, hexyne-1,6-dioate, benzoate, chlorobenzoate, methylbenzoate, hydroxybenzoate, methoxybenzoate, dinitrobenzoate, *o*-acetoxybenzoate, naphthalene-2-benzoate, phthalate, *p*-toluenesulfonate, *p*-bromobenzenesulfonate, p-chlorobenzenesulfonate, xylenesulfonate, phenylacetate, trifluoroacetate, phenylpropionate, phenylbutyrate, citrate, lactate, α-hydroxybutyrate, glycolate, tartrate, benzenesulfonate, methanesulfonate, ethanesulfonate, propanesulfonate, hydroxyethanesulfonate, 1-naphthalenesulfonate, 2-napththalenesulfonate, 1,5-naphthalenedisulfonate, mandelate, tartarate, and the like. Preferred pharmaceutically acceptable acid addition salts are those formed with mineral acids such as hydrochloric acid and hydrobromic acid, and those formed with organic acids such as maleic acid, oxalic acid and methanesulfonic acid.

Base addition salts include those derived from inorganic bases, such as ammonium or alkali or alkaline earth metal hydroxides, carbonates, bicarbonates, and the like. Such bases useful in preparing the salts of this invention thus include sodium hydroxide, potassium hydroxide, ammonium hydroxide, potassium carbonate, sodium carbonate, sodium bicarbonate, potassium bicarbonate, calcium hydroxide, calcium carbonate, and the like. The potassium and sodium salt forms are particularly preferred.

It should be recognized that the particular counterion forming a part of any salt of this invention is usually not of a critical nature, so long as the salt as a whole is pharmacologically acceptable and as long as the counterion does not contribute undesired qualities to the salt as a whole. It is further understood that the above salts may form hydrates or exist in a substantially anhydrous form.

As used herein, the term "stereoisomer" refers to a compound made up of the same atoms bonded by the same bonds but having different three-dimensional structures which are not interchangeable. The three-dimensional structures are called configurations. As used herein, the term "enantiomer" refers to two stereoisomers whose molecules are nonsuperimposable mirror images of one another. The term "chiral center" refers to a carbon atom to which four different groups are attached. As used herein, the term "diastereomers" refers to stereoisomers which are not enantiomers. In addition, two diastereomers which have a different configuration at only one chiral center are referred to herein as "epimers". The terms "racemate", "racemic mixture" or "racemic modification" refer to a mixture of equal parts of enantiomers.

The term "enantiomeric enrichment" as used herein refers to the increase in the amount of one enantiomer as compared to the other. A convenient method of expressing the enantiomeric enrichment achieved is the concept of enantiomeric excess, or "ee", which is found using the following equation:

$$ee = \frac{E^1 - E^2}{E^1 + E^2} X\ 100$$

wherein $E^1$ is the amount of the first enantiomer and $E^2$ is the amount of the second enantiomer. Thus, if the initial ratio of the two enantiomers is 50:50, such as is present in a racemic mixture, and an enantiomeric enrichment sufficient to produce a final ratio of 50:30 is achieved, the ee with respect to the first enantiomer is 25%. However, if the final ratio is 90:10, the ee with respect to the first enantiomer is 80%. An ee of greater than 90% is preferred, an ee of greater than 95% is most preferred and an ee of greater than 99% is most especially preferred. Enantiomeric enrichment is readily determined by one of ordinary skill in the art using standard techniques and procedures, such as gas or high performance liquid chromatography with a chiral column. Choice of the appropriate chiral column, eluent and conditions necessary to effect separation of the enantiomeric pair is well within the knowledge of one of ordinary skill in the art. In addition, the specific stereoisomers and enantiomers of compounds of formula I can be prepared by one of ordinary skill in the art utilizing well known techniques and processes, such as those disclosed by J. Jacques, et al., "Enantiomers, Racemates, and Resolutions", John Wiley and Sons, Inc., 1981, and E.L. Eliel and S.H. Wilen," Stereochemistry of Organic Compounds", (Wiley-Interscience 1994), and European Patent Application No. EP-A-838448, published April 29, 1998. Examples of resolutions include recrystallization techniques or chiral chromatography.

Some of the compounds of the present invention have one or more chiral centers and may exist in a variety

of stereoisomeric configurations. As a consequence of these chiral centers, the compounds of the present invention occur as racemates, mixtures of enantiomers and as individual enantiomers, as well as diastereomers and mixtures of diastereomers. All such racemates, enantiomers, and diastereomers are within the scope of the present invention.

The terms "R" and "S" are used herein as commonly used in organic chemistry to denote specific configuration of a chiral center. The term "R" (rectus) refers to that configuration of a chiral center with a clockwise relationship of group priorities (highest to second lowest) when viewed along the bond toward the lowest priority group. The term "S" (sinister) refers to that configuration of a chiral center with a counterclockwise relationship of group priorities (highest to second lowest) when viewed along the bond toward the lowest priority group. The priority of groups is based upon their atomic number (in order of decreasing atomic number). A partial list of priorities and a discussion of stereochemistry is contained in "Nomenclature of Organic Compounds: Principles and Practice". (J.H. Fletcher, et al., eds., 1974) at pages 103-120.

As used herein, the term "aromatic group" means the same as aryl, and includes phenyl and a polycyclic aromatic carbocyclic ring such as 1- or 2-naphthyl, 1,2-dihydronaphthyl, 1,2,3,4-tetrahydronaphthyl, and the like.

The term "heteroaromatic group" includes an aromatic 5-6 membered ring containing from one to four heteroatoms selected from oxygen, sulfur and nitrogen, and a bicyclic group consisting of a 5-6 membered ring containing from one to four heteroatoms selected from oxygen, sulfur and nitrogen fused with a benzene ring or another 5-6 membered ring containing one to four atoms selected from oxygen, sulfur and nitrogen. Examples of heteroaromatic groups are thienyl, furyl, oxazolyl, isoxazolyl, oxadiazoyl, pyrazolyl, thiazolyl, thiadiazolyl, isothiazolyl, imidazolyl, triazolyl, tetrazolyl, pyridyl, pyridazinyl, pyrimidyl, benzofuryl, benzothienyl, benzimidazolyl, benzoxazolyl, benzothiazolyl, indolyl, and quinolyl.

The term "substituted" as used in the term "substituted aromatic or heteroaromatic group" herein signifies that one or more (for example one or two) substituents may be present, said substituents being selected from atoms and groups which, when present in the compound of formula I, do not prevent the compound of formula I from functioning as a potentiator of glutamate receptor function.

Examples of substituents which may be present in a substituted aromatic or heteroaromatic group include halogen; nitro; cyano; hydroxyimino; (1-10C) alkyl; (2-10C)alkenyl; (2-10C)alkynyl; (3-8C)cycloalkyl; hydroxy(3-8C) cycloalkyl; oxo(3-8C)cycloalkyl; halo(1-10C)alkyl; $(CH_2)_yX^1R^9$ in which y is 0 or an integer of from 1 to 4, $X^1$ represents O, S, $NR^{10}$, CO, COO, OCO, $CONR^{11}$, $NR^{12}CO$, $NR^{12}COCOO$, $OCONR^{13}$, $R^9$ represents hydrogen, (1-10C) alkyl, (3-10C)alkenyl, (3-10C)alkynyl, pyrrolidinyl, tetrahydrofuryl, morpholino or (3-8C)cycloalkyl and $R^{10}$, $R^{11}$, $R^{12}$ and $R^{13}$ each independently represents hydrogen or (1-10C)alkyl, or $R^9$ and $R^{10}$, $R^{11}$, $R^{12}$ or $R^{13}$ together with the nitrogen atom to which they are attached form an azetidinyl, pyrrolidinyl, piperidinyl or morpholino group; N-(1-4C)alkylpiperazinyl; N-phenyl(1-4C)alkylpiperazinyl; thienyl; furyl; oxazolyl; isoxazolyl; pyrazolyl; imidazolyl; thiazolyl; pyridyl; pyridazinyl; pyrimidinyl; dihydrothienyl; dihydrofuryl; dihydrothiopyranyl; dihydropyranyl; dihydrothiazolyl; (1-4C)alkoxycarbonyl dihydrothiazolyl; (1-4C)alkoxycarbonyl dimethyl-dihydrothiazolyl; tetrahydrothienyl; tetrahydrofuryl; tetrahydrothiopyranyl; tetrahydropyranyl; indolyl; benzofuryl; benzothienyl; benzimidazolyl; and a group of formula $R^{14}$-$(L^a)_n$-$X^2$-$(L^b)_m$ in which $X^2$ represents a bond, O, NH, S, SO, $SO_2$, CO, CH(OH), CONH, NHCO, NHCONH, NHCOO, COCONH, $OCH_2CONH$, or CH=CH, $L^a$ and $L^b$ each represent (1-4C)alkylene, one of n and m is 0 or 1 and the other is 0, and $R^{14}$ represents a phenyl or heteroaromatic group which is unsubstituted or substituted by one or two of halogen; nitro; cyano; (1-10C) alkyl; (2-10C)alkenyl; (2-10C)alkynyl; (3-8C)cycloalkyl; 4-(1,1-dioxotetrahydro-1,2-thiazinyl); halo (1-10C)alkyl; cyano(2-10C)alkenyl; phenyl; and $(CH_2)_zX^3R^{15}$ in which z is 0 or an integer of from 1 to 4, $X^3$ represents O, S, $NR^{16}$, CO, CH(OH), COO, OCO, $CONR^{17}$, $NR^{18}CO$, $NHSO_2$, $SO_2NH$, $NHSO_2NR^{17}$, $OCONR^{19}$, N(CO(1-4C) alkyl)CO, or $NR^{19}COO$, $R^{15}$ represents hydrogen, (1-10C)alkyl, phenyl(1-4C)alkyl, hato(1-10C)alkyl, (1-4C)alkoxycarbonyl(1-4C)alkyl, (1-4C)alkylsulfonylamino(1-4C)alkyl, (1-4C)alkylaminosulfonyl(1-4C)alkyl, N-(1-4C)alkoxycarbonyl) (1-4C)alkylsulfonylamino(1-4C)alkyl, (3-10C)alkenyl, (3-10C)alkynyl, (3-8C)cycloalkyl, camphoryl, or an aromatic or heteroaromatic group which is unsubstituted or substituted by one or two of halogen, (1-4C)alkyl, halo(1-4C)alkyl, di (1-4C)alkylamino and (1-4C)alkoxy, and $R^{16}$, $R^{17}$, $R^{18}$ and $R^{19}$ each independently represents hydrogen or (1-10C) alkyl, or $R^{15}$ and $R^{16}$, $R^{17}$, $R^{18}$ or $R^{19}$ together with the nitrogen atom to which they are attached form an azetidinyl, pyrrolidinyl, piperidinyl or morpholino group.

The term (1-10C)alkyl includes (1-8C)alkyl, (1-6C)alkyl and (1-4C)alkyl. Particular values are methyl, ethyl, propyl, isopropyl, butyl, isobutyl, t-butyl, pentyl, hexyl, heptyl, octyl, nonyl and decyl.

The term (2-10C)alkenyl includes (3-10C)alkenyl, (2-8C)alkenyl, (2-6C)alkenyl and (2-4C)alkenyl. Particular values are vinyl and prop-2-enyl.

The term (2-10C)alkynyl includes (3-10C)alkynyl, (2-8C)alkynyl, (2-6C)alkynyl and (3-4C)alkynyl. A particular value is prop-2-ynyl.

The term (3-8C)cycloalkyl, as such or in the term (3-8C)cycloalkyloxy, includes monocyclic and polycyclic groups. Particular values are cyclopropyl, cyclobutyl, cyclopentyl, cyclohexyl and bicyclo[2.2.2]octane. The term includes (3-6C)cycloalkyl: cyclopropyl, cyclobutyl, cyclopentyl and cyclohexyl.

As used herein the terms "integer of from 1 to 4" or "integer of from 1 to 3" includes the integers 1, 2, 3, and

4, or the integers 1, 2, and 3, respectively.

The term (5-8C)cycloalkyl includes cyclopentyl, cyclohexyl, cycloheptyl and cyclooctyl.

The term hydroxy(3-8C)cycloalkyl includes hydroxy-cyclopentyl, such as 3-hydroxycyclopentyl.

The term oxo(3-8C)cycloalkyl includes oxocyclopentyl, such as 3-oxocyclopentyl.

The terms "halogen", "Hal" or "halide" include fluorine, chlorine, bromine and iodine unless otherwise specified.

The term halo(1-10C)alkyl includes halo(1-6C)alkyl, halo(1-4C)alkyl, fluoro(1-10C)alkyl, fluoro(1-6C)alkyl, fluoro(1-4C)alkyl, chloro(1-6C)alkyl and chtoro(1-4C)alkyl, such as trifluoromethyl, 2,2,2-trifluoroethyl, and chloromethyl.

The term (1-10C)alkoxy includes (1-6C)alkoxy and (1-4C)alkoxy, such as methoxy, ethoxy, propoxy, isopropoxy and isobutoxy;

The term cyano(2-10C)alkenyl includes 2-cyanoethenyl.

The term (2-4C)alkylene includes ethylene, propylene and butylene. A preferred value is ethylene.

The term thienyl includes thien-2-yl and thien-3-yl.

The term furyl includes fur-2-yl and fur-3-yl.

The term oxazolyl includes oxazol-2-yl, oxazot-4-yl and oxazol-5-yl.

The term isoxazolyl includes isoxazol-3-yl, isoxazol-4-yl and isoxazol-5-yl.

The term oxadiazolyl includes [1,2,4]oxadiazol-3-yl and [1,2,4]oxadiazol-5-yl.

The term pyrazolyl includes pyrazol-3-yl, pyrazol-4-yl and pyrazol-5-yl.

The term thiazolyl includes thiazol-2-yl, thiazol-4-yl and thiazol-5-yl.

The term thiadiazolyl includes [1,2,4]thiadiazol-3-yl, and [1,2,4]thiadiazol-5-yl.

The term isothiazolyl includes isothiazol-3-yl, isothiazol-4-yl and isothiazol-5-yl.

The term imidazolyl includes imidazol-2-yl, imidazolyl-4-yl and imidazolyl-5-yl.

The term triazolyl includes [1,2,4]triazol-3-yl and [1,2,4]triazol-5-yl.

The term tetrazolyl includes tetrazol-5-yl.

The term pyridyl includes pyrid-2-yl, pyrid-3-yl and pyrid-4-yl.

The term pyridazinyl includes pyridazin-3-yl, pyridazin-4-yl, pyridazin-5-yl and pyridazin-6-yl.

The term pyrimidyl includes pyrimidin-2-yl, pyrimidin-4-yl, pyrimidin-5-yl and pyrimidin-6-yl.

The term benzofuryl includes benzofur-2-yl and benzofur-3-yl.

The term benzothienyl includes benzothien-2-yl and benzothien-3-yl.

The term benzimidazolyl includes benzimidazol-2-yl.

The term benzoxazolyl includes benzoxazol-2-yl.

The term benzothiazolyl includes benzothiazol-2-yl.

The term indolyl includes indol-2-yl and indol-3-yl.

The term quinolyl includes quinol-2-yl.

The term dihydrothiazolyl includes 4,5-dihydrothiazol-2-yl, and the term (1-4C)alkoxycarbonyldihydrothiazolyl includes 4-methoxycarbonyl-4,5-dihydrothiazol-2-yl.

The term -(1-4C)alkyl(3-8C)cycloalkyl includes the following:

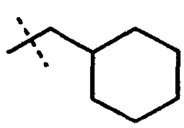 , 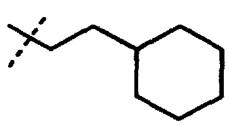 , 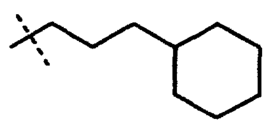 , 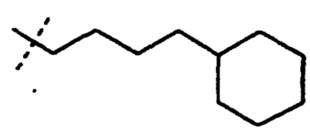

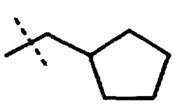 , 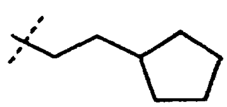 , 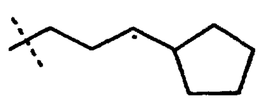 and 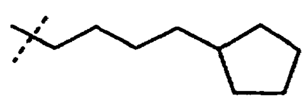 .

The term -(1-4C)alkylaromatic includes the following:

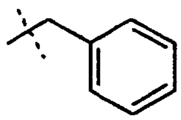, 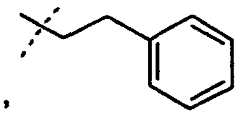, 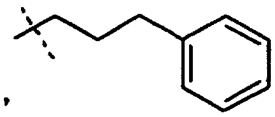. 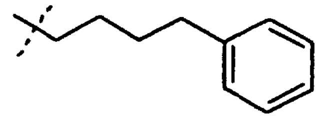.

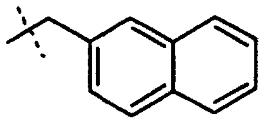, 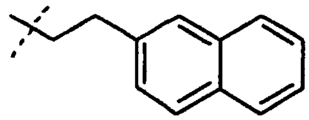, 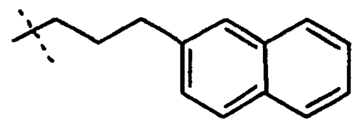 and

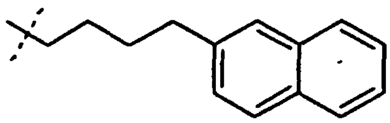

$R^2$ is preferably (1-6C)alkyl, with methyl, ethyl, propyl, 2-propyl, and butyl being most preferred, and 2-propyl being most especially preferred.

Examples of values for $R^9$ are hydrogen, methyl, ethyl, propyl, isopropyl, t-butyl, ethenyl, cyclopropyl, cyclobutyl, cyclopentyl, cyclohexyl, 2-pyrrolidinyl, morpholino or 2-tetrahydrofuryl.

$R^9$ is preferably (1-4C)alkyl, (2-4C)alkenyl, (3-6C)cycloalkyl, pyrrolidinyl, morpholino or tetrahydrofuryl with methyl and 2-propyl being especially preferred.

Examples of values for $R^{15}$ are hydrogen, methyl, ethyl, propyl, isopropyl, butyl, t-butyl, benzyl, 2,2,2-trifluoroethyl, 2-methoxycarbonylethyl, cyclohexyl, 10-camphoryl, phenyl, 2-fluorophenyl, 3-fluorophenyl, 2-trifluoromethylphenyl, 4-trifluoromethylphenyl, 2-methoxyphenyl, 3-methoxyphenyl, 4-methoxyphenyl, 1-(5-dimethylamino)naphthyl, and 2-thienyl.

$X^1$ preferably represents O, CO, CONH or NHCO.

z is preferably 0.

Particular values for the groups $(CH_2)_yX^1R^9$ and $(CH_2)_zX^3R^{15}$ include (1-10C)alkoxy, including (1-6C)alkoxy and (1-4C)alkoxy, such as methoxy, ethoxy, propoxy, isopropoxy and isobutoxy; (3-10C)alkenyloxy, including (3-6C) alkenyloxy, such as prop-2-enyloxy; (3-10C)alkynyloxy, including (3-6C)alkynyloxy, such as prop-2-ynyloxy; and (1-6C) alkanoyl, such as formyl and ethanoyl.

Examples of particular values for y are 0 and 1.

Examples of particular values for z are 0, 1, 2 and 3.

$L^a$ and $L^b$ preferably each independently represents $CH_2$.

$X^2$ preferably represents a bond, O, NH, CO, CH(OH), CONH, NHCONH or $OCH_2CONH$, with a bond, O, and CONH being especially preferred.

Preferably the group $(CH_2)yX^1R^9$ represents CHO; $COCH_3$, $OCH_3$; $OCH(CH_3)_2$; $NHCOR^9$ in which $R^9$ represents methyl, ethyl, isopropyl, t-butyl, ethenyl, cyclopropyl, cyclobutyl, cyclopentyl, cyclohexyl, 2-pyrolidinyl or morpholino; $CONHR^9$ in which $R^9$ represents cyclopropyl or cyclopentyl; NHCOCOOCH3; or 2-tetrahydrofurylmethoxy.

Preferably the group $(CH_2)_zX^3R^{15}$ represents $NH_2$; $CH_2NH_2$; $(CH_2)_2NH_2$; $(CH_2)_3NH_2$; $CONH_2$; $CONHCH_3$; $CON(CH_3)_2$; $N(C_2H_5)_2$; $CH_2OH$; $CH(OH)CH_3$; $CH(OH)CH_2CH_2$; CHO; $COCH_3$; COOH; $COOCH_3$; $CH_2NHCOOC(CH_3)_3$; $(CH_2)_2NHCOOC(CH_3)_3$; $SO_2NH_2$; $NHSO_2CH_3$; $NHSO_2CH(CH_3)_2$, $OCH(CH_3)CH_2NHSO_2CH(CH_3)_2$, $N(COCH_3)_2$; a group of formula $(CH_2)_2NHSO_2R^{15}$ in which $R^{15}$ represents $CH_3$, $CH_2CH_3$, $CH(CH_3)_2$, $(CH_2)_2CH_3$, $(CH_3)_3CH_3$, benzyl, $CH_2CF_3$, 2-methoxycarbonylethyl, cyclohexyl, 10-camphoryl, phenyl, 2-fluorophenyl, 4-fluorophenyl, 2-trifluoromethylphenyl, 4-trifluoromethylphenyl, 4-methoxyphenyl, 1-(2-dimethylamino)naphthyl or 2-thienyl; $CH(OH)CH_2NHSO_2CH_3$; $(CH_2)_3NHSO_2CH(CH_3)_2$; $COCH_2N(OCOC(CH_3)_2SO_2CH_3$; $COCH_2NHSO_2CH_3$; $(CH_2)_2NHCOR^{15}$ in which $R^{15}$ represents $CH_3$, $CH(CH_3)_2$, $CH_2CH(CH_3)_2$, phenyl, 3-fluorophenyl, 4-fluorophenyl, benzyl, 2-methoxyphenyl, 4-methoxyphenyl, 2-thienyl, CH=CH, CH=CHCN, $OCH_3$ or $O(CH_2)_3CH_3$.

Examples of particular values for $(L^a)_n$-$X^2$-$(L^b)_m$ are a bond, O, NH, S, SO, $SO_2$, CO, $CH_2$, $COCH_2$, COCONH, $CH(OH)CH_2$, CONH, NHCO, NHCONH, $CH_2O$, $OCH_2$, $OCH_2CONH$, $CH_2NH$, $NHCH_2$ and $CH_2CH_2$, with a bond, CONH, and $CH_2O$ being especially preferred.

$R^{14}$ is preferably an unsubstituted or substituted phenyl, naphthyl, furyl, thienyl, isoxazolyl, thiazolyl, tetrazolyl, pyridyl, pyrimidyl benzothienyl or benzothiazolyl group.

Examples of particular values for $R^{14}$ are phenyl, 2-fluorophenyl, 3-fluorophenyl, 4-fluorophenyl, 2-chloro-phenyl, 3-chlorophenyl, 4-chlorophenyl, 2-bromophenyl, 3-bromophenyl, 4-bromophenyl, 4-iodophenyl, 2,3-difluorophenyl, 2,4-difluorophenyl, 3,5-difluorophenyl, 3,4-dichlorophenyl, 3,5-dichlorophenyl, 4-cyanophenyl, 3-nitrophenyl, 4-hydroxyiminophenyl, 2-methylphenyl, 4-methylphenyl, 4-ethylphenyl, 3-propylphenyl, 4-t-butylphenyl, 2-prop-2-enylphenyl, 4-(4-(1,1-dioxotetrahydro-1,2-thiazinyl)phenyl, 2-trifluoromethylphenyl, 3-trifluoromethylphenyl, 4-trifluoromethylphenyl, 2-bromomethylphenyl, 2-fluoro-4-trifluoromethylphenyl, 4-(2-cyanoethenyl)phenyl, 4-phenyl, 2-formylphenyl, 3-formylphenyl, 4-formylphenyl, 2-acetylphenyl, 3-acetylphenyl, 4-acetylphenyl, 2-propanoylphenyl, 2-(2-methyl-propanoyl)phenyl, 2-methoxyphenyl, 3-methoxyphenyl, 4-methoxyphenyl, 4-butoxyphenyl, 2-hydroxymethylphenyl, 4-hydroxymethylphenyl, 2-(1-hydroxyethyl)phenyl, 3-(1-hydroxyethyl)phenyl, 4-(1-hydroxyethyl)phenyl, 2-(1-hydroxypropyl)phenyl, 4-(1-hydroxypropyl)phenyl, 2-(1-hydroxy-2,2-dimethyl-propyl)phenyl, 4-trifluoromethoxyphenyl, 2-aminophenyl,4-aminophenyl, 4-N,N-diethylaminophenyl, 4-aminomethylphenyl, 4-(2-aminoethyl)phenyl, 4-(3-aminopropyl)phenyl, 4-carboxyphenyl, 4-carbamoylphenyl, 4-N-methylcarbamoylphenyl, 4-N,N-dimethylcarbamoylphenyl, 2-isopropylaminomethylphenyl, 4-t-butoxycarbonylaminomethylphenyl, 4-(2-isopropoxy-carboxamido)ethylphenyl, 4-(2-t-butoxycarboxamido)ethyl-phenyl, 4-isopropylsulfonylaminophenyl, 4-(2-methane-sulfonylamino)ethylphenyl, 4-(2-ethylsulfonylamino)ethyl-phenyl, 4-(3-isopropylsulfonylamino)propylphenyl, 4-(1-(2-(2-propane)sulfonylamino) propyl)phenyl, 4-(2-propylsulfonylamino)ethylphenyl, 4-(2-isopropylsulfonylamino)ethylphenyl, 4-(2-butylsulfonylamino)ethylphenyl, 4-(1-isopropyl-sulfonylaminomethyl)ethylphenyl, 4-(1-hydroxy-2-methane-sulfonylamino)ethylphenyl, 4-(2-(2,2,2-trifluoroethyl)-sulfonylaminoethyl)phenyl, 4-(2-cyclohexylsulfonylamino)-ethylphenyl, 4-(2-(2,2,2-trifluoroethyl)sulfonylamino)-ethylphenyl, 4-(2-N,N-dimethylaminosulfonylamino)-ethylphenyl, 4-(2-phenylsulfonylaminoethyl) phenyl,4-(2-(2-fluorophenyl)sulfonylaminoethyl)phenyl,4-(2-(4-fluorophenyl)sulfonylaminoethyl)phenyl,4-(2-(2-trifluoromethylphenyl)sulfonylaminoethyl)phenyl, 4-(2-(4-trifluoromethylphenyl)sulfonylaminoethyl)phenyl, 4-(2-(4-methoxyphenyl)sulfonylaminoethyl)phenyl, 4-(2-(1-(5-dimethylamino)napthalenesulfonylamino)ethyl)phenyl, 4-(2-(2-thienyl) sulfonylamino)ethyl)phenyl, 4-(2-benzamidoethyl)-phenyl, 4-(2-(4-fluorobenzamido)ethyl)phenyl, 4-(2-(3-methoxybenzamido)ethyl)phenyl, 4-(2-(3-fluorobenzamido)-ethyl)phenyl, 4-(2-(4-methoxybenzamido)ethyl)phenyl, 4-(2-(2-methoxybenzamido)ethyl)phenyl, 4-(1-(2-(2-methoxycarbonylethanesulfonylamino)ethyl)phenyl, 4-(1-(2-(10-camphorsulfonylamino)ethyl)phenyl, 4-(1-(2-(benzylsulfonyl-amino)ethyl)phenyl, 4-(2-phenylacetamido)ethyl)phenyl, 4-methanesulfonylaminoethanoylphenyl, 4-(N-(t-butoxy-carbonyl)methanesulfonylaminoethanoyl)phenyl, 4-(2-(2-thienylcarboxamido)ethyl)phenyl, thien-2-yl, 5-hydroxy-methylthien-2-yl, 5-formylthien-2-yl, thien-3-yl, 5-hydroxymethylthien-3-yl, 5-formylthien-3-yl, 2-bromothien-3-yl, fur-2-yl, 5-nitrofur 2-yl, fur-3-yl, isoxazol-5-yl, 3-bromoisoxazol-5-yl, isoxazol-3-yl, 5-trimethylsilylisoxazot-3-yl, 5-methylisoxazol-3-yl, 5-hydroxymethylisoxazol-3-yl, 5-methyl-3-phenylisoxazol-4-yl, 5-(2-hydroxyethyl)isoxazol-3-yl, 5-acetylisoxazol-3-yl, 5-carboxyisoxazol-3-yl, 5-N-methylcarbamoylisoxazol-3-yl, 5-methoxycarbonylisoxazol-3-yl, 3-bromo[1,2,4]oxadiazol-5-yl, pyrazol-1-yl, thiazol-2-yl, 4-hydroxymethylthiazol-2-yl, 4-methoxycarbonylthiazol-2-yl, 4-carboxythiazol-2-yl, imidazol-1-yl, 2-sulfhydrylimidazol-1-yl, [1,2,4]triazol-1-yl, tetrazol-5-yl, 2-methyltetrazol-5-yl, 2-ethyltetrazol-5-yl, 2-isopropyl-tetrazol-5-yl, 2-(2-propenyl)tetrazol-5-yl, 2-benzyl-tetrazol-5-yl, pyrid-2-yl, 5-ethoxycarbonylpyrid-2-yl, pyrid-3-yl, 6-chloropyrid-3-yl, pyrid-4-yl, 5-trifluoro-methylpyrid-2-yl, 6-chloropyridazin-3-yl, 6-methylpyridazin-3-yl, 6-methoxypyrazin-3-yl, pyrimidin-5-yl, benzothien-2-yl, benzothiazol-2-yl, and quinol-2-yl.

Examples of an unsubstituted or substituted aromatic or heteroaromatic group represented by $R^1$ are unsubstituted or substituted phenyl, furyl, thienyl (such as 3-thienyl) and pyridyl (such as 3-pyridyl), with substituted phenyl being preferred.

Examples of an unsubstituted or substituted (5-8C)cycloalkyl group represented by $R^1$ are unsubstituted or substituted cyclopentyl, cyclohexyl, cycloheptyl and cyclooctyl, with cyclohexyl being preferred.

In particular, $R^1$ represents substituents of the following structures:

| |
|---|
| 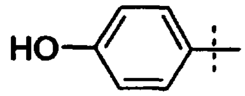 |
| 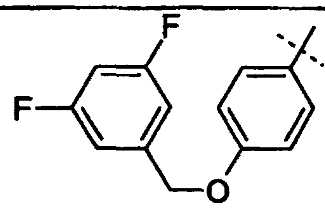 |
| 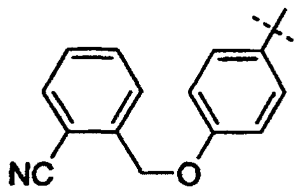 |
| 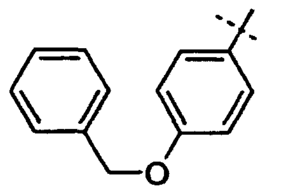 |
| 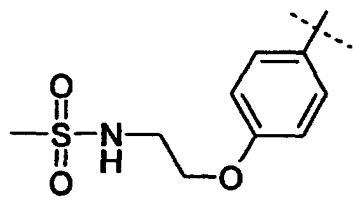 |
| 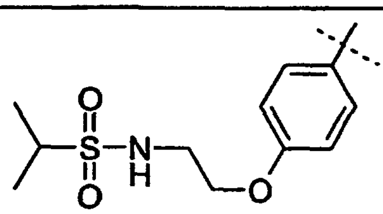 |

| |
|---|
| 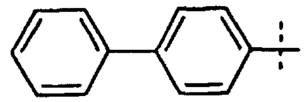 |
| 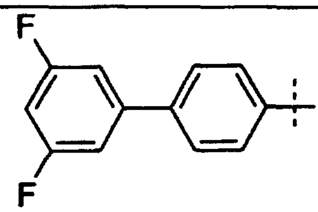 |
| 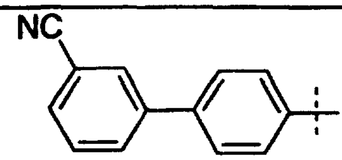 |
| 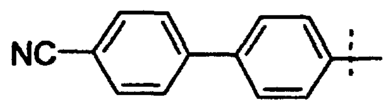 |
| 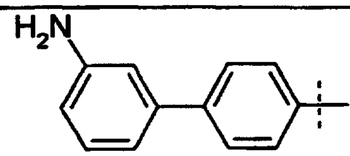 |
| 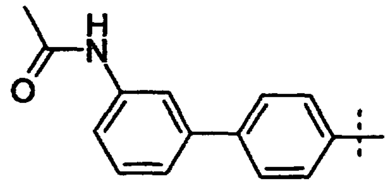 |
| 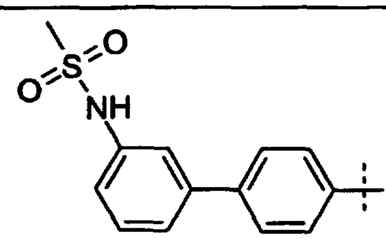 |
| 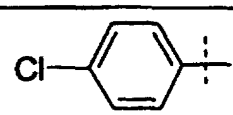 |
| 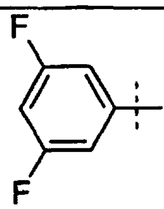 |

More preferably, $R^1$ represents a group of formula:

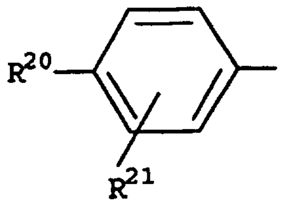
,
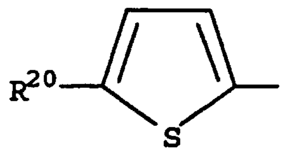
,
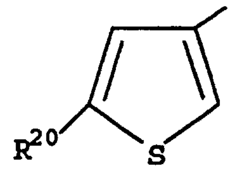
or

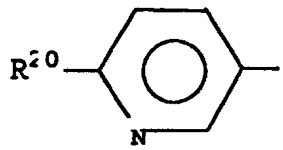
.

with a group of the formula:

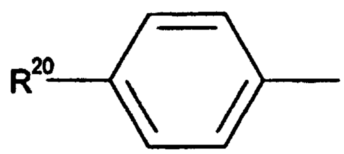

being most especially preferred, wherein

$R^{20}$ represents halogen; nitro; cyano; hydroxyimino; (1-10C)alkyl; (2-10C)alkenyl; (2-10C)alkynyl; (3-8C)cyclo-alkyl; hydroxy(3-8C)cycloalkyl; oxo(3-8C)cycloalkyl; halo(1-10C)alkyl; $(CH_2)_yX^1R^9$ in which y is 0 or an integer of from 1 to 4, $X^1$ represents O, S, $NHSO_2$, $SO_2NH$, $NR^{10}$, CO, COO, OCO, $CONR^{11}$, $NR^{12}CO$, $NR^{12}COCOO$, $OCONR^{13}$, $R^9$ represents hydrogen, (1-10C) alkyl, (3-10C)alkenyl, (3-10C)alkynyl, pyrrolidinyl, tetrahydrofuryl, morpholino or (3-8C)cycloalkyl and $R^{10}$, $R^{11}$, $R^{12}$ and $R^{13}$ each independently represents hydrogen or (1-10C)alkyl, or $R^9$ and $R^{10}$, $R^{11}$, $R^{12}$ or $R^{13}$ together with the nitrogen atom to which they are attached form an azetidinyl, pyrrolidinyl, piperidinyl or morpholino group; N-(1-4C)alkylpiperazinyl; N-phenyl(1-4C)alkylpiperazinyl; thienyl; furyl; oxazolyl; isoxazolyl; pyrazolyl; imidazolyl; thiazolyl; tetrazolyl; pyridyl; pyridazinyl; pyrimidinyl; dihydrothienyl; dihydrofuryl; dihydrothiopyranyl; dihydropyranyl; dihydrothiazolyl; (1-4C)alkoxycarbonyldihydrothiazolyl; (1-4C)alkoxycarbonyldimethyl-dihydrothiazolyl; tetrahydrothienyl; tetrahydrofuryl; tetrahydrothiopyranyl; tetrahydropyranyl; indolyl; benzofuryl; benzothienyl; benzimidazolyl; benzothiazolyl; and a group of formula $R^{14}$-$(L^a)_n$-$X^2$-$(L^b)_m$ in which $X^2$ represents a bond, O, NH, S, SO, $SO_2$, CO, CH(OH), CONH, NHCONH, NHCOO, COCONH, $OCH_2CONH$ or CH=CH, NHCO, $L^a$ and $L^b$ each represent (1-4C)alkylene, one of n and m is 0 or 1 and the other is 0, and $R^{14}$ represents a phenyl or hetero-aromatic group which is unsubstituted or substituted by one or two of halogen; nitro; cyano; (1-10C)alkyl; (2-10C)alkenyl; (2-10C) alkynyl; (3-8C)cycloalkyl; 4-(1,1-dioxotetrahydro-1,2-thiazinyl); halo(1-10C)alkyl; cyano(2-10C)alkenyl; phenyl; $(CH_2)_zX^3R^{15}$ in which z is 0 or an integer of from 1 to 4, $X^3$ represents O, S, $NR^{16}$, CO, CH(OH), COO, OCO, $CONR^{17}$, $NR^{18}CO$, $NHSO_2$, $SO_2NH$, $NHSO_2NR^{17}$, NHCONH, $OCONR^{19}$, N(CO(1-4C)alkyl)CO, or $NR^{19}COO$, $R^{15}$ represents hydrogen, (1-10C)alkyl, phenyl(1-4C)alkyl, halo(1-10C)alkyl, (1-4C)alkoxycarbonyl(1-4C)alkyl, (1-4C)alkylsulfonylamino(1-4C)alkyl, (1-4C)alkylaminosulfonyl(1-4C)alkyl, (N-(1-4C)alkoxycarbonyl)(1-4C)alkylsulfonylamino(1-4C)alkyl, (3-10C)alkenyl, (3-10C)alkynyl, (3-8C)cycloalkyl, camphoryl or an aromatic or heteroaromatic group which is unsubstituted or substituted by one or two of halogen, (1-4C)alkyl, halo(1-4C)alkyl, di(1-4C)alkylamino and (1-4C)alkoxy, and $R^{16}$, $R^{17}$, $R^{18}$ and $R^{19}$ each independently represents hydrogen or (1-10C)alkyl, or $R^{15}$ and $R^{16}$, $R^{17}$, $R^{18}$ or $R^{19}$ together with the nitrogen atom to which they are attached form an azetidinyl, pyrrolidinyl, piperidinyl or morpholino group; and

$R^{21}$ represents a hydrogen atom, a halogen atom, a (1-4C)alkyl group or a (1-4C)alkoxy group.

$R^{20}$ preferably represents halogen; (1-10C)alkyl; halo(1-10C)alkyl; $(CH_2)_yX^1R^9$ in which y is 0 or an integer of from 1 to 4, $X^1$ represents O, S, $NHSO_2$, $SO_2NH$, $NR^{10}$, CO, COO, OCO, $CONR^{11}$, $NR^{12}CO$, $NR^{12}COCOO$, $OCONR^{13}$, $R^9$ represents hydrogen, (1-10C) alkyl, (3-10C)alkenyl, (3-10C)alkynyl, pyrrolidinyl, tetrahydrofuryl, morpholino or (3-8C)cycloalkyl and $R^{10}$, $R^{11}$, $R^{12}$ and $R^{13}$ each independently represents hydrogen or (1-10C)alkyl, or $R^9$ and $R^{10}$, $R^{11}$, $R^{12}$ or $R^{13}$ together with the nitrogen atom to which they are attached form a pyrrolidinyl, piperidinyl or morpholino group; and a group of formula $R^{14}$-$(L^a)_n$-$X^2$-$(L^b)_m$ in which $X^2$ represents a bond, O, NH, S, SO, $SO_2$, CO, CH(OH), CONH, NHCONH, NHCOO, COCONH, $OCH_2CONH$ or CH=CH, NHCO, $L^a$ and $L^b$ each represent (1-4C)alkylene, one of n and m is 0 or 1 and the other is 0, and $R^{14}$ represents a phenyl group which is unsubstituted or substituted by one or two of halogen; nitro; cyano; (1-10C)alkyl; halo(1-10C)alkyl; phenyl; $(CH_2)_zX^3R^{15}$ in which z is 0 or an integer of from 1 to 4, $X^3$ represents O, S, $NR^{16}$, CO, CH(OH), COO, OCO, $CONR^{17}$, $NR^{18}CO$, $NHSO_2$, $SO_2NH$, $NHSO_2NR^{17}$, NHCONH, $OCONR^{19}$, N(CO(1-4C)alkyl)CO, or $NR^{19}COO$, $R^{15}$ represents hydrogen, (1-10C)alkyl, phenyl(1-4C)alkyl, halo(1-10C)alkyl, (1-4C)alkoxycarbonyl(1-4C)alkyl, (1-4C)alkylsulfonylamino(1-4C)alkyl, (1-4C)alkylaminosulfonyl(1-4C)alkyl, (N-(1-4C)alkoxycarbonyl)(1-4C)alkylsulfonylamino(1-4C)alkyl, or an aromatic or heteroaromatic group which is unsubstituted or substituted by one or two of halogen, (1-4C)alkyl, halo(1-4C)alkyl, di(1-4C)alkylamino and (1-4C)alkoxy, and $R^{16}$, $R^{17}$, $R^{18}$ and $R^{19}$ each independently represents hydrogen or (1-10C)alkyl, or $R^{15}$ and $R^{16}$, $R^{17}$, $R^{18}$ or $R^{19}$ together with the nitrogen atom to which they are attached form a pyrrolidinyl, piperidinyl or morpholino group.

$R^{20}$ most preferably represents $(CH_2)_yX^1R^9$ in which y is 0 or an integer of from 1 to 4, $X^1$ represents O, S, $NHSO_2$, $SO_2NH$, $NR^{10}$, CO, COO, OCO, $CONR^{11}$, $NR^{12}CO$, $NR^{12}COCOO$, $OCONR^{13}$, $R^9$ represents hydrogen, (1-10C) alkyl, pyrrolidinyl, tetrahydrofuryl, morpholino or (3-8C)cycloalkyl and $R^{10}$, $R^{11}$, $R^{12}$ and $R^{13}$ each independently represents hydrogen or (1-10C)alkyl, or $R^9$ and $R^{10}$, $R^{11}$, $R^{12}$ or $R^{13}$ together with the nitrogen atom to which they are attached form a pyrrolidinyl, piperidinyl or morpholino group; and a group of formula $R^{14}$-$(L^a)_n$-$X^2$-$(L^b)_m$ in which $X^2$ represents a bond, O, NH, S, SO, $SO_2$, CO, CONH, NHCOO, or NHCO, $L^a$ and $L^b$ each represent (1-4C)alkylene, one of n and m is 0 or 1 and the other is 0, and $R^{14}$ represents a phenyl group which is unsubstituted or substituted by one or two of halogen; (1-10C)alkyl; halo(1-10C)alkyl; phenyl; $(CH_2)_zX^3R^{15}$ in which z is 0 or an integer of from 1 to 4, $X^3$ represents O, S, $NR^{16}$, CO, COO, OCO, $CONR^{17}$, $NR^{18}CO$, $NHSO_2$, $SO_2NH$, $NHSO_2NR^{17}$, NHCONH, $OCONR^{19}$, N (CO(1-4C)alkyl)CO, or $NR^{19}COO$, $R^{15}$ represents hydrogen, (1-10C)alkyl, phenyl(1-4C)alkyl, halo(1-10C)alkyl, (1-4C) alkoxycarbonyl(1-4C)alkyl, (1-4C)alkylsulfonylamino(1-4C)alkyl, (1-4C)alkylaminosulfonyl(1-4C)alkyl, (N-(1-4C)alkoxycarbonyl)(1-4C)alkylsulfonylamino(1-4C)alkyl, or an aromatic or heteroaromatic group which is unsubstituted or substituted by one or two of halogen, (1-4C)alkyl, halo(1-4C)alkyl, di(1-4C)alkylamino and (1-4C)alkoxy, and $R^{16}$, $R^{17}$, $R^{18}$ and $R^{19}$ each independently represents hydrogen or (1-10C)alkyl, or $R^{15}$ and $R^{16}$, $R^{17}$, $R^{18}$ or $R^{19}$ together with the nitrogen atom to which they are attached form a pyrrolidinyl, piperidinyl or morpholino group.

Examples of particular values for $R^{20}$ are fluorine, chlorine, bromine, cyano, hydroxyimino, methyl, ethyl, propyl, 2-propyl, butyl, 2-methylpropyl, 1,1-dimethylethyl, cyclopentyl, cyclohexyl, 3-hydroxycyclopentyl, 3-oxocyclopentyl, methoxy, ethoxy, propoxy, 2-propoxy, acetyl, acetylamino, ethylcarboxamido, propylcarboxamido, 1-butanoylamido, t-butylcarboxamido, acryloylamido, 2-pyrrolidinylcarboxamido, 2-tetrahydrofurylmethoxy, morpholinocarboxamido, methyloxalylamido, cyclo-propylcarboxamido, cyclobutylcarboxamido, cyclopentyl-carboxamido, cyclohexylcarboxamido, cyclopropylcarbamoyl, cyclopentylcarbamoyl, pyrrolidin-1-yl, morpholino, piperidin-1-yl, N-methylpiperazinyl, N-benzylpiperazinyl, 2-thienyl, 3-thienyl, 2-furyl, 3-furyl, isoxazol-3-yl, thiazol-2-yl, tetrazol-5-yl, pyrid-2-yl, pyrid-3-yl, pyrid-4-yl, pyrimidin-5-yl, 4,5-dihydrothiazol-2-yl, 4,5-dihydro-4-methoxycarbonylthiazol-2-yl, 4,5-dihydro-4-methoxy-carbonyl-5,5-dimethylthiazol-2-yl, benzothien-2-yl, benzothiazol-2-yl, phenyl, 2-fluorophenyl, 3-fluorophenyl, 2,3-difluorophenyl, 4-chlorophenyl, 3,4-dichlorophenyl, 3,5-dichlorophenyl, 3-nitrophenyl, 4-cyanophenyl, 2-methylphenyl, 4-methylphenyl, 4-(4-(1,1-dioxotetrahydro-1,2-thiazinyl)phenyl, 3-trifluoromethylphenyl, 4-trifluoro-methylphenyl, 4-(2-cyanoethenyl)phenyl, 2-formylphenyl, 3-formylphenyl, 4-formylphenyl, 3-acetylphenyl, 4-acetylphenyl, 4-carboxyphenyl, 2-methoxyphenyl, 4-methoxyphenyl, 2-hydroxymethylphenyl, 4-hydroxymethylphenyl, 3-(1-hydroxyethyl) phenyl, 4-(1-hydroxyethyl)phenyl, 4-(1-hydroxypropyl)phenyl, 2-aminophenyl, 4-aminophenyl, 4-N,N-diethylaminophenyl, 4-aminomethylphenyl, 4-(2-aminoethyl)-phenyl, 4-(3-aminopropyl)phenyl, 4-(2-acetylaminoethyl)-phenyl, 4-t-butoxycarboxylaminoethyl)phenyl, 4-(2-t-butoxycarboxylaminoethyl)phenyl, benzylsulfonylamino, 4-isopropylsulfonylaminophenyl, 4-(2-methanesulfonylaminoethyl)phenyl, 4-(2-ethylsulfonylaminoethyl)phenyl, 4-(2-propylsulfonylaminoethyl)phenyl, 4-(2-butylsulfonyl-aminoethyl)phenyl, 4-(2-isopropylsulfonylaminoethyl)phenyl, 4-(1-hydroxy-2-methanesulfonylaminoethyl)phenyl, 4-(2-dimethylaminosulfonylaminoethyl)phenyl, 4-(1-(2-(2-propyl)sulfonylaminopropyl)phenyl, 4-(2-(2,2,2-trifluoroethyl)sulfonylaminoethyl)phenyl, 4-(2-cyclohexylsulfonyl-aminoethyl)phenyl, 4-(2-phenylsulfonylaminoethyl)phenyl, 4-(2-(2-fluorophenyl)sulfonylaminoethyl)phenyl, 4-(2-(4-fluorophenyl)sulfonylaminoethyl)phenyl, 4-(2-(2-trifluoromethylphenyl)sulfonylaminoethyl)phenyl, 4-(2-(4-trifluoromethylphenyl)sulfonylaminoethyl)phenyl, 4-(2-(4-methoxyphenyl)sulfonylaminoethyl)phenyl, 4-(2-(1-(5-dimethylamino)napthalenesulfonylamino)ethyl)phenyl, 4-(2-(2-thienyl)sulfonylamino)ethyl)phenyl, 4-(2-benzamidoethyl)-phenyl, 4-(2-(4-fluorobenzamido)ethyl)phenyl, 4-(2-(3-methoxybenzamido)ethyl)phenyl, 4-(2-(3-fluorobenzamido)-ethyl)phenyl, 4-(2-(4-methoxybenzamido)ethyl)phenyl, 4-(2-(2-methoxybenzamido)ethyl)phenyl, 4-(2-(2-thienyl-carboxamido)ethyl)phenyl, 4-carbamoylphenyl, 4-methyl-carbamoylphenyl, 4-dimethylcarbamoylphenyl, 4-(2-(2-methylpropaneamido)ethyl)phenyl, 4-(2-(3-methyl-butaneamido)ethyl)phenyl, benzoylmethyl, benzamido, 2-fluorobenzamido, 3-flurobenzamido, 4-fluorobenzamido, 2,4-difluorobenzamido, 3-chlorobenzamido, 4-chlorobenzamido, 4-bromobenzamido, 4-iodobenzamido, 4-cyanobenzamido, 3-methylbenzamido, 4-methylbenzamido, 4-ethylbenzamido, 4-propylbenzamido, 4-t-butylbenzamido, 4-vinylbenzamido, 2-trifluoromethylbenzamido, 3-trifluoromethylbenzamido, 4-trifluoromethylbenzamido, 2-fluoro-4-trifluoromethylbenzamido, 2-methoxybenzamido, 3-methoxybenzamido, 4-methoxybenzamido, 4-butoxybenzamido, 4-phenylphenyl-carboxamido, 4-benzylcarboxamido, 4-phenoxymethyl-carboxamido, 2-fluorobenzylamino, benzyloxy, 2-fluorobenzyloxy, 2-hydroxy-2-phenylethyl, 2-fluorophenylcarbamoyl, 4-(1-(2-(2-methoxycarbonylethanesulfonylamino)ethyl)phenyl, 4-(1-(2-(10-camphorsulfonylamino)ethyl)phenyl, 4-(1-(2-(benzylsulfonylamino)ethyl)phenyl, 4-(2-phenylacetamido)-ethyl)phenyl, 4-(methanesulfonylaminoethanoyl)phenyl, 4-(N-t-butoxycarbonyl)methanesulfonylaminoethanoyl)phenyl, 2-thienylcarboxamido, 2-furylcarboxamido, 3-(5-methyl-isoxazolyl)carboxamido, 5-isoxazolylcarboxamido, 2-benzathienylcarboxamido, 4-(5-methyl-3-phenylisoxazolyl)-carboxamido, 4-pyridylcarboxamido, 2-(5-nitrofuryl)-carboxamido, 2-pyridylcarboxamido, 6-chloro-2-pyridyl-carboxamido, 2-thienylsulfonamido, 2-thienylmethylamino, 3-thienylmethylamino, 2-furylmethylamino, 3-furylmethylamino, 3-acetylureido and 2-(2-thienyl)ethylureido.

Examples of particular values for $R^{21}$ are hydrogen and chlorine with hydrogen being most preferred. $R^{21}$ is preferably ortho to $R^{20}$.

Examples of particular values for $R^1$ are 2-naphthyl, 4-bromophenyl, 4-cyanophenyl, 4-benzamidophenyl, 4-methylphenyl, 4-isopropyl-phenyl, 4-isobutylphenyl, 4-*t*-butylphenyl, 4-methoxyphenyl, 4-isopropoxyphenyl, 4-cyclopentylphenyl, 4-cyclohexylphenyl, 4-(2-hydroxymethylphenyl)phenyl, 4-(4-hydroxymethylphenyl)-phenyl, 4-(2-furyl)phenyl, 4-(3-furyl)phenyl, 4-(2-thienyl)phenyl, 4-(3-thienyl)phenyl, 4-(pyrrolidin-1-yl)phenyl, 4-(piperidin-l-yl)phenyl, 3-chloro-4-piperidin-1-ylphenyl, 4-benzyloxyphenyl, 4-(2-fluorophenyl)phenyl, 4-(3-fluoro-phenyl)phenyl, 4-(2-formylphenyl)phenyl, 4-(3-formylphenyl)-phenyl, 4-(4-formylphenyl)phenyl, 4-(4-methylphenyl)phenyl, and 4-(2-methoxyphenyl)phenyl.

The compounds of formula I can be prepared by one of ordinary skill in the art following art recognized techniques and procedures. Such techniques and procedures can be found for example in International Patent Application Publications: WO 98/33496 published August 6, 1998; WO 99/43285 published September 2, 1999; WO 00/06539; WO 00/06537, WO 00/06176, WO 00/06159, WO 00/06158, WO 00/06157, WO 00/06156, WO 00/06149, WO 00/06148, and WO 00/06083, all published February 10, 2000; and WO 00/66546 published November 9, 2000. More specifically, compounds of formula I can be prepared as set forth in Scheme I. The reagents and starting materials are readily available to one of ordinary skill in the art. All substituents, unless otherwise specified are as previously defined.

Scheme I

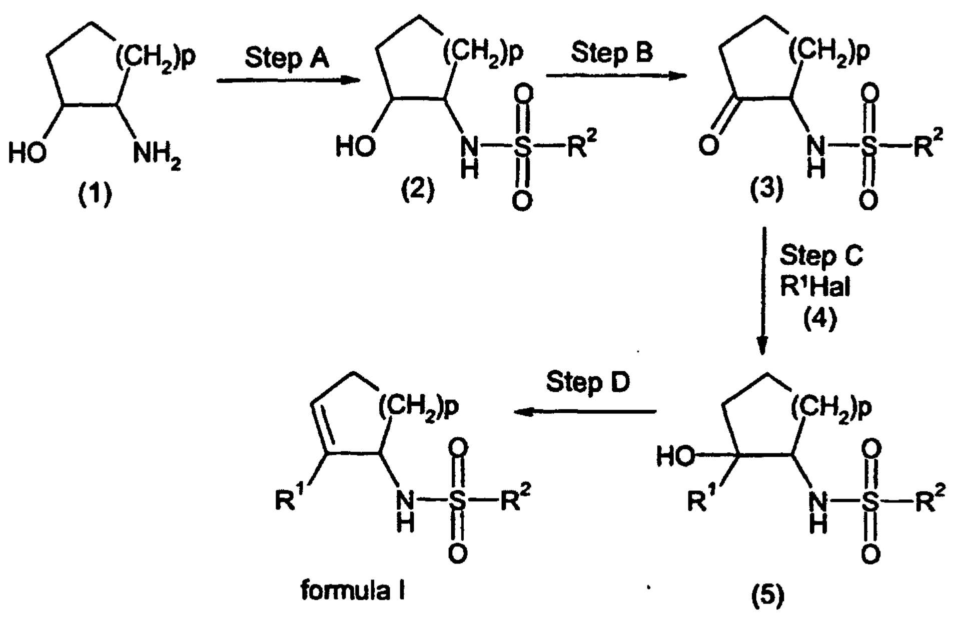

(1) (2) (3) (5) formula I

In Scheme I, step A the amine of structure (1) is converted to the sulfonamide of structure (2) under conditions well known in the art. For example, amine (1) is dissolved in a suitable organic solvent. Examples of suitable organic solvents include methylene chloride, tetrahydrofuran, and the like. The solution is treated with a slight excess of a suitable base, and then cooled to about -78°C to about 0°C. Examples of suitable bases include triethylamine, pyridine, 1,8-diazabicyclo[5.4.0]undec-7-ene (DBU), and the like. To the stirring solution is added one equivalent of $LgSO_2R^2$. The term "Lg" as used herein refers to a suitable leaving group. Examples of suitable leaving groups include, Cl, Br, and the like. Cl is the preferred leaving group. The reaction mixture is stirred at about 0°C to about 50°C for about 0.5 hours to about 16 hours. The sulfonamide (2) is then isolated and purified by techniques well known in the art, such as extraction techniques and chromatography. For example, the mixture is washed with 10% sodium bisulfate, the

layers separated and the aqueous extracted with several times with a suitable organic solvent, such as methylene chloride. The organic extracts are combined, dried over anhydrous sodium sulfate, filtered and concentrated under vacuum. The residue is then purified by flash chromatography on silica gel with a suitable eluent such as ethyl acetate/hexane to provide the sulfonamide (2).

In Scheme I, step B, the sulfonamide (2) is oxidized under conditions well known in the art, to provide the ketone of structure (3). For example, compound (2) is combined with about 1.1 to 1.5 equivalents of a suitable oxidizing agent, such as pyridinium chlorochromate in a suitable organic solvent, such as methylene chloride. The reaction is stirred at room temperature for about 2 to 8 hours and then filtered through Celite. The filtrate is washed with water, dried over anhydrous potassium carbonate, filtered, and concentrated to provide the crude ketone (3). This crude material can then be purified by chromatography on silica gel with a suitable eluent, such as methylene chloride/ethyl acetate to provide the purified ketone (3). In addition, the Swem Oxidation, which is well known in the art, is another example of oxidizing conditions that may be utilized to provide the ketone (3). See for example Jerry March, "*Advanced Organic Chemistry: Reactions, Mechanisms, and Structure,"* Fourth Edition, John Wiley & Sons, (1992) pages 1193-1194.

In Scheme I, step C, the ketone (3) is treated with a Grignard reagent prepared from the halide of structure (4) under standard conditions to provide the alcohol of structure (5), for example see Jerry March, "*Advanced Organic Chemistry: Reactions, Mechanisms, and Structure*," Fourth Edition, John Wiley & Sons, (1992) pages 920-929. In Scheme I, Hal represents Cl or Br in structure (4). More specifically, for example, magnesium turnings are added to a suitable organic solvent, such as anhydrous THF under an atmosphere of nitrogen. A small amount of ketone (3) is added along with an iodine crystal and a catalytic amount of dibromoethane. The reaction is heated with vigorous stirring to initiate the Grignard formation. A total of about 1.1 equivalents of compound (4) is added dropwise to the reaction. The reaction is then allowed to cool to room temperature and about 1.06 equivalents of ketone (3) is added dropwise to the reaction. The reaction mixture is then heated at reflux for about 2 hours, it is then allowed to cool and stir at room temperature for about 4 to 12 hours. The alcohol (5) is then isolated using standard techniques well known in the art. Saturated aqueous ammonium chloride is then added to precipitate salts and the organic layer is decanted. The remaining salts are washed with ether, the organic layer and washes are combined and concentrated under vacuum. The crude residue is taken up in a suitable organic solvent, such as ethyl acetate, washed with water, dried over anhydrous potassium carbonate, filtered, and concentrated under vacuum to provide the crude alcohol (5). The crude alcohol (5) can then be purified by chromatography on silica gel with a suitable eluent, such as methylene chloride/ethyl acetate to provide purified alcohol (5).

In Scheme I, step D, the alcohol (5) is dehydrated under conditions well known to one of ordinary skill in the art to provide the cycloalkenylsulfonamide of formula I. For example, the alcohol (5) is treated with an excess of DAST in a suitable organic solvent, such as methylene chloride at about -78°C. The reaction mixture is then allowed to warm to room temperature and diluted with methylene chloride. The organic layer is washed with water, dried over anhydrous sodium sulfate, filtered, and concentrated under vacuum to provide the crude cycloalkenylsulfonamide of formula I. This compound can then be purified by chromatography on silica gel with a suitable eluent, such as methylene chloride/ethyl acetate to provide the purified compound of formula I.

Scheme Ia provides an alternative procedure for the preparation of compounds of formula I. The reagents and starting materials are readily available to one of ordinary skill in the art. All substituents, unless otherwise specified are as previously defined.

## Scheme Ia

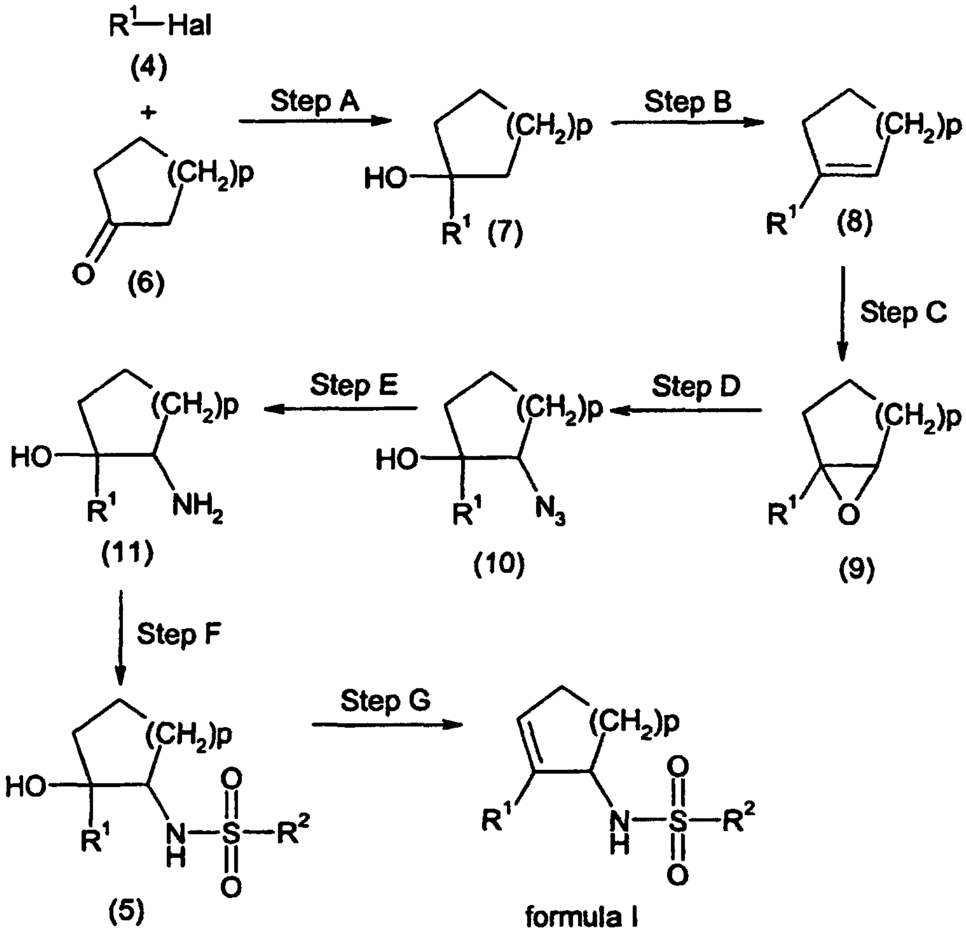


(4) (6) (7) (8) (9) (10) (11) (5) formula I

In Scheme Ia, step A, the halide of structure (4) is combined with the ketone of structure (6) under standard Grignard reaction conditions in a manner analogous to the procedure described in Scheme I, step C to provide the alcohol of structure (7).

In Scheme Ia, step B the alcohol (7) is dehydrated under standard conditions to provide the compound (8). For example, the alcohol (7) is dissolved in a suitable organic solvent, such as toluene and treated with p-toluenesulfonic acid. The reaction mixture is heated at reflux for about 4 hours and water is removed using a Dean-Stark trap. The reaction mixture is then allowed to cool to room temperature and concentrated under vacuum. The crude residue is taken up in a suitable organic solvent, such as methylene chloride, washed with water, dried over potassium carbonate, filtered, and concentrated under vacuum. The crude product (8) can be purified by chromatography on silica gel with a suitable eluent, such as hexane/methylene chloride.

In Scheme Ia, step C the compound (8) is epoxidized under conditions well known in the art to provide the epoxide (9). For example, about 3 equivalents m-chloroperbenzoic acid is added portionwise to about 5 equivalents of sodium fluoride in a suitable organic solvent, such as methylene chloride. The reaction mixture is allowed to stir for about 30 minutes at room temperature and compound (8) is added to the mixture in one portion. The reaction mixture is then allowed to stir for about 2 to 4 hours at room temperature, and then is filtered. The filtrate is washed with 1 N sodium hydroxide, water, dried over potassium carbonate, filtered, and concentrated under vacuum to provide the crude epoxide (9). This crude material can be purified using silica gel chromatography with a suitable eluent, such as hexane/methylene chloride to provide the purified epoxide (9).

In Scheme Ia step D, the epoxide (9) is opened under standard conditions to provide the azide (10). For example, 1.08 equivalents of epoxide (9) dissolved in a suitable organic solvent, such as DMF, is added dropwise at room temperature to a mixture of sodium azide in water. The reaction is heated at 90°C with stirring for about 8 to 12 hours. The mixture is then poured into water and extracted with a suitable organic solvent, such as ether. The organic layer is washed with water, dried over potassium carbonate, filtered, and concentrated under vacuum to provide the azide (10).

In Scheme Ia, step E, the azide (10) is converted to the amine (11) under conditions well known in the art. For example, azide (10) is dissolved in a suitable organic solvent, such as toluene and added dropwise to a suitable reducing agent, such as Red-Al® (available from Aldrich Chemical Company, Milwaukee, Wisconsin) under a nitrogen atmosphere at room temperature. The reaction mixture is allowed to stir for 1 to 4 hours and then is poured into water. The aqueous is extracted with a suitable organic solvent, such as ethyl acetate. The organic extracts are combined, rinsed with water, dried over anhydrous potassium carbonate, filtered, and concentrated under vacuum to provide the crude amine (11). This crude material can be purified by chromatography on silica gel with a suitable eluent, such as methylene chloride/methanol to provide purified amine (11).

In Scheme Ia step F, the amine (11) is converted to the sulfonamide of compound (5) in a manner analogous to the procedure described in Scheme I, step A.

In Scheme Ia, step G, the sulfonamide (5) is dehydrated in a manner analogous to the procedure described in Scheme I, step D to provide the compound of formula I.

Compounds of formula Ia and Ib can be prepared following the procedure set forth in Scheme II. The reagents and starting materials are readily available to one of ordinary skill in the art. All substituents, unless otherwise specified are as previously defined.

## Scheme II

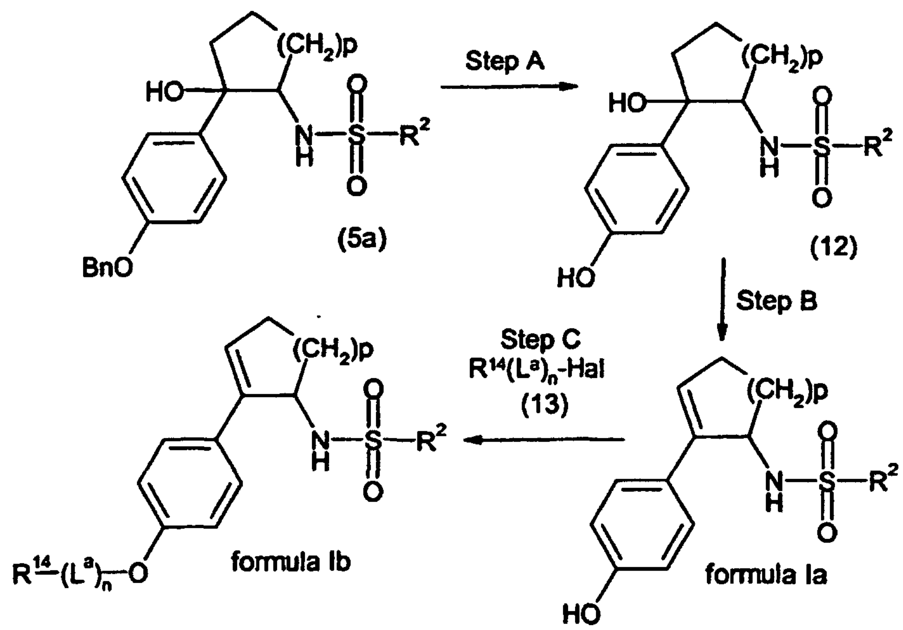


(5a)

(12)

formula Ia

formula Ib

In Scheme II, step A the compound of structure (5a) wherein "Bn" represents a benzyl group is converted to the compound of structure (12) under standard conditions. For example, compound (5a) is dissolved in a suitable organic solvent, such as ethyl acetate and treated with a catalytic amount of a hydrogenation catalyst, such as 5% palladium on carbon. The reaction mixture is placed under a hydrogen atmosphere at about 45 psi for about 2 to 6 hours with shaking. The reaction mixture is then filtered and the filtrate is concentrated under vacuum to provide the crude compound (12). This crude material can then be purified by chromatography on silica gel with a suitable eluent, such as methylene chloride/methanol to provide the purified compound (12).

In Scheme II, step B the compound (12) is dehydrated in a manner analogous to the procedure described in Scheme I, step D to provide the compound of formula Ia.

In Scheme II, step C, the compound of formula Ia is alkylated with a compound of structure (13) under conditions well known in the art to provide the compound of formula Ib. For example, compound of formula Ia is combined with about 1.1 equivalents of compound (13) and about 1.2 equivalents of a suitable base, such as potassium carbonate in a suitable organic solvent, such as acetone. The reaction mixture is then stirred at room temperature for about 4 to 12 hours. The reaction mixture is then filtered and the filtrate is concentrated to provide the crude compound of formula Ib. This crude material can then be purified by chromatography on silica gel with a suitable eluent, such as methylene chloride/ethyl acetate to provide the purified compound of formula Ib.

## Scheme III

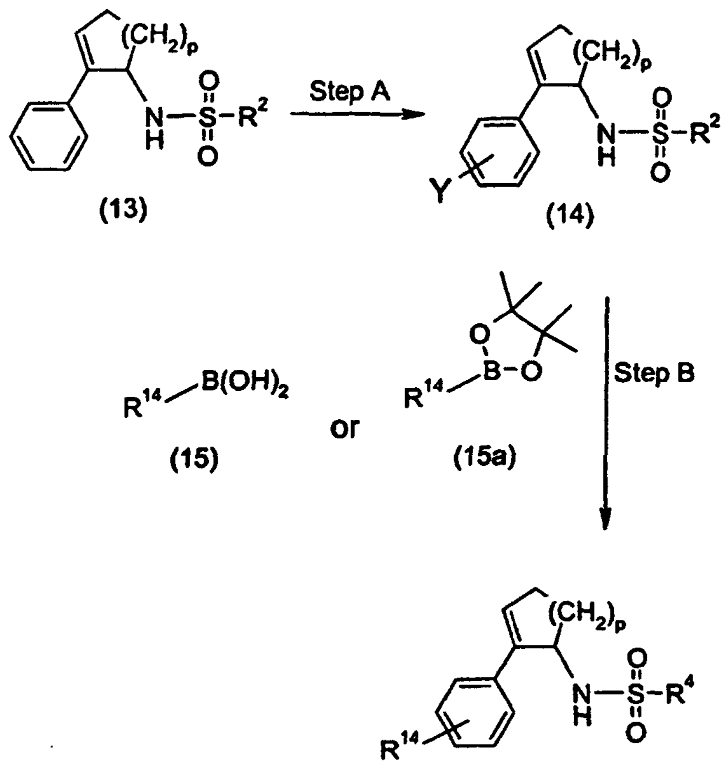


(13) (14)

(15) or (15a)

Y represents Br or I.

formula Ic

In Scheme III, step A, the compound of structure (13) can be converted to the compound of structure (14) wherein Y represents iodine. For example, compound of formula I is dissolved in a suitable solvent, such as glacial acetic acid. To this solution is added concentrated sulfuric acid followed by about 0.5 equivalents of iodine and about 0.4 equivalents of diiodine pentoxide. The reaction mixture is protected from light and heated at about 90°C for about 22 hours. The reaction mixture is then treated slowly with 10% aqueous sodium bisulfite, cooled to 0°C for about one hour and the precipitate collected by filtration. The precipitate is dissolved in a suitable organic solvent, such as warm diethyl ether and then washed with water, saturated sodium bicarbonate, dried over anhydrous sodium sulfate, filtered and concentrated under vacuum to provide the iodo compound of structure (14). Alternatively, compounds wherein Y represents Br are also readily prepared by one of ordinary skill in the art following known techniques and procedures.

In Scheme II, step B, compound (14), wherein Y represents Br or I, may be converted into compounds of formula Ic under conditions well known in the art, such as by reaction with an appropriate boronic acid (15) or boronic ester (15a) wherein $R^{14}$ represents an unsubstituted or substituted phenyl or heteroaromatic group as defined hereinabove with an unsubstituted or substituted phenyl being preferred. See for example, International Publication Number WO 98/33496, published August 6, 1998, the disclosure of which is hereby incorporated by reference. In addition, see Suzuki, A., *Journal of Organometallic Chemistry*, 576, 147-168 (1999), and Miyaura and Suzuki, *Chemical Reviews*, 95, 2457-2483 (1995) for examples of Suzuki-type coupling reactions and conditions. More specifically, the reaction is conveniently performed in the presence of a tetrakis (fiarylphosphine)palladium(0) catalyst, such as tetrakis (triphenylphosphine)palladium(0) and a base such as potassium carbonate. Suitable solvents for the reaction include aromatic hydrocarbons, such as toluene. The temperature at which the reaction is conducted is conveniently in the range of from 0 to 150°C, preferably 75 to 120°C. Alternatively, the coupling reaction may be carried out using palladium diacetate with a suitable organic solvent, such as n-propanol or acetone. See for example, *Organic Synthesis* 1998, *75*, 61; Goodson, F. E.; Wallow, T. I.; Novak, B. M. and *Organic Synthesis* 1998, *75*, 53; Huff, B. E.; Koenig, T. M.; Mitchell, D.; Staszak, M. A. wherein analogous coupling conditions are employed.

More specifically, for example, compound (14) and about 1.2 equivalents of boronic acid (15) or boronic ester (15a) are dissolved in a suitable organic solvent, such as n-propanol. About 1.2 equivalents of potassium carbonate in water is added, followed by a catalytic amount of palladium acetate. The reaction mixture is heated at reflux for about 4 hours, filtered and the solid rinsed with ethyl acetate. The filtrates are combined, diluted with additional ethyl acetate,

and washed with saturated sodium bicarbonate and brine. The organic phase is then dried over anhydrous sodium sulfate, filtered, and concentrated under vacuum to provide the crude compound of formula Ic. This crude material can then be purified by techniques well known in the art, such as flash chromatography or radial chromatography on silica gel with a suitable eluent, such as methylene chloride/ethyl acetate or reverse phase chromatography on a C18 column with a suitable eluent, such as aqueous acetonitrile.

Alternatively, compounds of formula Ic'

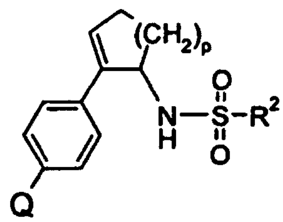

formula Ic'

wherein Q represents a bromo or triflate group may be coupled to boronic acid (15) in a manner analogous to the procedures set forth above in Scheme III, step B.

The boronic acid (15) used as a starting material may be prepared by reacting a trialkyl borate, such as triisopropyl borate with an appropriate organolithium compound at reduced temperature. For example, 2-fluorobenzeneboronic acid may be prepared by reacting 2-fluorobromobenzene with butyllithium in tetrahydrofuran at about -78°C to afford 2-fluorophenyl lithium, and then reacting this organolithium compound with triisopropyl borate. This is followed by hydrolysis with aqueous HCl.

Scheme IIIA

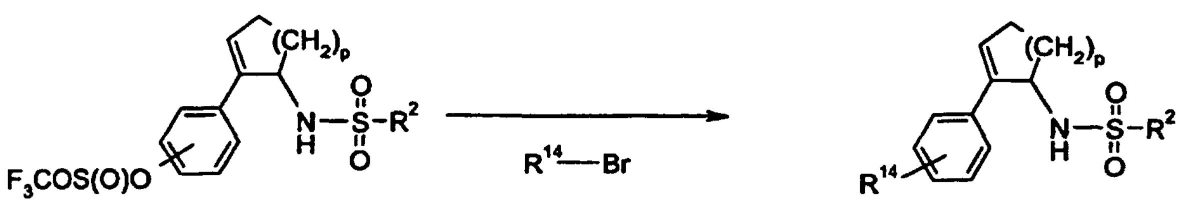

(16) (15b) formula Ic

Alternatively, compounds of formula Ic can be prepared under Suzuki-type reaction conditions as appreciated by one of ordinary skill in the art, from a compound of structure (16) and a compound of structure (15b). For example, the compound (16) is dissolved in a suitable organic solvent, such as DMSO and treated with about 3 equivalents of potassium acetate. The reaction mixture is degassed and treated with about 1.1 equivalents of bis(pinacolato)diboron followed by addition of a catalytic amount of a suitable palladium catalyst, such as [1,1'-bis(diphenylphosphino)-ferrocene] dichloropalladium (II), complex with dichloromethane 1:1. The reaction mixture is then heated at about 80°C under nitrogen with stirring for about 1 to about 4 hours. The reaction mixture is then cooled to room temperature and about one equivalent of the compound (15b) is added followed by addition of about 3 equivalents of sodium carbonate, water, and a catalytic amount of a suitable palladium catalyst, such as [1,1'-bis(diphenylphosphino)-ferrocene] dichloropalladium (II), complex with dichloromethane 1:1. The reaction mixture is then heat at about 105°C for about 10 to about 20 hours. It is then allowed to cool and is diluted with a suitable organic solvent, such as methylene chloride. The mixture is washed with water, brine, dried over anhydrous magnesium sulfate, filtered and concentrated under vacuum to provide the crude compound formula Ic. This crude material can then be purified by techniques well known in the art, such as chromatography on silica gel with a suitable eluent, such as 1% methanol/methylene chloride to provide the purified compound of formula Ic.

The following examples further illustrate the invention and represent typical syntheses of the compounds of formula I as described generally above. The reagents and starting materials are readily available to one of ordinary skill in the art. As used herein the term "Chromatotron®" (Harrison Research Inc., 840 Moana Court, Palo Alto California 94306) is recognized by one of ordinary skill in the art as an instrument which is used to perform centrifugal thin-layer chromatography. As used herein, the following terms have the meanings indicated: "eq" refers to equivalents; "g" refers

to grams; "mg" refers to milligrams; "L" refers to liters; "mL" refers to milliliters; "μL" refers to microliters; "mol" refers to moles; "mmol" refers to millimoles; "psi" refers to pounds per square inch; "min" refers to minutes; "h" or "hr" refers to hours; "°C" refers to degrees Celsius; "TLC" refers to thin layer chromatography; "HPLC" refers to high performance liquid chromatography; "$R_f$" refers to retention factor; "$R_t$" refers to retention time; "δ"refers to part per million downfield from tetramethylsilane; "THF" refers to tetrahydrofuran; "DMF" refers to N,N-dimethylformamide; "DMSO" refers to methyl sulfoxide; "LDA" refers to lithium diisopropylamide; "EtOAc" refers to ethyl acetate; "aq" refers to aqueous; "iPrOAc" refers to isopropyl acetate; "MTBE" refers to tert-butyl methyl ether; "methyl DAST" refers to dimethylaminosulfur trifluoride, "DAST" refers to diethylaminosulfur trifluoride, "DBU" refers to 1,8-diazabicyclo[5.4.0]undec-7-ene; as used herein "$Pd(dppf)_2Cl_2$ catalyst" refers to ([1,1'-bis(diphenylphosphino)ferrocene]dichloropalladium(II) complex with $CH_2Cl_2$; as used herein the terms "Me", "Et", "Pr", "iPr", and "Bu" refer to methyl, ethyl, propyl, isopropyl, and butyl respectively, and "RT" refers to room temperature.

Example 1

Preparation of [2-(4-hydroxyphenyl)cyclohex-2-enyl][(methylethyl)sulfonyl]amine.

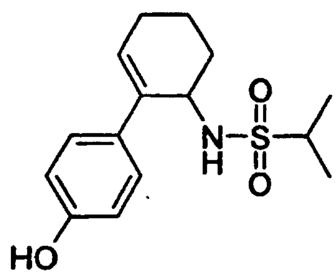

Preparation of (2-hydroxycyclohexyl)[(methylethyl)sulfonyl]amine.

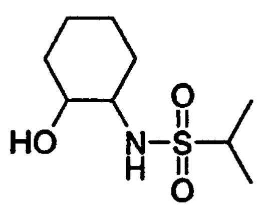

Scheme I, step A: In a 1L-3 neck flask fitted with a stirrer and thermometer, propanesulfonyl chloride (31.26 g, 1.1 eq) is added dropwise to 2-aminocyclohexanol (23.04 g, 200 mmol) and DBU (45.63 g, 1.15 eq) in methylene chloride (500 mL) while stirring at 0°C under a nitrogen atmosphere. The reaction is allowed to warm to room temperature and stirred overnight at this temperature. The reaction is then diluted with methylene chloride (500 mL) and the organic layer is washed two times with water, dried over anhydrous sodium sulfate, filtered, and concentrated under reduced vacuum to yield 61 g of a viscous oil. This two spot material is purified via silica gel chromatography employing a Prep 2000 and eluting with a solvent of methylene chloride/ethyl acetate 4:1 to yield the intermediate title compound (8.60 g, 19%, bottom spot by TLC) as a white solid. Ion spray M.S. 220 (M* - 1).

| Calculated for: $C_9H_{19}NO_3S$: | | | |
|---|---|---|---|
| Theory | C 48.84, | H 8.65, | N 6.33. |
| Found | C 48.79, | H 8.51, | N 6.51. |

Preparation of 2-{[(methylethyl)sulfonyl]amino}cyclohexan-1-one.

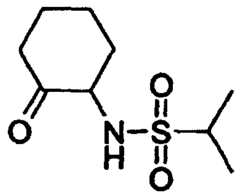

Scheme I, step B: Into a flame dried 500 mL 3 neck flask fitted with a thermometer and magnetic stirrer, DMSO (5.50 mL) in methylene chloride (30 mL) is added dropwise to oxalyl chloride (2.50 mL) in methylene chloride (80 mL) while stirring at -55°C under a nitrogen atmosphere. After 2 minutes, (2-hydroxycyclohexyl)[(methylethyl)sulfonyl]amine (7.53 g, 34 mmol) in methylene chloride (30 mL) is added dropwise at this temperature and the reaction is stirred for an additional 15 minutes. Triethylamine (23.0 mL) is then added dropwise and the reaction was allowed to warm to room temperature. Water (160 mL) is added at room temperature and the layers are separated. The organic layer is washed once with water, dried over potassium carbonate, filtered, and concentrated under reduced vacuum to yield 7.41 g as an oil. This one spot material is purified via silica gel chromatography employing a Prep 2000 and eluting with a solvent of methylene chloride/ethyl acetate 19:1 to yield the intermediate title compound (6.14 g, 82%) as a white solid. Ion spray M.S. 218 (M* - 1).

| Calculated for: $C_9H_{17}NO_3S$: | | | |
|---|---|---|---|
| Theory | C 49.29, | H 7.81, | N 6.39. |
| Found | C 49.25, | H 7.58, | N 6.32. |

Preparation of [2-hydroxy-2-(4-methoxyphenyl)cyclohexyl][(methylethyl)sulfonyl]amine.

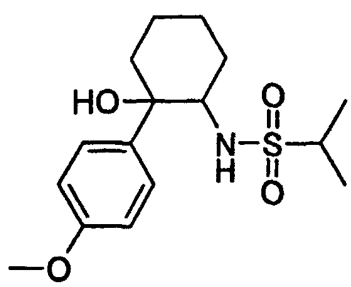

Scheme I, step C: Into a flame dried 250 mL 3 neckflask that is fitted with a thermometer and condenser, and while stirring at room temperature under a nitrogen atmosphere, 2-{[(methylethyl)sulfonyl]amino}cyclohexan-1-one (2.90 g, 13.2 mmol) in THF (50 mL) is added dropwise to 4-methoxyphenylmagnesium bromide (35 mL, 17.5 mmol). The addition of 2-{[(methylethyl)sulfonyl]amino}cyclohexan-1-one is continued dropwise, keeping the temperature above 35°C. After the addition of 2-{[(methylethyl)sulfonyl]amino}cyclohexan-1-one is complete, the reaction is stirred overnight at room temperature. Enough saturated ammonium chloride in water is then added to precipitate salts and the organic layer is decanted off.
The remaining salts are washed two times with ether and the combined organic layers are concentrated under reduced vacuum. The resulting semi-solid is taken into ethyl acetate, washed once with water, dried over potassium carbonate, filtered, and concentrated under reduced vacuum to yield 3.02 g as a dark semi-solid. This material is purified via silica gel chromatography employing a Water's Prep 2000 and eluting with a gradient solvent of methylene chloride/ethyl acetate 19:1 to methylene chloride/ethyl acetate 9:1 to provide the intermediate title compound (1.03 g, 18%) as a white solid. Ion Spray M.S. 328.1 (M*- 1).

| Calculated for $C_{16}H_{25}$ $NO_4$ S-1/2$H_2O$: | | | |
|---|---|---|---|
| Theory | C 57.11, | H7.74, | N 4.16. |
| Found | C 57.69, | H 7.49, | N 3.76. |

Preparation of the final title compound.

Scheme I, step D: Into a 100 mL-3 neck flask fitted with a magnetic stirrer and thermometer, boron tribromide (0.5 mL, 3 eq) in methylene chloride (5 mL) is added dropwise to [2-hydroxy-2-(4-methoxyphenyl)cyclohexyl][(methylethyl)sulfonyl]amine (500 mg, 1.52 mmol) in methylene chloride (25 mL) while stirring at room temperature under a nitrogen atmosphere. (exotherm) After stirring for one hour at this temperature, water (10 mL) is then added slowly and the resulting layers are separated. The organic layer is washed with water, dried over anhydrous sodium sulfate, filtered, and concentrated under reduced vacuum to yield 410 mg of material as an oil. This material is then purified via silica gel chromatography employing the Chromatotron® and using a 4000 micron rotor while eluting with a solvent of methylene chloride/ethyl acetate 9:1 to provide the final title compound (260 mg, 55%) as a white solid. Fd M.S. 295.1 (M*).

| Calculated for $C_{15}H_{21}NO_3S$-1/2 $H_2O$ : | | | |
|---|---|---|---|
| Theory | C 59.16, | H 7.28, | N 4.60. |
| Found | C 58.64, | H 6.90, | N 4.62. |

Example 2

Preparation of [(methylethyl)sulfonyl](2-{4-[(trifluoromethoxy)sulfinyloxy]phenyl}cyclohex-2-enyl)amine.

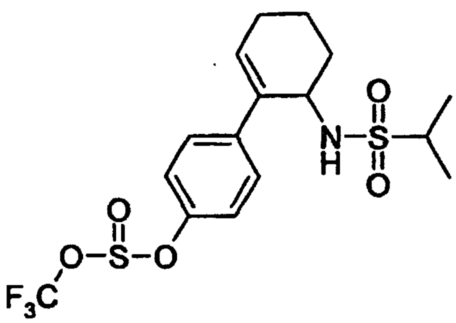

Into a 100 mL-3 neck flask fitted with a stirrer and thermometer, trifluoromethanesulfonic anhydride (0.44 mL, excess, triflic anhydride) is added syringe-wise to [2-(4-hydroxyphenyl)cyclohex-2-enyl][(methylethyl)sulfonyl]amine (500 mg, 1.69 mmol, prepared in example 1) and pyridine (2.0 mL) in THF (20 mL) while stirring at 0°C under a nitrogen atmosphere. The reaction is allowed to warm to room temperature and stirred overnight. The reaction mixture is then poured into water and the desired material is extracted into ethyl acetate. The organic layer is washed once with water, dried over potassium carbonate, filtered, and concentrated under reduced vacuum to yield 660 mg as an orange oil. This material is purified via silica gel chromatography employing the Chromatotron® using a 4000 micron rotor and eluting with a gradient solvent of methylene chloride to methylene chloride/ethyl acetate 1:1 to provide the title compound (250 mg, 35%) as a yellow solid. Ion spray M.S. 426.2 (M*-1).

| Calculated for $C_{16}H_{20}NO_5S_2F_3$: | | | |
|---|---|---|---|
| Theory | C 44.96, | H 4.72, | N 3.28. |
| Found | C 45.26, | H 4.66, | N 3.05. |

Example 3

Preparation of (2-{4-[(3,5-difluorophenyl)methoxy]phenyl}cyclohex-2-enyl)[(methylethyl)sulfonyl]amine.

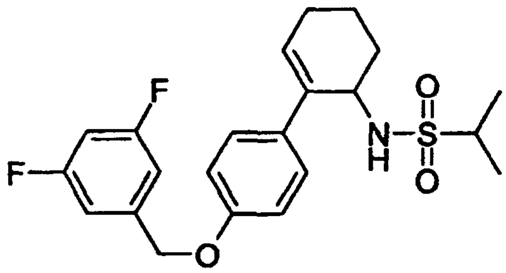

Scheme II, step C: [2-(4-Hydroxyphenyl)cyclohex-2-enyl][(methylethyl)sulfonyl]amine (150 mg, 0.51 mmol, prepared in example 1), 3,5-difluorobenzyl bromide (116 mg, 1.1 eq), and potassium carbonate (85 mg, 1.2 eq) are combined in acetone (20 mL) and stirred overnight at room temperature under a nitrogen atmosphere. In the morning, the solution is filtered and the filtrate is concentrated under reduced vacuum to yield 243 mg as a brown oil. This material is then purified via silica gel chromatography employing the Chromatotron® using a 4000 micron rotor while eluting with a gradient solvent of methylene chloride to methylene chloride/ethyl acetate 4:1 to provide the title compound (125 mg, 58%) as a slowly crystallizing oil. Ion spray M.S. 420.3 (M* - 1).

| Calculated for $C_{22}H_{25}NO_3SF_2$ : | | | |
|---|---|---|---|
| Theory | C 62.69, | H 5.98, | N 3.32. |
| Found | C 62.89, | H 5.97, | N 3.10. |

Example 4

Preparation of 2-{[4-(6-{[(methylethyl)sulfonyl]amino}cyclohex-1-enyl)phenoxy]methyl}benzenecarbonitrile.

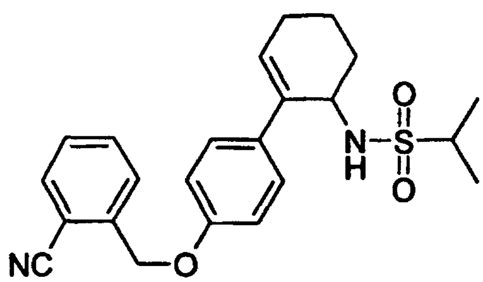

Scheme II, step C: [2-(4-Hydroxyphenyl)cyclohex-2-enyl][(methylethyl)sulfonyl]amine (150 mg, 0.51 mmol), 2-cyanobenzyl bromide (102 mg, 1.1 eq), and potassium carbonate (85 mg, 1.2 eq) are combined with acetone (20 mL) and stirred overnight at room temperature under a nitrogen atmosphere. In the morning, the solution is filtered and the filtrate is concentrated under reduced vacuum to yield 245 mg as an oil. This material is then purified via silica gel chromatography employing the Chromatotron® using a 4000 micron rotor while eluting with a gradient solvent of methylene chloride to methylene chloride/ethyl acetate 4:1 to provide the title compound (90 mg, 44%) as a white solid. Ion spray M.S. 409.3 (M* - 1).

| Calculated for $C_{23}H_{26}N_2O_3S$ : | | | |
|---|---|---|---|
| Theory | C 67.30, | H 6.38, | N 6.82. |
| Found | C 67.80, | H 6.64, | N 6.38. |

Example 5

Preparation of [(methylethyl)sulfonyl]{2-[4-(phenylmethoxy)phenyl]cyclopent-2-enyl}amine.

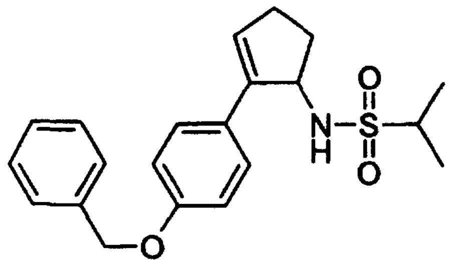

Preparation of [(methylethyl)sulfonyl][(2-(phenylmethoxy)cyclopentyl]amine.

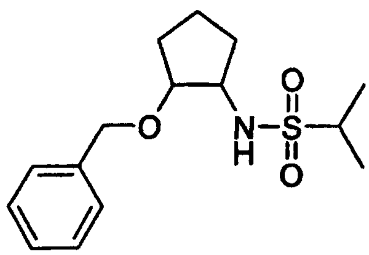

In a 250 mL-3 neck flask fitted with a stirrer and thermometer, isopropylsulfonyl chloride (4.47 g, 1.2 eq) is added dropwise to (1S,2S)-2-benzyloxycyclopentylamine (5.00 g, 10 mmol) and DBU (5.98 g, 1.5 eq) in methylene chloride (100 mL) while stirring at 0°C under a nitrogen atmosphere. The reaction is allowed to warm to room temperature and stirred overnight at this temperature. In the morning, the reaction is diluted with methylene chloride (100 mL) and the organic layer is washed two times with water, dried over anhydrous sodium sulfate, filtered, and concentrated under reduced vacuum to yield 10.13 g as a viscous oil. This material is purified via silica gel chromatography employing the Water's Prep. 2000 and eluting with a solvent of hexanelethyle acetate 4:1 to provide the intermediate title compound (8.0 g, 95%) as a white solid. Ion spray M.S. 296 (M* - 1).

| Analysis calculated for $C_{15}H_{23}NO_3S$: | | | |
|---|---|---|---|
| Theory | C 60.58, | H 7.80, | N 4.71 |
| Found | C 60.39, | H 7.79, | N 4.73 |

Preparation of (2-hydroxycyclopentyl)[(methylethyl)sulfonyl]amine.

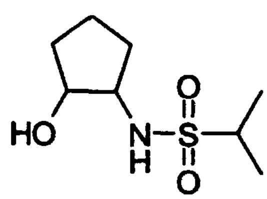

[(Methylethyl)sulfonyl][2-(phenylmethoxy)cyclopentyl]amine (2.00 g, 6.72 mmol) and palladium on carbon (250 mg, 1.1 eq) are combined with ethanol (50 mL) and placed on the power shaker under a hydrogen atmosphere

at 60 psi's at room temperature.. After about 10 hours the solution is filtered over a Celite mat and the resulting filtrate is concentrated under reduced vacuum to yield the intermediate title compound (1.50,g) as a viscous oil. Ion spray M. S. 206 (M* - 1).

| Analysis calculated for $C_8H_{17}NO_3S$: | | | |
|---|---|---|---|
| Theory | C 46.35, | H 8.26, | N 6.75 |
| Found | C 46.27, | H 7.97, | N 6.70 |

Preparation of 2-{[(methylethyl)sulfonyl]amino}cyclopentan-1-one.

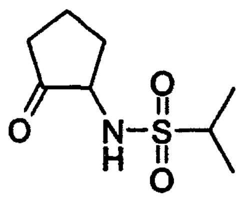

Scheme I, step B: Into a 250 mL single neck flask, (2-hydroxycyclopentyl)[(methylethyl)sulfonyl]amine (1.50g, 7.24 mmol) and pyridinium chlorochromate (2.34 g, 1.5 eq) are combined with methylene chloride (100 mL) and stirred for 4 hours at room temperature under a nitrogen atmosphere. The solution is then filtered over a Celite mat and the resulting filtrate is washed once with water, dried over potassium carbonate, filtered, and concentrated under reduced vacuum to yield 1.31 g as an oil. This material is purified via silica gel chromatography employing the Chromatotron® and using a 4000 micron rotor while eluting with a solvent of methylene chloride/ethyl acetate 4:1 to the intermediate title compound (450 mg, 30%) as a slowly crystallizing oil. Ion spray M.S. 204 (M* - 1).

| Analysis calculated for $C_8H_{15}NO_3S$: | | | |
|---|---|---|---|
| Theory | C 46.81, | H 7.37, | N 6.82 |
| Found | C 46.08, | H 7.12, | N 6.58 |

Alternative preparation of 2-{[(methylethyl)sulfonyl]amino}cyclopentan-1-one.

Scheme I, step B: Into a flame dried 500 mL 3 neck flask fitted with a thermometer and magnetic stirrer, 6.16 mL of DMSO (6.16 mL) in methylene chloride (20 mL) is added dropwise to oxalyl chloride (3.80 mL) in methylene chloride (100 mL) while stirring at -55°C under a nitrogen atmosphere. After 2 minutes, (2-hydroxycyclopentyl)[(methylethyl)sulfonyl]amine (8.00 g, 38.6 mmol) in methylene chloride (45 mL) is added dropwise at this temperature and the reaction is stirred for an additional 15 minutes. Then triethylamine (25.5 mL) is added dropwise and the reaction is allowed to warm to room temperature. Water (180 mL) is then added at room temperature and the layers are separated. The organic layer is then washed once with water, dried over potassium carbonate, filtered, and concentrated under reduced vacuum to yield 7.83 g as a dark oil. This material is purified via silica gel chromatography employing a Water's Prep. 2000 and eluting with a solvent of methylene chloride/ethyl acetate 9:1 to provide (5.76 g, 73%) as a yellow oil. Ion Spray M.S. 204.1 (M* - 1).

| Calculated for $C_8H_{15}NO_3S$: | | | |
|---|---|---|---|
| Theory | C 46.81, | H 7.37, | N 6.82. |
| Found | C 46.56, | H 7.32, | N 6.77. |

Preparation of [{2-hydroxy-2-[4-(phenylmethoxy)phenyl]cyclopentyl}[(methylethyl)sulfonyl]amine.

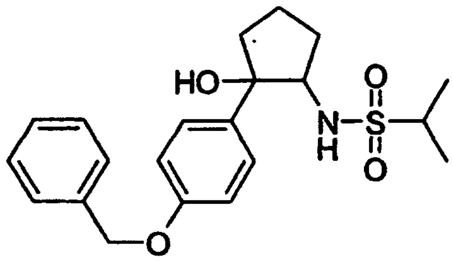

Into a flame dried 500 mL 3 neck flask that is fitted with a thermometer and condenser, magnesuim turnings (899 mg, 38 mmol) are placed in anhydrous THF (15 mL). While stirring at room temperature under a nitrogen atmosphere, a small amount of 4-benzyloxyphenyl bromide in THF (100 mL) is added dropwise along with one iodine crystal and .01 mL of dibromoethane. This mixture is stirred vigorously and heated with a heat gun until the grignard is initiated as foaming is observed from metal turnings. The addition of 4-benzyloxyphenyl bromide (10.54 g, 40 mmol) is continued dropwise, keeping the temperature above 50°C. After the addition of 4-benzyloxyphenyl bromide is complete, the reaction is heated at reflux for 45 minutes to insure complete grignard formation. The reaction is allowed to cool to room temperature. Then 2-{[(methylethyl)sulfonyl]amino}cyclopentan-1-one (5.70 g, 27.8 mmol) in THF (35 mL) is added dropwise. After addition, the reaction is refluxed for 2 hours and then stirred overnight at room temperature. In the morning, enough saturated ammonium chloride in water is added to precipitate salts and the organic layer is decanted off. The remaining salts are washed two times with ether and the combined organic layers are concentrated under reduced vacuum. The resulting material is taken into ethyl acetate, washed once with water, dried over potassium carbonate, filtered, and concentrated under reduced vacuum to yield 11.51 g of 2 spot material as a dark oil. This isomeric mixture is purified via silica gel chromatography employing a Water's Prep 2000 while eluting with a gradient solvent of methylene chloride/ethyl acetate 19:1 to methylene chloride/ethyl acetate 9:1 to provide (1.35 g)) as an oil. Ion Spray M.S. 388.2 (M*-1).

Preparation of final title compound.

Scheme I, step D: Into a 50 mL 3 neck flask fitted with a stirrer and thermometer [{2-hydroxy-2-[4-(phenylmethoxy)phenyl]cyclopentyl}[(methylethyl)sulfonyl]amine (385 mg. 0.1 mmol) in methylene chloride (10 mL) is added dropwise to DAST (0.02 mL, excess) in methylene chloride (25 mL) while stirring at -78°C under a nitrogen atmosphere. The reaction is allowed to warm to room temperature and diluted with methylene chloride (20 mL). The organic layer is washed with water, dried over anhydrous sodium sulfate, and concentrated under reduced vacuum to yield 384 mg of a two spot mixture as an oil. This two spot material is purified via silica gel chromatography employing the Chromatotron® and using a 4000 micron rotor while eluting with a solvent of hexane/ethyl acetate 7:3 to provide the final title compound (141 mg, 38%) of the top spot as a semi-solid. Fd M.S. 371.2 (M*).

| Calculated for $C_{21}H_{25}NO_3S$: | | | |
|---|---|---|---|
| Theory | C 67.89, | H 6.78, | N 3.77. |
| Found | C 67.52, | H 6.68, | N 3.67. |

Example 6

Preparation of [2-(4-hydroxyphenyl)cyclopent-2-enyl][(methylethyl)sulfonyl]amine.

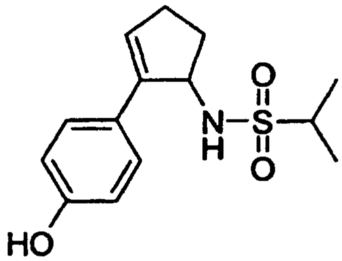

Preparation of [2-hydroxy-2-(4-hydroxyphenyl)cyclopentyl][(methylethyl)sulfonyl]amine.

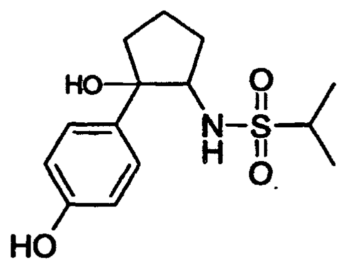

Scheme II, step A: [{2-Hydroxy-2-[4-(phenylmethoxy)phenyl]cyclopentyl}(methylethyl)sulfonyl]amine (1.44 g, 3.70 mmol, prepared in example 5) and 5% palladium on carbon (1.70 g) are combined with ethyl acetate (150 mL) and the mixture is placed on the power shaker under a hydrogen atmosphere at 45 psi's for 4 hours. The solution is then filtered over a Celite mat and the resulting filtrate is concentrated under reduced vacuum to yield 1.2 g as a white foam. This material is purified via silica gel chromatography employing the Chromatotron® using a 4000 micron rotor while eluting with a gradient solvent of methylene chloride/methanol 9:1 to methylene chloride/methanol 1:1 to provide the intermediate title compound (710 mg, 65%) as a white foam. Ion Spray M.S. 298.2 (M* - 1).

| Calculated for $C_{14}H_{21}NO_4S$: | | | |
|---|---|---|---|
| Theory | C 56.17, | H 7.07, | N 4.68. |
| Found | C 55.94, | H 6.98, | N 4.51. |

Preparation of final title compound.

Scheme II, step B: Into a 50 mL 3 neck flask fitted with a stirrer and thermometer, [2-hydroxy-2-(4-hydroxyphenyl)cyclopentyl][(methylethyl)sulfonyl]amine (150 mg, 0.5 mmol) in methylene chloride (3 mL) is added dropwise to DAST (0.1 mL, excess) in methylene chloride (7 mL) while stirring at -78°C under a nitrogen atmosphere. The reaction is then allowed to warm to room temperature and diluted with methylene chloride (20 mL). The organic layer is washed with water, dried over anhydrous sodium sulfate, and concentrated under reduced vacuum to yield 141 mg as a foam. This material is purified via silica gel chromatography employing the Chromatotron® and using a 2000 micron rotor while eluting with a solvent of methylene chloride/ethyl acetate 9:1 to provide the final title compound (41 mg, 29%) as an oil. Fd M.S. 281.2 (M*).

| Calculated for $C_{14}H_{19}NO_3S\text{-}1/2H_2O$: | | | |
|---|---|---|---|
| Theory | C 57.90, | H 6.94, | N 4.82. |
| Found | C 58.43, | H 6.80, | N 3.93. |

Example 7

Preparation of (2-{4-[(3,5-difluorophenyl)methoxy]phenyl}cyclopent-2-enyl)[(methylethyl)sulfonyl]amine.

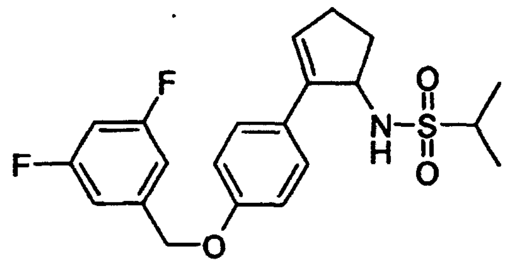

Preparation of (2-{4-[(3,5-difluorophenyl)methoxy]phenyl}-2-hydroxycyclopentyl)[(methylethyl)sulfonyl]amine.

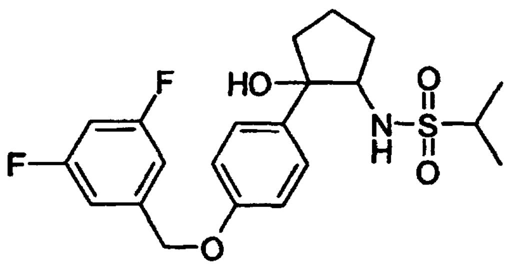

[2-Hydroxy-2-(4-hydroxyphenyl)cyclopentyl][(methylethyl)sulfonyl]amine (200 mg, 0.67 mmol, prepared in example 6), 3,5-difluorobenzyl bromide (153 mg, 1.1 eq) and potassium carbonate (111 mg, 1.2 eq) are combined with acetone (20 mL) and stirred overnight at room temperature under a nitrogen atmosphere.

In the morning, the solution is filtered and the filtrate is concentrated under reduced vacuum to yield 339 mg as a brown oil. This material is purified via silica gel chromatography employing the Chromatotron® using a 4000 micron rotor while eluting with a gradient solvent of methylene chloride to methylene chloride/ethyl acetate 9:1 to provide the intermediate title compound (233 mg, 82%) as an oil. Fd M.S. 425.3 (M*).

| Calculated for $C_{21}H_{25}NO_4$ $SF_2$-1/2 $H_2O$ : | | | |
|---|---|---|---|
| Theory | C 58.05, | H 6.03, | N 3.22. |
| Found | C 58.23, | H 5.80, | N 3.07. |

Preparation of final title compound.

Into a 50 mL 3 neck flask fitted with a stirrer and thermometer, (2-{4-[(3,5-difluorophenyl)methoxy]phenyl}-2-hydroxycyclopentyl)[(methylethyl)sulfonyl]amine (200 mg, 0.4 mmol) in methylene chloride (3 mL) is added dropwise to DAST (0.06 mL, excess) in methylene chloride (7 mL) while stirring at -78°C under a nitrogen atmosphere. The reaction is allowed to warm to room temperature and diluted with methylene chloride (20 mL). The organic layer is washed with water, dried over anhydrous sodium sulfate, filtered, and concentrated under reduced vacuum to yield 187 mg as a foam. This material is purified via silica gel chromatography employing the Chromatotron® and using a 2000 micron rotor while eluting with a solvent of hexane/ethyl acetate 7:3 to provide the final title compound (137 mg, 84%) as an oil. Fd M.S. 407.2 (M*).

| Calculated for $C_{21}H_{23}NO_3S$-1/2$H_2O$: | | | |
|---|---|---|---|
| Theory | C 60.55, | H 5.80, | N 3.36. |
| Found | C 59.89, | H 5.60, | N 3.37. |

Example 8

Preparation of [(methylethyl)sulfonyl][2-(4-{2-[(methylsulfonyl)amino]ethoxy}phenyl)cyclopent-2-enyl]amine.

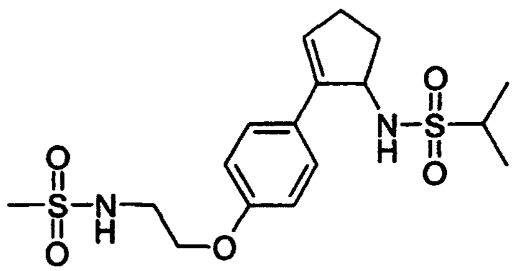

Preparation of 2-[4-(5-{[(methylethyl)sulfonyl]amino}cyclopent-1-enyl)phenoxy]ethanenitrile.

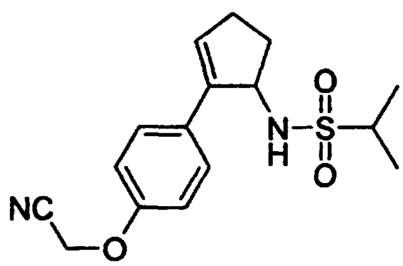

2-(4-Hydroxyphenyl)cyclopent-2-enyl][(methylethyl)sulfonyl]amine (335 mg, 1.19 mmol, prepared in example 6), bromoacetonitrile (156 mg, 1.1 eq) and potassium carbonate (200 mg, 1.2 eq) are combined in acetone (25 mL) and stirred for about 14 hours at room temperature under a nitrogen atmosphere. The solution is then filtered and the filtrate is concentrated under reduced vacuum to yield the crude product. This crude material can then be purified via silica gel chromatography employing the Chromatotron® using a 4000 micron rotor while eluting with a suitable eluent, such as hexane/ethyl acetate to yield the intermediate title compound.

Additional preparation of 2-[4-(5-{[(methylethyl)sulfonyl]amino}cyclopent-1-enyl)phenoxy]ethanenitrile.

2-(4-Hydroxyphenyl)cyclopent-2-enyl][(methylethyl)sulfonyl]amine (900 mg, 3.2 mmol, prepared in example 6), bromoacetonitrile (458 mg, 1.2 eq) and potassium carbonate (663 mg, 1.5 eq) are combined in acetone (90 mL) and stirred for about 14 hours at room temperature under a nitrogen atmosphere. The solution is then filtered and the filtrate is concentrated under reduced vacuum to yield the crude product (1.14 g) as a semi-solid. This crude material is then purified via silica gel chromatography employing a Prep. 2000 and 1 cartridge while eluting with a solvent of hexane/ethyl acetate (7:3) to yield the intermediate title compound (241 mg, 24% yield) as a solid. Ion spray M.S. 319.4 (M*-1).

| Calculated for $C_{16}$ $H_{20}$ $N_2$ $O_3$ S : | | | |
|---|---|---|---|
| Theory | C 59.98, | H 6.29, | N 8.74. |
| Found | C 60.13. | H 6.37, | N 8.65. |

Preparation of {2-[4-(2-aminoethoxy)phenyl]cyclopent-2-enyl}[(methylethyl)sulfonyl]amine.

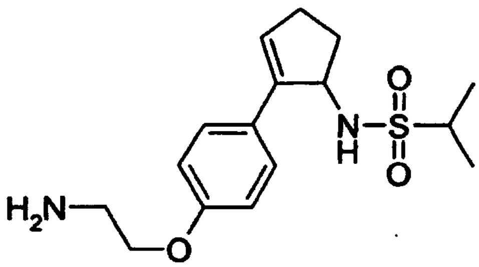

Into a 50 mL, 3 neck flask with stirrer, 2-[4-(5-{[(methylethyl)sulfonyl]amino}cyclopent-1-enyl)phenoxy] ethanenitrile (385 mg, 1.14 mmol) in toluene (15 mL) is added dropwise to Red-AL (3 mL) while stirring at room temperature under a nitrogen atmosphere. The reaction mixture is then stirred at room temperature for 2 hours. The mixture is poured into water and the desired material is extracted into ethyl acetate. The organic layer is washed once with water, dried over pottasium carbonate, filtered, and concentrated under reduced vacuum. This material can then be purified via silica gel chromatography employing the Chromatotron® and using a 2000 micron rotor while eluting with a suitable solvent, such as hexane/ethyl acetate to provide the intermediate title compound.

Additional preparation of {2-[4-(2-aminoethoxy)phenyl]cyclopent-2-enyl}[(methylethyl)sulfonyl]amine.

Into a 100 mL 3-neck flask fitted with a stirrer and thermometer, 2-[4-(5-{[(methylethyl)sulfonyl]amino}cyclopent-1-enyl)phenoxy]ethanenitrile (230 mg) in toluene (20 mL) is added dropwise to a stirred solution of 1.0 mL Red-Al (1 M in toluene) at room temperature under a nitrogen atmosphere. The reaction is heated at 60° C for two hours. The solution is allowed to cool to room temperature and poured into 50 mL 1N HCl. The acid media is washed once with ethyl acetate and then made basic with 5N NaOH. The desired amine is extracted into ethyl acetate. The organic layer is washed once with water, dried over potassium carbonate, filtered, and concentrated under reduced vacuum to yield 160 mg of crude material as an oil. This material is purified via silica gel chromatography employing a Chromatotron® and using a 1000 micron rotor while eluting with a solvent of methylene chloride/methanol (9:1) to provide the intermediate title compound (117 mg, 50% yield) as a gum. Ion spray M.S. 323.4 (M*-1).

Preparation of final title compound.

In a 50 mL 3 neck flask fitted with a stirrer and thermometer, methanesulfonyl chloride (77 mg, 1.1 eq) is added dropwise to {2-[4-(2-aminoethoxy)phenyl]cyclopent-2-enyl}[(methylethyl)sulfonyl]amine (202 mg, 0.59 mmol) and DBU (108 mg, 1.2 eq) in methylene chloride (25 mL) while stirring at 0°C under a nitrogen atmosphere. The reaction is allowed to warm to room temperature and is stirred for about 14 hours at this temperature. The reaction is then diluted with methylene chloride (30 mL) and the organic layer is washed two times with $H_2O$, dried over $Na_2SO_4$, filtered, and concentrated under reduced vacuum to yield the crude product. This material can be purified via silica gel chromatography employing the Chromatotron® using a 2000 micron rotor and eluting with a suitable eluent, such as hexane/ethyl acetate to provide the final title compound.

Additional preparation of final title compound.

In a 100 mL, 3 neck flask fitted with a stirrer and thermometer, methanesulfonyl chloride (38 mg, 1.2 eq) is added dropwise to {2-[4-(2-aminoethoxy)phenyl]cyclopent-2-enyl}[(methylethyl)sulfonyl]amine (90 mg, 0.28 mmol) and DBU (64 mg, 1.5 eq) in methylene chloride (25 mL) while stirring at 0°C under a nitrogen atomsphere. The reaction is allowed to warm to room temperature and stirred overnight at this temperature. In the morning, the reaction is diluted with methylene chloride (25 mL) and the organic layer is washed two times with water, dried over anhydrous sodium sulfate, filtered, and concentrated under reduced vacuum to yield 112 mg of crude material as a viscous oil. This material is purified via silica gel chromatography employing a Chromatotron® and using a 1000 micron rotor while eluting with a solvent of methylene chloride/ethyl acetate (9:1) to yield the final title compound (71 mg, 63% yield) as a white foam.
Ion spray M.S. 401.4 (M* - 1).

| Calculated for: $C_{17} H_{26} N_2 O_5 S_2$: | | | |
|---|---|---|---|
| Theory | C 50.73, | H 6.51, | N 6.96. |
| Found | C 50.38, | H 6.62, | N 6.83. |

Example 9

Preparation of [(methylethyl)sulfonyl]{2-[4-(2-{[(methylethyl)sulfonyl]amino}ethoxy)phenyl]cyclopent-2-enyl}amine.

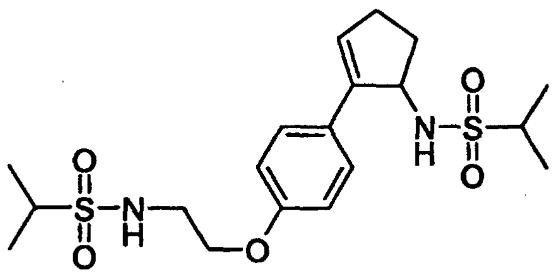

The title compound is prepared in a manner analogous to the procedure set forth in example 8 from isopropylsulfonyl chloride (84 mg, 1.1 eq), DBU (108 mg, 1.2 eq) and {2-[4-{2-aminoethoxy)phenyl]cyclopent-2-enyl}[(methylethyl)sulfonyl]amine (202 mg, 0.59 mmol).

Additional preparation of title compound.

In a 100 mL, 3 neck flask fitted with a stirrer and thermometer, propanesulfonyl chloride (47 mg, 1.2 eq) is added dropwise to to {2-[4-(2-aminoethoxy)phenyl]cyclopent-2-enyl}[(methylethyl)sulfonyl]amine (90 mg, 0.28 mmol, prepared in example 8) and DBU (64 mg, 1.5 eq) in methylene chloride (25 mL) while stirring at 0°C under a nitrogen atomsphere. The reaction is allowed to warm to room temperature and stirred overnight at this temperature. In the morning, the reaction is diluted with methylene chloride (25 mL) and the organic layer is washed two times with water, dried over anhydrous sodium sulfate, filtered, and concentrated under reduced vacuum to yield 111 mg of crude material as a viscous oil. This material is purified via silica gel chromatography employing a Chromatotron® and using a 1000 micron rotor while eluting with a solvent of methylene chloride/ethyl acetate (9: 1) to provide the title compound (33 mg, 28% yield) as a white foam.
Ion spray M.S. 429.3 (M* - 1).

| Calculated for $C_{19} H_{30} N_2 O_5 S_2$-1/2$H_2O$: | | | |
|---|---|---|---|
| Theory | C 51.91, | H 7.10, | N 6.37. |
| Found | C 51.93. | H 6.71, | N 6.26. |

Example 10

Preparation of [(methylethyl)sulfonyl][2-(4-phenylphenyl)cyclopent-2-enyl]amine.

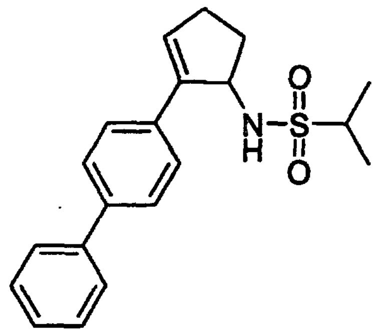

Preparation of [(methylethyl)sulfonyl](2-{4-[(trifluoromethoxy)sulfinyloxy]phenyl}cyclopent-2-enyl)amine.

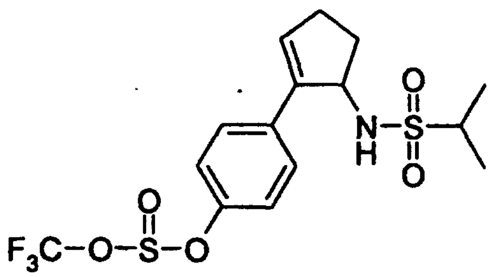

Into a 100 mL 3 neck flask fitted with a stirrer and thermometer, trifluoromethanesulfonic anhydride (0.44 mL, excess, triflic anhydride) is added syringe wise to [2-(4-hydroxyphenyl)cyclopent-2-enyl][(methylethyl)sulfonyl]amine (476 mg, 1.69 mmol) and pyridine (2 mL) in THF (20 mL) while stirring at 0°C under a nitrogen atmosphere. The reaction is allowed to warm to room temperature and stirred for about 14 hours. The reaction mixture is then poured into water and the desired material is extracted into ethyl acetate. The organic layer is washed once with water, dried over potassium carbonate, filtered, and concentrated under reduced vacuum to yield the crude product. This crude material can be purified via silica gel chromatography employing the Chromatotron® using a 4000 micron rotor and eluting with a suitable eluent, such as hexane/ethyl acetate of to provide the intermediate title compound.

Preparation of the final title compound.

Into a 50 mL single neck flask is placed [(methylethyl)sulfonyl](2-{4-[(trifluoromethoxy)sulfinyloxy]phenyl}cyclopent-2-enyl)amine (215 mg, 0.52 mmol), bis(pinocolato)dibron (146 mg, 0.58 mmol), and potassium acetate (155 mg, 1.58 mmol) in DMF (10 mL). This solution is degassed with nitrogen while stirring at room temperature for 10 minutes. $PdCl_2$(dppf) (10 mg) is then added portion wise and the mixture is heated at 80°C under a nitrogen atmosphere for 2 hours. The mixture is allowed to cool to room temperature and phenylbromide (97 mg, 0.62 mmol), 2.0 M sodium carbonate (1.3 mL, 5 eq), and an additional amount of $PdCl_2$(dppf) (10 mg) is added portion wise. The mixture is then heated at 80° C under a nitrogen atmosphere for about 14 hours. The reaction is then allowed to cool to room temperature, poured into 50 mL of water and the desired material is extracted with ethyl acetate. The organic layer is washed two times with $H_2O$, dried over $Na_2SO_4$, filtered, and concentrated under reduced vacuum to yield the crude product. This crude material can be purified via silica gel chromatography employing the Chromatotron® using a 4000 micron rotor and eluting with a suitable eluent, such as ethyl acetate/hexane to provide the final title compound.

Example 11

Preparation of {2-[4-(3,5-difluorophenyl)phenyl]cyclopent-2-enyl}[(methylethyl)sulfonyl]amine.

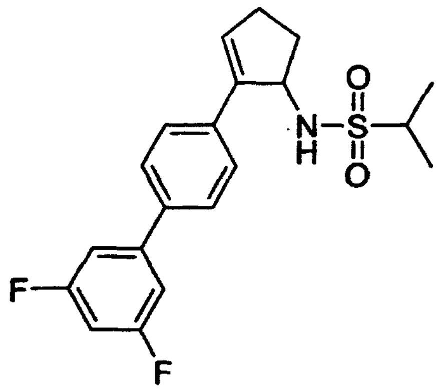

The title compound is prepared in a manner analogous to the procedure described in example 10 from 3,5-difluorophenyl bromide and [(methylethyl)sulfonyl](2-{4-[(trifluoromethoxy)sulfinyloxy]phenyl}cyclopent-2-enyl)amine.

Alternative preparation of {2-[4-(3,5-difluorophenyl)phenyl]cyclopent-2-enyl}[(methylethyl)sulfonyl]amine.

Preparation of (2-hydroxy-2-{4-[(trifluoromethoxy)sulfinyloxy]phenyl}cyclophentyl)[(methylethyl)sulfonyl]amine.

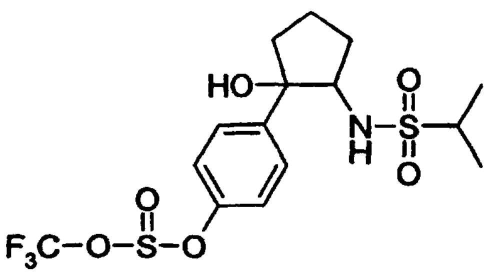

Into a 100 mL, 3 neck flask fitted with a stirrer and thermometer, triflic anhydride (0.58 mL, excess) is added syringe wise to [2-hydroxy-2-(4-hydroxyphenyl)cyclopentyl][(methylethyl)sulfonyl]amine (800 mg, 2.67 mmol, intermediate prepared in example 6) and pyridine (0.36 mL) in THF (25 mL) while stirring at 0°C under a nitrogen atmosphere. The reaction is allowed to warm to room temperature and stirred overnight. In the morning, the reaction mixture is poured into water and the desired material is extracted into ethyl acetate. The organic layer is washed once with water, dried over potassium carbonate, filtered, and concentrated under reduced vacuum to yield 936 mg of crude material as a dark oil. This material is purified via silica gel chromatography employing a Chromatotron® using a 4000 micron rotor and eluting with a solvent of hexane/ethyl acetate (7:3) to yield the intermediate title compound (614 mg, 54%) as a viscous oil. Ion spray M.S. 414 (M*-OH).

Preparation of {2-[4-(3,5-difluorophenyl)phenyl]-2-hydroxycyclopentyl}[(methylethyl)sulfonyl]amine.

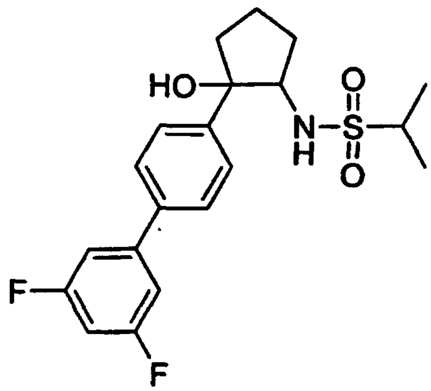

Into a 100 mL, single neck flask, (2-hydroxy-2-{4-[(trifluoromethoxy)sulfinyloxy]phenyl}cyclopentyl)[(methylethyl)sulfonyl]amine (300 mg, 0.70 mmol), 3,5-difluorophenylboronic acid (143 mg, 1.3 eq), tetrakis (triphenylphosphine)palladium(0) and sodium carbonate (4.0 mL, 5.6 eq, 2M) are combined in dioxane (20 mL) and stirred at reflux for 16 hours. The reaction is cooled to room temperature and poured into water. The desired material is extracted into ethyl acetate and the organic layer is washed once with water, dried over potassium carbonate, filtered, and concentrated under reduced vacuum to yield 416 mg of crude material as a dark oil. This material is purified via silica gel chromatography employing a Chromatotron® using a 4000 micron rotor and eluting with a solvent of hexane/ethyl acetate (7:3) to yield the intermediate title compound (41 mg, 15% yield, top spot by TLC) as a white solid. Ion spray M.S. 396.9 (M*+1).

Preparation of final title compound.

Into a 50 mL, 3 neck flask fitted with a stirrer and thermometer, {2-[4-(3,5-difluorophenyl)phenyl]-2-hydroxycyclopentyl}[(methylethyl)sulfonyl]amine 170 mg, 0.4 mmol) in methylene chloride (10 mL) is added dropwise to DAST (0.01 mL) in methylene chloride (21 0mL) while stirring at -78°C under a nitrogen atomsphere. The reaction is allowed to warm to room temperature and diluted with methylene chloride (20 mL). The organic layer is washed with water, dried over anhydrous sodium sulfate, filtered, and concentrated under reduced vacuum to yield 170 mg of crude material as a two spot mixture (by TLC) as an oil. This two spot material is purified via silica gel chromatography employing the Chromatotron® and using a 4000 micron rotor while eluting with a solvent of methylene chloride/ethyl acetate (9:1) to yield the final title compound (39 mg, 24% yield, top spot by TLC) as solid.
Fd M.S. 378.2 (M*+1).

Example 12

Preparation of 3-[4-(5-{[(methylethyl)sulfonyl]amino}cyclopent-1-enyl)phenyl]benzenecarbonitrile.

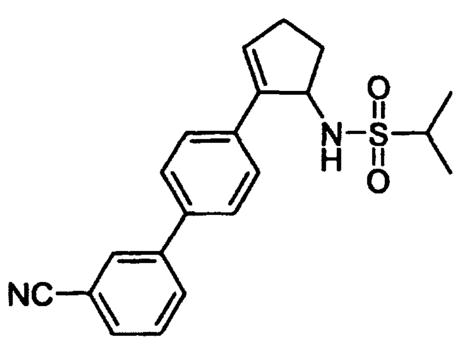

The title compound is prepared in a manner analogous to the procedure described in example 10 from 3-cyanophenyl bromide and [(methylethyl)sulfonyl](2-{4-[(trifluoromethoxy)sulfinyloxy]phenyl}cyclopent-2-enyl)amine.

Example 13

Preparation of 4-[4-(5-{[(methylethyl)sulfonyl]amino}cyclopent-1-enyl)phenyl]benzenecarbonitrile.

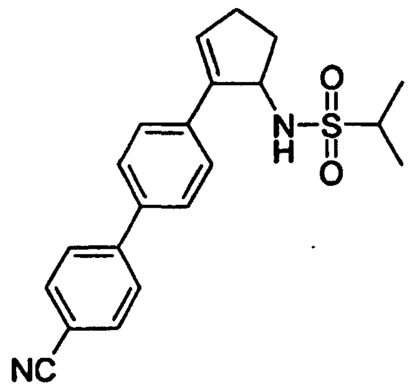

The title compound is prepared in a manner analogous to the procedure described in example 10 from 4-cyanophenyl bromide and [(methylethyl)sulfonyl](2-{4-[(trifluoromethoxy)sulfinyloxy]phenyl}cyclopent-2-enyl)amine.

Example 14

Preparation of {2-[4-(3-aminophenyl)phenyl]cyclopent-2-enyl}[(methylethyl)sulfonyl]amine.

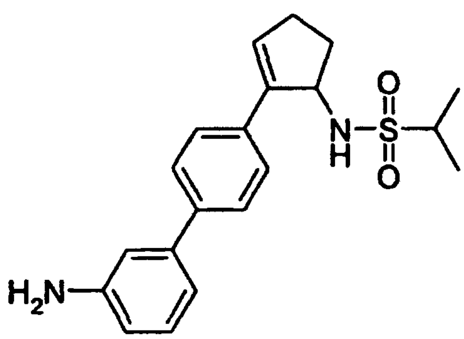

The title compound is prepared in a manner analogous to the procedure described in example 10 from 2-aminophenyl bromide and [(methylethyl)sulfonyl](2-{4-[(trifluoromethoxy)sulfinyloxy]phenyl}cyclopent-2-enyl)amine.

Example 15

Preparation of N-{3-[4-(5-{[(methylethyl)sulfonyl]amino}cyclopent-1-enyl)phenyl]phenyl}acetamide.

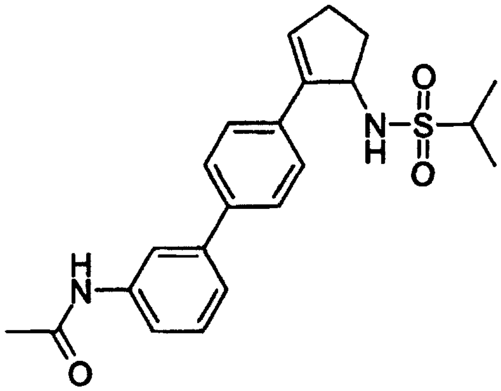

Into a 50 mL-3n flask fitted with a stirrer and thermometer, acetyl chloride (47 mg, 1.1 eq) is added dropwise to {2-[4-(3-aminophenyl)phenyl]cyclopent-2-enyl}[(methylethyl)sulfonyl]amine (192 mg, 0.53 mmol) and triethylamine (66 mg, 1.2 eq) in methylene chloride (25 mL) while stirring at 0° C under a nitrogen atmosphere. The reaction is allowed to warm to room temperature and stirred for one hour. 25 mL of water is added and the layers are separated. The organic layer is washed two times with $H_2O$, dried over $Na_2SO_4$, filtered, and concentrated under reduced vacuum to yield the crude product. This crude material is purified via silica gel chromatography employing the Chromatotron® using a 4000 micron rotor and eluting with a suitable eluent, such as hexane/ethyl acetate to provide the title compound.

Example 16

Preparation of [(methylethyl)sulfonyl][2-(4-{3-[(methylsulfonyl)amino]phenyl}phenyl)cyclopent-2-enyl]amine.

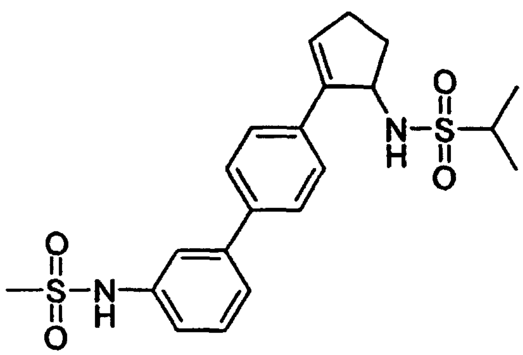

In a 50 mL 3 neck flask fitted with a stirrer and thermometer, methanesulfonyl chloride (69 mg, 1.1 eq) is added dropwise to {2-[4-(3-aminophenyl)phenyl]cyclopent-2-enyl}[(methylethyl)sulfonyl]amine (192 mg, 0.53 mmol) and DBU (97 mg, 1.2 eq) in methylene chloride (25 mL) while stirring at 0°C under a nitrogen atmosphere. The reaction is allowed to warm to room temperature and is stirred for about 14 hours at this temperature. The reaction is then diluted with methylene chloride (30 mL) and the organic layer is washed two times with $H_2O$, dried over $Na_2SO_4$, filtered, and concentrated under reduced vacuum to yield the crude product. This crude material can be purified via silica gel chromatography employing the Chromatotron® using a 4000 micron rotor and eluting with a suitable eluent, such as hexane/ethyl acetate to provide the title compound.

Example 17

Preparation of [(methylethyl)sulfonyl][2-(4-{2-[(methylsulfonyl)amino]ethoxy}phenyl)cyclohex-2-enyl]amine.

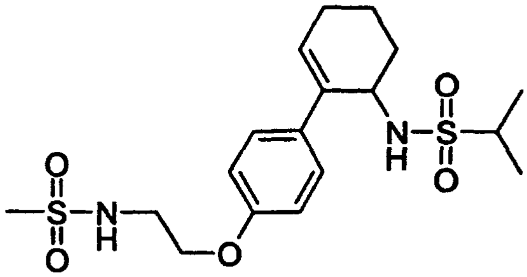

Preparation of 2-[4-(6-{[(methylethyl)sulfonyl]amino}cyclohex-1-enyl)phenoxy]ethanenitrile.

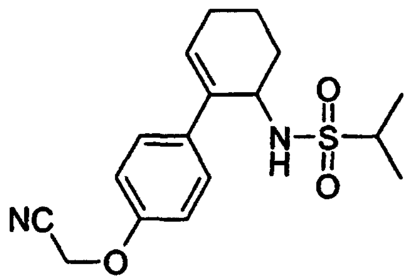

[2-(4-Hydroxyphenyl)cyclohex-2-enyl][(methylethyl)sulfonyl]amine (350 mg, 1.19 mmol, prepared in example 1), bromoacetonitrile (156 mg, 1.1 eq), and potassium carbonate (200 mg, 1.2 eq) were combined with acetone (25 mL) and stirred overnight at room temperature under a nitrogen atmosphere. In the morning, the solution was then filtered and the filtrate was concentrated under reduced vacuum to yield 471 mg as a brown oil. This material was purified via silica gel chromatography employing the Chromatotron® using a 4000 micron rotor while eluting with a solvent of methylene chloride/ethyl acetate 19:1 to provide the intermediate title compound (361 mg, 91%) as an oil. Ion spray M.S. 333 (M* - 1).

| Calculated for $C_{17}H_{22}N_2O_3S$-1/2 $H_2O$ : | | | |
|---|---|---|---|
| Theory | C 59.43, | H 6.75, | N 8.16. |
| Found | C 59.68, | H 6.47, | N 7.82. |

Preparation of {2-[4-(2-aminoethoxy)phenyl]cyclohex-2-enyl}[(methylethyl)sulfonyl]amine.

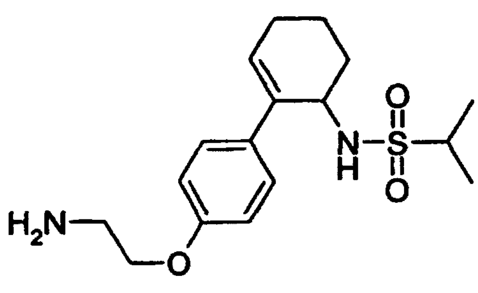

Into a 50 mL, 3 neck flask with stirrer, 2-[4-(6-{[(methylethyl)sulfonyl]amino}cyclohex-1-enyl)phenoxy] ethanenitrile(400 mg, 1.14 mmol) in toluene (15 mL) is added dropwise to Red-AL (3 mL) while stirring at room temperature under a nitrogen atmosphere. The reaction mixture is then stirred at room temperature for 2 hours. The mixture is poured into water and the desired material is extracted into ethyl acetate. The organic layer is washed once with water, dried over pottasium carbonate, filtered, and concentrated under reduced vacuum. This material can then be purified via silica gel chromatography employing the Chromatotron® and using a 2000 micron rotor while eluting with a solvent of methylene chloride/ethyl acetate 4:1 to yield the intermediate title compound.

Preparation of final title compound.

In a 50 mL 3 neck flask fitted with a stirrer and thermometer, methanesulfonyl chloride (77 mg, 1.1 eq) is added dropwise to {2-[4-(2-aminoethoxy)phenyl]cyclohex-2-enyl}[(methylethyl)sulfonyl]amine (200 mg, 0.59 mmol) and DBU (108 mg, 1.2 eq) in methylene chloride (25 mL) while stirring at 0°C under a nitrogen atmosphere. The reaction is allowed to warm to room temperature and is stirred for about 14 hours at this temperature. The reaction is then diluted with methylene chloride (30 mL) and the organic layer is washed two times with $H_2O$, dried over $Na_2SO_4$, filtered, and concentrated under reduced vacuum to yield the crude product. This material can be purified via silica gel chromatography employing the Chromatotron® using a 4000 micron rotor and eluting with a solvent of methylene chloride to provide the final title compound.

Example 18

Preparation of [(methylethyl)sulfonyl]{2-[4-(2-{[(methylethyl)sulfonyl]amino}ethoxy)phenyl]cyclohex-2-enyl}amine.

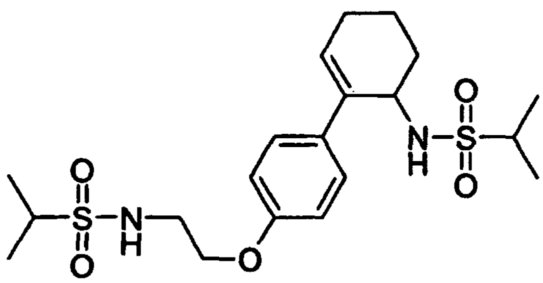

The title compound is prepared in a manner analogous to the procedure set forth in example 17 from isopropylsulfonyl chloride (84 mg, 1.1 eq), DBU (108 mg, 1.2 eq) and {2-[4-(2-aminoethoxy)phenyl]cyclohex-2-enyl}[(methylethyl)sulfonyl]amine (200 mg, 0.59 mmol).

Example 19

Preparation of [(methylethyl)sulfonyl][2-(4-phenylphenyl)cyclohex-2-enyl]amine.

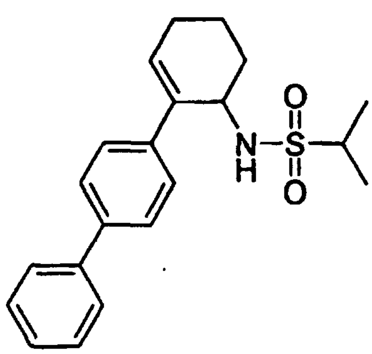

The title compound is prepared in a manner analogous to the procedure set forth in Example 10 from [(methylethyl)sulfonyl](2-{4-[(trifluoromethoxy)sulfinyloxy]phenyl}cyclohex-2-enyl)amine and phenylbromide.

Example 20

Preparation of {2-[4-(3,5-difluorophenyl)phenyl]cyclohex-2-enyl}[(methylethyl)sulfonyl]amine.

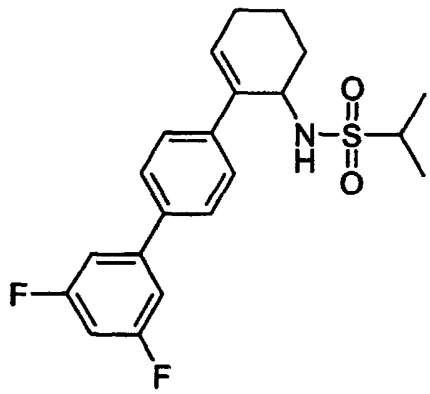

The title compound is prepared in a manner analogous to the procedure set forth in Example 10 from [(methylethyl)sulfonyl](2-{4-[(trifluoromethoxy)sulfinyloxy]phenyl}cyclohex-2-enyl)amine and 3,5-difluorophenyl bromide.

Example 21

Preparation of 3-[4-(6-{[(methylethyl)sulfonyl]amino}cyclohex-1-enyl)phenyl]benzenecarbonitrile.

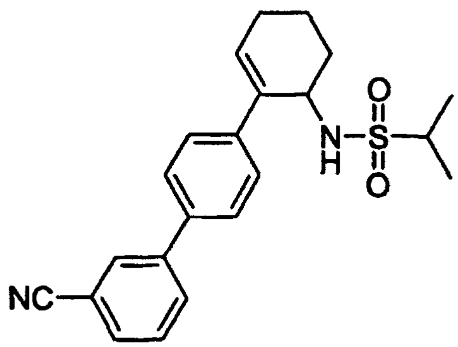

The title compound is prepared in a manner analogous to the procedure set forth in Example 10 from [(methylethyl)sulfonyl](2-{4-[(trifluoromethoxy)sulfinyloxy]phenyl}cyclohex-2-enyl)amine and 3-cyanophenyl bromide.

Example 22

Preparation of 4-[4-(6-{[(methylethyl)sulfonyl]amino}cyclohex-1-enyl)phenyl]benzenecarbonitrile.

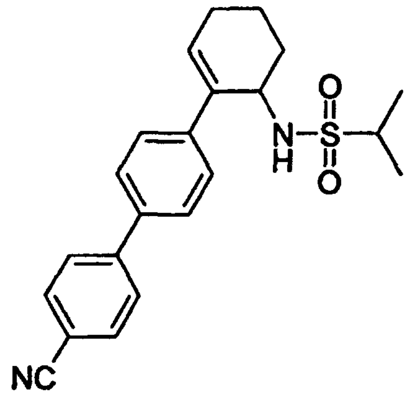

The title compound is prepared in a manner analogous to the procedure set forth in Example 10 from [(methylethyl)sulfonyl](2-{4-[(trifluoromethoxy)sulfinyloxy]phenyl}cyclohex-2-enyl)amine and 4-cyanophenyl bromide.

Example 23

Preparation of {2-[4-(3-aminophenyl)phenyl]cyclohex-2-enyl}[(methylethyl)sulfonyl]amine.

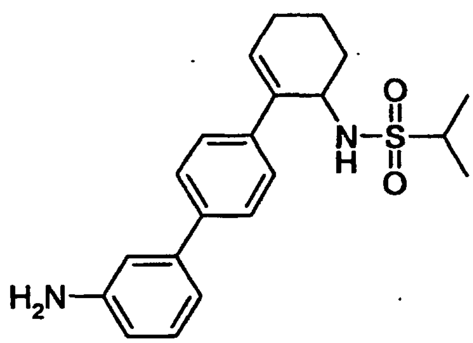

The title compound is prepared in a manner analogous to the procedure set forth in Example 10 from [(methylethyl)sulfonyl](2-{4-[(trifluoromethoxy)sulfinyloxy]phenyl}cyclohex-2-enyl)amine and 3-aminophenyl bromide.

Example 24

Preparation of N-{3-[4-(6-{[(methylethyl)sulfonyl]amino}cyclohex-1-enyl)phenyl]phenyl}acetamide.

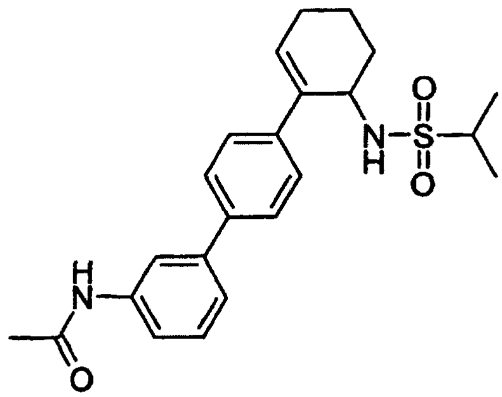

The title compound is prepared in a manner analogous to the procedure set forth in Example 15 from {2-[4-(3-aminophenyl)phenyl]cyclohex-2-enyl}[(methylethyl)sulfonyl]amine prepared in Example 23.

Example 25

Preparation of [(methylethyl)sulfonyl][2-(4-{3-[(methylsulfonyl)amino]phenyl}phenyl)cyclohex-2-enyl]amine.

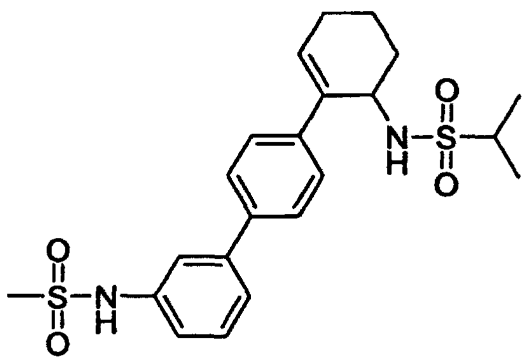

The title compound is prepared in a manner analogous to the procedure set forth in Example 16 from {2-[4-(3-aminophenyl)phenyl]cyclohex-2-enyl}[(methylethyl)sulfonyl]amine prepared in Example 23.

Example 26

Preparation of [2-(4-chlorophenyl)cyclopent-2-enyl][(methylethyl)sulfonyl]amine.

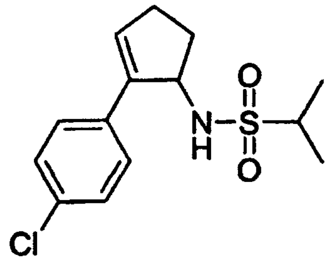

Preparation of [2-(4-chlorophenyl)-2-hydroxycyclopentyl][(methylethyl)sulfonyl]amine.

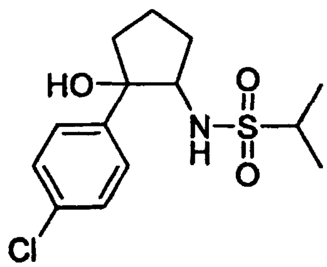

Into a flame dried 100 mL, 3 neck flask that is fitted with a thermometer and condenser, and while stirring at room temperature under a nitrogen atmosphere, 2-{[(methylethyl)sulfonyl]amino}cyclopentan-1-one (1.00 g, 4.87 mmol, prepared as intermediate in example 5) in THF (25 mL) is added dropwise to 4-chlorophenylmagnesium bromide (7.3 mL, 1.5 eq, 1.0 M solution). The addition is continued dropwise, keeping the temperature above 35°C. After addition is complete, the reaction is refluxed for 2 hours. The reaction is then cooled to room temperature and enough saturated ammonium chloride in water is added to precipitate salts nicely and the organic layer is decanted off. The remaining salts are washed two times with THF and the combined organic layers are concentrated under reduced vacuum. The resulting semi-solid is taken into ethyl acetate, washed once with water, dried over potassium carbonate, filtered, and concentrated under reduced vacuum to yield 1.21 gm of crude material as an oil. This material was purified via silica gel chromatography employing a Chromatotron® and using a 4000 micron rotor while eluting with a gradient solvent of methylene chloride to methylene chloride/ethyl acetate (9:1) to provide the intermediate title compound (757 mg, 49% yield) as a white solid. Ion Spray M.S. 316.1 (M*- 1).

Preparation of final title compound.

Into a 100 mL, 3 neck flask fitted with a stirrer and thermometer, [2-(4-chlorophenyl)-2-hydroxycyclopentyl][(methylethyl)sulfonyl]amine (710 mg, 2.23 mmol) in methylene chloride (10 mL) is added dropwise to DAST (0.2 mL) in methylene chloride (15 mL) while stirring at -78°C under a nitrogen atomsphere. The reaction is allowed to warm to room temperature and diluted with methylene chloride (20 mL). The organic layer is washed with water, dried over anhydrous sodium sulfate, filtered, and concentrated under reduced vacuum to yield 700 mg of crude material (as a two spot mixture by TLC) as an oil. This two spot material is purified via silica gel chromatography employing a Chromatotron® and using a 4000 micron rotor while eluting with a solvent of methylene chloride/ethyl acetate (9:1) to yield the final title compound (151 mg, 23% yield, top spot by TLC) as solid.
Ion Spray M.S. 300.2 (M*+1) and 298.2 (M*-1).

Example 27

Preparation of [2-(3,5-difluorophenyl)cyclopent-2-enyl][(methylethyl)sulfonyl]amine.

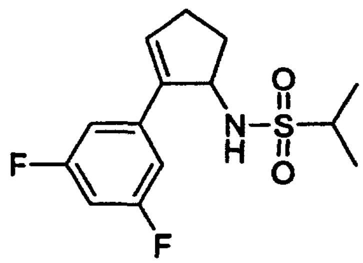

Preparation of [2-(3,5-difluorophenyl)-2-hydroxycyclopentyl][(methylethyl)sulfonyl]amine.

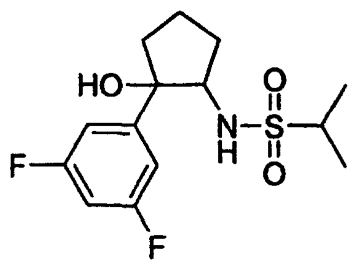

Into a flame dried 100 mL, 3 neck flask that is fitted with a thermometer and condenser, and while stirring at room temperature under a nitrogen atmosphere, 2-{[(methylethyl)sulfonyl]amino}cyclopentan-1-one (500 mg, 2.44 mmol, prepared as an intermediate in example 5) in THF (20 mL) is added dropwise to 3,5-difluorophenylmagnesium bromide (7.3 mL, 0.5 M solution). The addition is continued dropwise, keeping the temperature above 30°C. After the addition is complete, the reaction is refluxed for 2 hours. The reaction is then cooled to room temperature and enough saturated ammonium chloride in water is added to precipitate salts nicely and the organic layer is decanted off. The remaining salts are washed two times with THF and the combined organic layers are concentrated under reduced vacuum. The resulting semi-solid is taken into ethyl acetate, washed once with water, dried over potassium carbonate, filtered, and concentrated under reduced vacuum to yield 721 mg of crude material as an oil. This material is purified via silica gel chromatography employing the Chromatotron® and using a 4000 micron rotor while eluting with a solvent of methylene chloride/ethyl acetate (9:1) to yield the intermediate title compound (463 mg, 59% yield) as a white foam. Ion Spray M.S. 318.1 (M*-1).

Preparation of final title compound.

Into a 100 mL, 3 neck flask fitted with a stirrer and thermometer, [2-(3,5-difluorophenyl)-2-hydroxycyclopentyl] [(methylethyl)sulfonyl]amine 300 mg, 0.94 mmol) in methylene chloride (10 mL) is added dropwise to DAST (0.1 mL) in methylene chloride (15 mL) while stirring at -78°C under a nitrogen atomsphere. The reaction is allowed to warm to room temperature and diluted with methylene chloride (20 mL). The organic layer is washed with water, dried over anhydrous sodium sulfate, filtered, and concentrated under reduced vacuum to yield 232 mg of crude material (a two spot mixture by TLC) as an oil. This two spot material is purified via silica gel chromatography employing a Chromatotron® and using a 4000 micron rotor while eluting with a solvent of methylene chloride to yield the final title compound (25 mg, 9% yield, top spot by TLC) as solid.
Ion Spray M.S. 302.1 (M*+1).

The ability of compounds of formula I to potentiate glutamate receptor-mediated response may be determined using fluorescent calcium indicator dyes (Molecular Probes, Eugene, Oregon, Fluo-3) and by measuring glutamate-evoked efflux of calcium into GluR4 transfected HEK293 cells, as described in more detail below.

In one test, 96 well plates containing confluent monolayers of HEK 293 cells stably expressing human GluR4B

(obtained as described in European Patent Application Publication Number EP-A1-583917) are prepared. The tissue culture medium in the wells is then discarded, and the wells are each washed once with 200 µl of buffer (glucose, 10mM, sodium chloride, 138mM. magnesium chloride, 1mM, potassium chloride, 5mM, calcium chloride, 5mM, N-[2-hydroxyethyl]-piperazine-N-[2-ethanesulfonic acid], 10mM, to pH 7.1 to 7.3). The plates are then incubated for 60 minutes in the dark with 20 µM Fluo3-AM dye (obtained from Molecular Probes Inc., Eugene, Oregon) in buffer in each well. After the incubation, each well is washed once with 100 µl buffer, 200 µl of buffer is added and the plates are incubated for 30 minutes.

Solutions for use in the test are also prepared as follows. 30 µM, 10 µM, 3 µM and 1 µM dilutions of test compound are prepared using buffer from a 10 mM solution of test compound in DMSO. 100 µM cyclothiazide solution is prepared by adding 3 µl of 100 mM cyclothiazide to 3 mL of buffer. Control buffer solution is prepared by adding 1.5 µl DMSO to 498.5 µl of buffer.

Each test is then performed as follows. 200 µl of control buffer in each well is discarded and replaced with 45 µl of control buffer solution. A baseline fluorescent measurement is taken using a FLUOROSKAN II fluorimeter (Obtained from Labsystems, Needham Heights, MA, USA, a Division of Life Sciences International Plc). The buffer is then removed and replaced with 45 µl of buffer and 45 µl of test compound in buffer in appropriate wells. A second fluorescent reading is taken after 5 minutes incubation. 15 µl of 400 µM glutamate solution is then added to each well (final glutamate concentration 100 µM), and a third reading is taken. The activities of test compounds and cyclothiazide solutions are determined by subtracting the second from the third reading (fluorescence due to addition of glutamate in the presence or absence of test compound or cyclothiazide) and are expressed relative to enhance fluorescence produced by 100 µM cyclothiazide.

In another test, HEK293 cells stably expressing human GluR4 (obtained as described in European Patent Application Publication No. EP-A1-0583917) are used in the electrophysiological characterization of AMPA receptor potentiators. The extracellular recording solution contains (in mM): 140 NaCl, 5 KCl, 10 HEPES, 1 $MgCl_2$, 2 $CaCl_2$, 10 glucose, pH = 7.4 with NaOH, 295 mOsm kg-1. The intracellular recording solution contains (in mM): 140 CsCl, 1 $MgCl_2$, 10 HEPES, (N-[2-hydroxyethyl]piperazine-N1-[2-ethanesulfonic acid]) 10 EGTA (ethylene-bis(oxyethylene-nitrilo)tetraacetic acid), pH = 7.2 with CsOH, 295 mOsm kg-1. With these solutions, recording pipettes have a resistance of 2-3 MΩ. Using the whole-cell voltage clamp technique (Hamill et al.(1981)Pflügers Arch., 391: 85-100), cells are voltage-clamped at -60mV and control current responses to 1 mM glutamate are evoked. Responses to 1 mM glutamate are then determined in the presence of test compound. Compounds are deemed active in this test if, at a test concentration of 10 µM or less, they produce a greater than 10% increase in the value of the current evoked by 1 mM glutamate.

In order to determine the potency of test compounds, the concentration of the test compound, both in the bathing solution and co-applied with glutamate, is increased in half log units until the maximum effect was seen. Data collected in this manner are fit to the Hill equation, yielding an $EC_{50}$ value, indicative of the potency of the test compound. Reversibility of test compound activity is determined by assessing control glutamate 1 mM responses. Once the control responses to the glutamate challenge are re-established, the potentiation of these responses by 100 µM cyclothiazide is determined by its inclusion in both the bathing solution and the glutamate-containing solution. In this manner, the efficacy of the test compound relative to that of cyclothiazide can be determined.

According to another aspect, the present invention provides a pharmaceutical composition, which comprises a compound of formula I or a pharmaceutically acceptable salt thereof and a pharmaceutically acceptable diluent or carrier.

The pharmaceutical compositions are prepared by known procedures using well-known and readily available ingredients. In making the compositions of the present invention, the active ingredient will usually be mixed with a carrier, or diluted by a carrier, or enclosed within a carrier, and may be in the form of a capsule, sachet, paper, or other container. When the carrier serves as a diluent, it may be a solid, semi-solid, or liquid material which acts as a vehicle, excipient, or medium for the active ingredient. The compositions can be in the form of tablets, pills, powders, lozenges, sachets, cachets, elixirs, suspensions, emulsions, solutions, syrups, aerosols, ointments containing, for example, up to 10% by weight of active compound, soft and hard gelatin capsules, suppositories, sterile injectable solutions, and sterile packaged powders.

Some examples of suitable carriers, excipients, and diluents include lactose, dextrose, sucrose, sorbitol, mannitol, starches, gum, acacia, calcium phosphate, alginates, tragcanth, gelatin, calcium silicate, micro-crystalline cellulose, polyvinylpyrrolidone, cellulose, water syrup, methyl cellulose, methyl and propyl hydroxybenzoates, talc, magnesium stearate, and mineral oil. The formulations can additionally include lubricating agents, wetting agents, emulsifying and suspending agents, preserving agents, sweetening agents, or flavoring agents. Compositions of the invention may be formulated so as to provide quick, sustained, or delayed release of the active ingredient after administration to the patient by employing procedures well known in the art.

The compositions are preferably formulated in a unit dosage form, each dosage containing from about 1 mg to about 500 mg, more preferably about 5 mg to about 300 mg (for example 25 mg) of the active ingredient. The term "unit dosage form" refers to a physically discrete unit suitable as unitary dosages for human subjects and other mammals, each unit containing a predetermined quantity of active material calculated to produce the desired therapeutic effect, in association with a suitable pharmaceutical carrier, diluent, or excipient.

As used herein the term "patient" refers to a mammal, such as a mouse, guinea pig, rat, dog or human. It is understood that the preferred patient is a human.

As used herein, the terms "treating" or "to treat" each mean to alleviate symptoms, eliminate the causation either on a temporary or permanent basis, or to prevent or slow the appearance of symptoms of the named disorder. As such, the methods of this invention encompass both therapeutic and prophylactic administration.

As used herein, the term "effective amount" refers to the amount of a compound of formula I which is effective, upon single or multiple dose administration to a patient, in treating the patient suffering from the named disorder.

An effective amount can be readily determined by the attending diagnostician, as one skilled in the art, by the use of known techniques and by observing results obtained under analogous circumstances. In determining the effective amount or dose, a number of factors are considered by the attending diagnostician, including, but not limited to: the species of mammal; its size, age, and general health; the specific disease or disorder involved; the degree of or involvement or the severity of the disease or disorder; the response of the individual patient; the particular compound administered; the mode of administration; the bioavailability characteristics of the preparation administered; the dose regimen selected; the use of concomitant medication; and other relevant circumstances.

The compounds of formula I can be administered by a variety of routes including oral, rectal, transdermal, subcutaneous, intravenous, intramuscular, bucal or intranasal routes. Alternatively, the compounds of formula I may be administered by continuous infusion. A typical daily dose will contain from about 0.01 mg/kg to about 100 mg/kg of the compound of formula I. Preferably, daily doses will be about 0.05 mg/kg to about 50 mg/kg, more preferably from about 0.1 mg/kg to about 25 mg/kg.

## Claims

**1.** A compound of the formula:

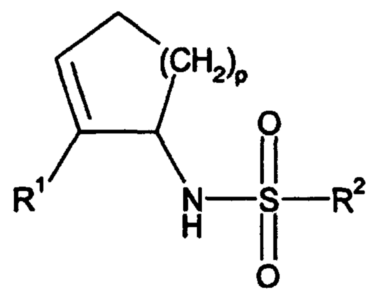

wherein
p represents integer 1 or 2;
$R^1$ represents an unsubstituted or substituted aromatic group, or an unsubstituted or substituted heteroaromatic group; and
$R^2$ represents (1-6C)alkyl, (3-6C)cycloalkyl, fluoro(1-6C)alkyl, chloro(1-6C)alkyl, (2-6C)alkenyl, phenyl which is unsubstituted or substituted by halogen, (1-4C)alkyl or (1-4C)alkoxy, or a group of formula $R^3R^4N$ in which $R^3$ and $R^4$ each independently represents (1-4C)alkyl or, together with the nitrogen atom to which they are attached form an azetidinyl, pyrrolidinyl, piperidinyl, morpholino, piperazinyl, hexahydroazepinyl or octahydroazocinyl group;
or a pharmaceutically acceptable salt thereof.

**2.** A compound according to claim 1 wherein $R^2$ is (1-6C)alkyl.

**3.** A compound according to claim 2 wherein $R^2$ is 2-propyl.

**4.** A compound according to any one of claims 1 to 3 wherein $R^1$ represents:

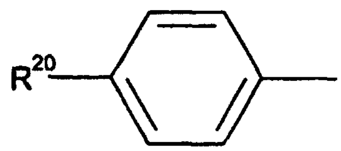

wherein

$R^{20}$ represents halogen; nitro; cyano; hydroxyimino; (1-10C)alkyl; (2-10C)alkenyl; (2-10C)alkynyl; (3-8C)cycloalkyl; hydroxy(3-8C)cycloalkyl; oxo(3-8C)cycloalkyl; halo(1-10C)alkyl; $(CH_2)_yX^1R^9$ in which y is 0 or an integer of from 1 to 4, $X^1$ represents O, S, $NHSO_2$, $SO_2NH$, $NR^{10}$, CO, COO, OCO, $CONR^{11}$, $NR^{12}CO$, $NR^{12}COCOO$, $OCONR^{13}$, $R^9$ represents hydrogen, (1-10C) alkyl, (3-10C)alkenyl, (3-10C)alkynyl, pyrrolidinyl, tetrahydrofuryl, morpholino or (3-8C)cycloalkyl and $R^{10}$, $R^{11}$, $R^{12}$ and $R^{13}$ each independently represents hydrogen or (1-10C) alkyl, or $R^9$ and $R^{10}$, $R^{11}$, $R^{12}$ or $R^{13}$ together with the nitrogen atom to which they are attached form an azetidinyl, pyrrolidinyl, piperidinyl or morpholino group; N-(1-4C)alkylpiperazinyl; N-phenyl(1-4C)alkylpiperazinyl; thienyl; furyl; oxazolyl; isoxazolyl; pyrazolyl; imidazolyl; thiazolyl; tetrazolyl; pyridyl; pyridazinyl; pyrimidinyl; dihydrothienyl; dihydrofuryl; dihydrothiopyranyl; dihydropyranyl; dihydrothiazolyl; (1-4C)alkoxycarbonyldihydrothiazolyl; (1-4C) alkoxycarbonyldimethyl-dihydrothiazolyl; tetrahydrothienyl; tetrahydrofuryl; tetrahydrothiopyranyl; tetrahydropyranyl; indolyl; benzofuryl; benzothienyl; benzimidazolyl; benzothiazolyl; and a group of formula $R^{14}-(L^a)_n-X^2-(L^b)_m$ in which $X^2$ represents a bond, O, NH, S, SO, $SO_2$, CO, CH(OH), CONH, NHCONH, NHCOO, COCONH, $OCH_2CONH$ or CH=CH, NHCO, $L^a$ and $L^b$ each represent (1-4C)alkylene, one of n and m is 0 or 1 and the other is 0, and $R^{14}$ represents a phenyl or hetero-aromatic group which is unsubstituted or substituted by one or two of halogen; nitro; cyano; (1-10C)alkyl; (2-10C)alkenyl; (2-10C)alkynyl; (3-8C)cycloalkyl; 4-(1,1-dioxotetrahydro-1,2-thiazinyl); halo (1-10C)alkyl; cyano(2-10C)alkenyl; phenyl; $(CH_2)_zX^3R^{15}$ in which z is 0 or an integer of from 1 to 4, $X^3$ represents O, S, $NR^{16}$, CO, CH(OH), COO, OCO, $CONR^{17}$, $NR^{18}CO$, $NHSO_2$, $SO_2NH$, $NHSO_2NR^{17}$, NHCONH, $OCONR^{19}$, N(CO(1-4C)alkyl)CO, or $NR^{19}COO$, $R^{15}$ represents hydrogen, (1-10C)alkyl, phenyl(1-4C)alkyl, halo(1-10C)alkyl, (1-4C)alkoxycarbonyl(1-4C)alkyl, (1-4C)alkylsulfonylamino(1-4C)alkyl, (1-4C)alkylaminosulfonyl(1-4C)alkyl, (N-(1-4C)alkoxycarbonyl)(1-4C)alkylsulfonylamino(1-4C)alkyl, (3-10C)alkenyl, (3-10C)alkynyl, (3-8C)cycloalkyl, camphoryl or an aromatic or heteroaromatic group which is unsubstituted or substituted by one or two of halogen, (1-4C)alkyl, halo(1-4C)alkyl, di(1-4C)alkylamino and (1-4C)alkoxy, and $R^{16}$, $R^{17}$, $R^{18}$ and $R^{19}$ each independently represents hydrogen or (1-10C)alkyl, or $R^{15}$ and $R^{16}$, $R^{17}$, $R^{18}$ or $R^{19}$ together with the nitrogen atom to which they are attached form an azetidinyl, pyrrolidinyl, piperidinyl or morpholino group.

**5.** A compound according to claim 4 wherein $R^{20}$ represents halogen; (1-10C)alkyl; halo(1-10C)alkyl; $(CH_2)_yX^1R^9$ in which y is 0 or an integer of from 1 to 4, $X^1$ represents O, S, $NHSO_2$, $SO_2NH$, $NR^{10}$, CO, COO, OCO, $CONR^{11}$, $NR^{12}CO$, $NR^{12}COCOO$, $OCONR^{13}$, $R^9$ represents hydrogen, (1-10C) alkyl, (3-10C)alkenyl, (3-10C)alkynyl, pyrrolidinyl, tetrahydrofuryl, morpholino or (3-8C)cycloalkyl and $R^{10}$, $R^{11}$, $R^{12}$ and $R^{13}$ each independently represents hydrogen or (1-10C)alkyl, or $R^9$ and $R^{10}$, $R^{11}$, $R^{12}$ or $R^{13}$ together with the nitrogen atom to which they are attached form a pyrrolidinyl, piperidinyl or morpholino group; and a group of formula $R^{14}-(L^a)_n-X^2-(L^b)_m$ in which $X^2$ represents a bond, O, NH, S, SO, $SO_2$, CO, CH(OH), CONH, NHCONH, NHCOO, COCONH, $OCH_2CONH$ or CH=CH, NHCO, $L^a$ and $L^b$ each represent (1-4C)alkylene, one of n and m is 0 or 1 and the other is 0, and $R^{14}$ represents a phenyl group which is unsubstituted or substituted by one or two of halogen; nitro; cyano; (1-10C)alkyl; halo (1-10C)alkyl; phenyl; $(CH_2)_zX^3R^{15}$ in which z is 0 or an integer of from 1 to 4, $X^3$ represents O, S, $NR^{16}$, CO, CH (OH), COO, OCO, $CONR^{17}$, $NR^{18}CO$, $NHSO_2$, $SO_2NH$, $NHSO_2NR^{17}$, NHCONH, $OCONR^{19}$, N(CO(1-4C)alkyl) CO, or $NR^{19}COO$, $R^{15}$ represents hydrogen, (1-10C)alkyl, phenyl(1-4C)alkyl, halo(1-10C)alkyl, (1-4C)alkoxycarbonyl(1-4C)alkyl, (1-4C)alkylsulfonylamino(1-4C)alkyl, (1-4C)alkylaminosulfonyl(1-4C)alkyl, (N-(1-4C)alkoxycarbonyl)(1-4C)alkylsulfonylamino(1-4C)alkyl, or an aromatic or heteroaromatic group which is unsubstituted or substituted by one or two of halogen, (1-4C)alkyl, halo(1-4C)alkyl, di(1-4C)alkylamino and (1-4C)alkoxy, and $R^{16}$, $R^{17}$, $R^{18}$ and $R^{19}$ each independently represents hydrogen or (1-10C)alkyl, or $R^{15}$ and $R^{16}$, $R^{17}$, $R^{18}$ or $R^{19}$ together with the nitrogen atom to which they are attached form a pyrrolidinyl, piperidinyl or morpholino group.

**6.** A compound according to claim 5 wherein $R^{20}$ represents $(CH_2)_yX^1R^9$ in which y is 0 or an integer of from 1 to 4, $X^1$ represents O, S, $NHSO_2$, $SO_2NH$, $NR^{10}$, CO, COO, OCO, $CONR^{11}$, $NR^{12}CO$, $NR^{12}COCOO$, $OCONR^{13}$, $R^9$ represents hydrogen, (1-10C) alkyl, pyrrolidinyl, tetrahydrofuryl, morpholino or (3-8C)cycloalkyl and $R^{10}$, $R^{11}$, $R^{12}$ and $R^{13}$ each independently represents hydrogen or (1-10C)alkyl, or $R^9$ and $R^{10}$, $R^{11}$, $R^{12}$ or $R^{13}$ together with the nitrogen atom to which they are attached form a pyrrolidinyl, piperidinyl or morpholino group; and a group of formula $R^{14}-(L^a)_n-X^2-(L^b)_m$ in which $X^2$ represents a bond, O, NH, S, SO, $SO_2$, CO, CONH, NHCOO, or NHCO, $L^a$ and $L^b$ each represent (1-4C)alkylene, one of n and m is 0 or 1 and the other is 0, and $R^{14}$ represents a phenyl

group which is unsubstituted or substituted by one or two of halogen; (1-10C)alkyl; halo(1-10C)alkyl; phenyl; $(CH_2)_zX^3R^{15}$ in which z is 0 or an integer of from 1 to 4, $X^3$ represents O, S, $NR^{16}$, CO, COO, OCO, $CONR^{17}$, $NR^{18}CO$, $NHSO_2$, $SO_2NH$, $NHSO_2NR^{17}$, NHCONH, $OCONR^{19}$, N(CO(1-4C)alkyl)CO, or $NR^{19}COO$, $R^{15}$ represents hydrogen, (1-10C)alkyl, phenyl(1-4C)alkyl, halo(1-10C)alkyl, (1-4C)alkoxycarbonyl(1-4C)alkyl, (1-4C)alkylsulfonylamino(1-4C)alkyl, (1-4C)alkylaminosulfonyl(1-4C)alkyl, (N-(1-4C)alkoxycarbonyl)(1-4C)alkylsulfonylamino(1-4C)alkyl, or an aromatic or heteroaromatic group which is unsubstituted or substituted by one or two of halogen, (1-4C)alkyl, halo(1-4C)alkyl, di(1-4C)alkylamino and (1-4C)alkoxy, and $R^{16}$, $R^{17}$, $R^{18}$ and $R^{19}$ each independently represents hydrogen or (1-10C)alkyl, or $R^{15}$ and $R^{16}$, $R^{17}$, $R^{18}$ or $R^{19}$ together with the nitrogen atom to which they are attached form a pyrrolidinyl, piperidinyl or morpholino group.

**7.** A compound according to claim 6 wherein $R^{20}$ represents $R^{14}$-$(L^a)_n$-$X^2$-$(L^b)_m$.

**8.** A compound according to claim 7 wherein $(L^a)_n$-$X^2$-$(L^b)_m$ represents a bond, CONH, or $CH_2O$.

**9.** A compound according to claim 8 wherein $R^{14}$ represents a phenyl which is substituted by one or two of fluoro; chloro, cyano; (1-4C)alkyl; trifluoromethyl; and $(CH_2)_zX^3R^{15}$ in which z is 0, or 2, $X^3$ represents $NR^{16}$, CO, COO, $CONR^{17}$, $NR^{18}CO$, $NHSO_2$, $SO_2NH$, $R^{15}$ represents hydrogen, (1-4C)alkyl, phenyl(1-4C)alkyl, or halo(1-4C)alkyl, and $R^{16}$, $R^{17}$, $R^{18}$ and $R^{19}$ each independently represent hydrogen or (1-4C)alkyl.

**10.** A compound according to claim 1 which is selected from the group consisting of:

[2-(4-hydroxyphenyl)cyclohex-2-enyl][(methylethyl)sulfonyl]amine;
(2-{4-[(3,5-difluorophenyl)methoxy]phenyl}cyclohex-2-enyl)[(methylethyl)sulfonyl]amine;
2-{[4-(6-{[(methylethyl)sulfonyl]amino}cyclohex-1-enyl)phenoxy]methyl}benzenecarbonitrile;
[(methylethyl)sulfonyl]{2-[4-(phenylmethoxy)phenyl]cyclopent-2-enyl}amine; (2-{4-[(3,5-difluorophenyl)methoxy]phenyl}cyclopent-2-enyl)[(methylethyl)sulfonyl]amine;
[2-(4-chlorophenyl)cyclopent-2-enyl][(methylethyl)sulfonyl]amine; and
[2-(3,5-difluorophenyl)cyclopent-2-enyl][(methylethyl)sulfonyl]amine;

or a pharmaceutically acceptable salt thereof.

**11.** A pharmaceutical composition, which comprises a compound as claimed in any one of Claims 1 to 10 and a pharmaceutically acceptable diluent or carrier.

**12.** A compound according to any of claims 1 to 10, or a pharmaceutically acceptable salt thereof, for use as a pharmaceutical.

**13.** The use of a compound according to any of claims 1 to 10, or a pharmaceutically acceptable salt thereof, for the manufacture of a medicament for potentiating glutamate receptor function.

**14.** The use of a compound according to any of claims 1 to 10 for the manufacture of a medicament for treating a cognitive disorder; Alzheimer's disease; age-related dementia; age-induced memory impairment; depression; attention deficit disorder; attention deficit hyperactivity disorder; psychosis; cognitive deficits associated with psychosis; drug-induced psychosis, Parkinson's disease, or stroke.

**Patentansprüche**

**1.** Verbindung der Formel:

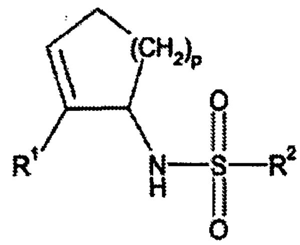

worin
p steht für eine ganze Zahl von 1 oder 2;
$R^1$ steht für eine unsubstituierte oder substituierte aromatische Gruppe oder eine unsubstituierte oder substituierte heteroaromatische Gruppe; und
$R^2$ steht für ($C_1$-$C_6$)-Alkyl, ($C_3$-$C_6$)-Cycloalkyl, Fluor($C_1$-$C_6$)-alkyl, Chlor($C_1$-$C_6$)-alkyl, ($C_2$-$C_6$)-Alkenyl, Phenyl, das unsubstituiert oder substituiert ist durch Halogen, ($C_1$-$C_4$)-Alkyl oder ($C_1$-$C_4$)-Alkoxy, oder eine Gruppe der Formel $R^3R^4N$, in der $R^3$ und $R^4$ jeweils unabhängig stehen für ($C_1$-$C_4$)-Alkyl oder zusammen mit dem Stickstoffatom, an das sie gebunden sind, eine Azetidinyl-, Pyrrolidinyl-, Piperidinyl-, Morpholino-, Piperazinyl-, Hexahydroazepinyl- oder Octahydroazocinylgruppe bilden;
oder ein pharmazeutisch annehmbares Salz davon.

2. Verbindung gemäß Anspruch 1, worin $R^2$ für ($C_1$-$C_6$)-Alkyl steht.

3. Verbindung gemäß Anspruch 2, worin $R^2$ für 2-Propyl steht.

4. Verbindung gemäß einem der Ansprüche 1 bis 3, worin $R^1$ steht für:

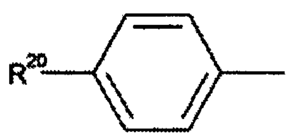

,

worin
$R^{20}$ steht für Halogen; Nitro; Cyano; Hydroxyimino; ($C_1$-$C_{10}$)-Alkyl; ($C_2$-$C_{10}$)-Alkenyl; ($C_2$-$C_{10}$)-Alkinyl; ($C_3$-$C_8$)-Cycloalkyl; Hydroxy($C_3$-$C_8$)-cycloalkyl; Oxo($C_3$-$C_8$)-cycloalkyl; Halogen($C_1$-$C_{10}$)-alkyl; $(CH_2)_yX^1R^9$, worin y steht für 0 oder eine ganze Zahl von 1 bis 4, $X^1$ steht für O, S, $NHSO_2$, $SO_2NH$, $NR^{10}$, CO, COO, OCO, $CONR^{11}$, $NR^{12}CO$, $NR^{12}COCOO$, $OCONR^{13}$, $R^9$ steht für Wasserstoff, ($C_1$-$C_{10}$)-Alkyl, ($C_3$-$C_{10}$)-Alkenyl, ($C_3$-$C_{10}$)-Alkinyl, Pyrrolidinyl, Tetrahydrofuryl, Morpholino oder ($C_3$-$C_8$)-Cycloalkyl und $R^{10}$, $R^{11}$, $R^{12}$ und $R^{13}$ jeweils unabhängig steht für Wasserstoff oder ($C_1$-$C_{10}$)-Alkyl, oder $R^9$ und $R^{10}$, $R^{11}$, $R^{12}$ oder $R^{13}$ bilden zusammen mit dem Stickstoffatom, an das sie gebunden sind, eine Azetidinyl-, Pyrrolidinyl-, Piperidinyl- oder Morpholinogruppe; N-($C_1$-$C_4$)-Alkylpiperazinyl; N-Phenyl($C_1$-$C_4$)-alkylpiperazinyl; Thienyl; Furyl; Oxazolyl; Isoxazolyl; Pyrazolyl, Imidazolyl; Thiazolyl; Tetrazolyl; Pyridyl; Pyridazinyl; Pyrimidinyl; Dihydrothienyl; Dihydrofuryl; Dihydrothiopyranyl; Dihydropyranyl; Dihydrothiazolyl; ($C_1$-$C_4$)-Alkoxycarbonyldihydrothiazolyl; ($C_1$-$C_4$)-Alkoxycarbonyldimethyldihydrothiazolyl; Tetrahydrothienyl; Tetrahydrofuryl; Tetrahydrothiopyranyl; Tetrahydropyranyl; Indolyl; Benzofuryl; Benzothienyl; Benzimidazolyl; Benzothiazolyl; und eine Gruppe der Formel $R^{14}$-$(L^a)_n$-$X^2$-$(L^b)_m$, in der $X^2$ steht für eine Bindung, O, NH, S, SO, $SO_2$, CO, CH(OH), CONH, NHCONH, NHCOO, COCONH, $OCH_2CONH$ oder CH=CH, NHCO, $L^a$ und $L^b$ jeweils stehen für ($C_1$-$C_4$)-Alkylen, eines von n und m für 0 oder 1 steht, und das andere für 0 steht, und $R^{14}$ steht für eine Phenyl- oder heteroaromatische Gruppe, die unsubstituiert oder substituiert ist durch ein oder zwei von Halogen; Nitro; Cyano; ($C_1$-$C_{10}$)-Alkyl, ($C_2$-$C_{10}$)-Alkenyl; ($C_2$-$C_{10}$)-Alkinyl; ($C_3$-$C_8$)-Cycloalkyl; 4-(1,1-Dioxotetrahydro-1,2-thiazinyl); Halogen($C_1$-$C_{10}$)-alkyl; Cyano($C_2$-$C_{10}$)-alkenyl; Phenyl; $(CH_2)_zX^3R^{15}$, worin z steht für 0 oder eine ganze Zahl von 1 bis 4, $X^3$ steht für O, S, $NR^{16}$, CO, CH(OH), COO, OCO, $CONR^{17}$, $NR^{18}CO$, $NHSO_2$, $SO_2NH$, $NHSO_2NR^{17}$, NHCONH, $OCONR^{19}$, N(CO($C_1$-$C_4$)-Alkyl)CO oder $NR^{19}COO$, $R^{15}$ steht für Wasserstoff, ($C_1$-$C_{10}$)-Alkyl, Phenyl ($C_1$-$C_4$)-alkyl, Halogen($C_1$-$C_{10}$)-alkyl, ($C_1$-$C_4$)-Alkoxycarbonyl($C_1$-$C_4$)-alkyl, ($C_1$-$C_4$)-Alkylsulfonylamino($C_1$-$C_4$)-alkyl, ($C_1$-$C_4$)-Alkylaminosulfonyl($C_1$-$C_4$)-alkyl, (N-($C_1$-$C_4$)-Alkoxycarbonyl)($C_1$-$C_4$)-alkylsulfonylamino($C_1$-$C_4$)-alkyl, ($C_3$-$C_{10}$)-Alkenyl, ($C_3$-$C_{10}$)-Alkinyl, ($C_3$-$C_8$)-Cycloalkyl, Campheryl oder eine aromatische oder heteroaromatische

Gruppe, die unsubstituiert oder substituiert ist durch ein oder zwei von Halogen, $(C_1-C_4)$-Alkyl, Halogen$(C_1-C_4)$-alkyl, Di$(C_1-C_4)$-alkylamino und $(C_1-C_4)$-Alkoxy, und $R^{16}$, $R^{17}$, $R^{18}$ und $R^{19}$ jeweils unabhängig stehen für Wasserstoff oder $(C_1-C_{10})$-Alkyl, oder $R^{15}$ und $R^{16}$, $R^{17}$, $R^{18}$ oder $R^{19}$ zusammen mit dem Stickstoffatom, an das sie gebunden sind, eine Azetidinyl-, Pyrrolidinyl-, Piperidinyl- oder Morpholinogruppe bilden.

**5.** Verbindung gemäß Anspruch 4, worin $R^{20}$ steht für $R^{20}$ steht für Halogen; Halogen$(C_1-C_{10})$-alkyl; $(CH_2)_yX^1R^9$, worin y steht für 0 oder eine ganze Zahl von 1 bis 4, $X^1$ steht für O, S, $NHSO_2$, $SO_2NH$, $NR^{10}$, CO, COO, OCO, $CONR^{11}$, $NR^{12}CO$, $NR^{12}COCOO$, $OCONR^{13}$, $R^9$ steht für Wasserstoff, $(C_1-C_{10})$-Alkyl, $(C_3-C_{10})$-Alkenyl, $(C_3-C_{10})$-Alkinyl, Pyrrolidinyl, Tetrahydrofuryl, Morpholino oder $(C_3-C_8)$-Cycloalkyl und $R^{10}$, $R^{11}$, $R^{12}$ und $R^{13}$ jeweils unabhängig steht für Wasserstoff oder $(C_1-C_{10})$-Alkyl, oder $R^9$ und $R^{10}$, $R^{11}$, $R^{12}$ oder $R^{13}$ bilden zusammen mit dem Stickstoffatom, an das sie gebunden sind, eine Pyrrolidinyl-, Piperidinyl- oder Morpholinogruppe; und eine Gruppe der Formel $R^{14}-(L^a)_n-X^2-(L^b)_m$, in der $X^2$ steht für eine Bindung, O, NH, S, SO, $SO_2$, CO, CH(OH), CONH, NHCONH, NHCOO, COCONH, $OCH_2CONH$ oder CH=CH, NHCO, $L^a$ und $L^b$ jeweils stehen für $(C_1-C_4)$-Alkylen, eines von n und m für 0 oder 1 steht, und das andere für 0 steht, und $R^{14}$ steht für eine Phenylgruppe, die unsubstituiert oder substituiert ist durch ein oder zwei von Halogen; Nitro; Cyano; $(C_1-C_{10})$-Alkyl, Halogen$(C_1-C_{10})$-alkyl; Phenyl; $(CH_2)_zX^3R^{15}$, worin steht z für 0 oder eine ganze Zahl von 1 bis 4, $X^3$ steht für O, S, $NR^{16}$, CO, CH(OH), COO, OCO, $CONR^{17}$, $NR^{18}CO$, $NHSO_2$, $SO_2NH$, $NHSO_2NR^{17}$, NHCONH, $OCONR^{19}$, N(CO$(C_1-C_4)$-Alkyl)CO oder $NR^{19}COO$, $R^{15}$ steht für Wasserstoff, $(C_1-C_{10})$-Alkyl, Phenyl$(C_1-C_4)$-alkyl, Halogen$(C_1-C_{10})$-alkyl, $(C_1-C_4)$-Alkoxycarbonyl$(C_1-C_4)$-alkyl, $(C_1-C_4)$-Alkylsulfonylamino$(C_1-C_4)$-alkyl, $(C_1-C_4)$-Alkylaminosulfonyl$(C_1-C_4)$-alkyl, (N-$(C_1-C_4)$-Alkoxycarbonyl)$(C_1-C_4)$-alkylsulfonylamino$(C_1-C_4)$-alkyl oder eine aromatische oder heteroaromatische Gruppe, die unsubstituiert oder substituiert ist durch ein oder zwei von Halogen, $(C_1-C_4)$-Alkyl, Halogen $(C_1-C_4)$-alkyl, Di$(C_1-C_4)$-alkylamino und $(C_1-C_4)$-Alkoxy, und $R^{16}$, $R^{17}$, $R^{18}$ und $R^{19}$ jeweils unabhängig stehen für Wasserstoff oder $(C_1-C_{10})$-Alkyl, oder $R^{15}$ und $R^{16}$, $R^{17}$, $R^{18}$ oder $R^{19}$ zusammen mit dem Stickstoffatom, an das sie gebunden sind, eine Pyrrolidinyl-, Piperidinyl- oder Morpholinogruppe bilden.

**6.** Verbindung nach Anspruch 5, worin $R^{20}$ steht für $R^{20}$ steht für $(CH_2)_yX^1R^9$, worin y steht für 0 oder eine ganze Zahl von 1 bis 4, $X^1$ steht für O, S, $NHSO_2$, $SO_2NH$, $NR^{10}$, CO, COO, OCO, $CONR^{11}$, $NR^{12}CO$, $NR^{12}COCOO$, $OCONR^{13}$, $R^9$ steht für Wasserstoff, $(C_1-C_{10})$-Alkyl, Pyrrolidinyl, Tetrahydrofuryl, Morpholino oder $(C_3-C_8)$-Cycloalkyl und $R^{10}$, $R^{11}$, $R^{12}$ und $R^{13}$ jeweils unabhängig steht für Wasserstoff oder $(C_1-C_{10})$-Alkyl, oder $R^9$ und $R^{10}$, $R^{11}$, $R^{12}$ oder $R^{13}$ bilden zusammen mit dem Stickstoffatom, an das sie gebunden sind, eine Pyrrolidinyl-, Piperidinyl- oder Morpholinogruppe; und eine Gruppe der Formel $R^{14}-(L^a)_n-X^2-(L^b)_m$, in der $X^2$ steht für eine Bindung, O, NH, S, SO, $SO_2$, CO, CONH, NHCOO oder NHCO, $L^a$ und $L^b$ jeweils stehen für $(C_1-C_4)$-Alkylen, eines von n und m für 0 oder 1 steht, und das andere für 0 steht, und $R^{14}$ steht für eine Phenylgruppe, die unsubstituiert oder substituiert ist durch ein oder zwei von Halogen; $(C_1-C_{10})$-Alkyl, Halogen$(C_1-C_{10})$-alkyl; Phenyl; $(CH_2)_zX^3R^{15}$, worin z für 0 oder eine ganze Zahl von 1 bis 4 steht, $X^3$ steht für O, S, $NR^{16}$, CO, COO, OCO, $CONR^{17}$, $NR^{18}CO$, $NHSO_2$, $SO_2NH$, $NHSO_2NR^{17}$, NHCONH, $OCONR^{19}$, N(CO$(C_1-C_4)$-Alkyl)CO oder $NR^{19}COO$, $R^{15}$ steht für Wasserstoff, $(C_1-C_{10})$-Alkyl, Phenyl$(C_1-C_4)$-alkyl, Halogen$(C_1-C_{10})$-alkyl, $(C_1-C_4)$-Alkoxycarbonyl$(C_1-C_4)$-alkyl, $(C_1-C_4)$-Alkylsulfonylamino$(C_1-C_4)$-alkyl, $(C_1-C_4)$-Alkylaminosulfonyl$(C_1-C_4)$-alkyl, (N-$(C_1-C_4)$-Alkoxycarbonyl)$(C_1-C_4)$-alkylsulfonylamino$(C_1-C_4)$-alkyl oder eine aromatische oder heteroaromatische Gruppe, die unsubstituiert oder substituiert ist durch ein oder zwei von Halogen, $(C_1-C_4)$-Alkyl, Halogen$(C_1-C_4)$-alkyl, Di$(C_1-C_4)$-alkylamino und $(C_1-C_4)$-Alkoxy, und $R^{16}$, $R^{17}$, $R^{18}$ und $R^{19}$ jeweils unabhängig stehen für Wasserstoff oder $(C_1-C_{10})$-Alkyl, oder $R^{15}$ und $R^{16}$, $R^{17}$, $R^{18}$ oder $R^{19}$ zusammen mit dem Stickstoffatom, an das sie gebunden sind, eine Pyrrolidinyl-, Piperidinyl- oder Morpholinogruppe bilden.

**7.** Verbindung gemäß Anspruch 6, worin $R^{20}$ steht für $R^{14}-(L^a)_n-X^2-(L^b)_{m-}$

**8.** Verbindung gemäß Anspruch 7, worin $(L^a)_n-X^2-(L^b)_m$ steht für eine Bindung, CONH oder $CH_2O$.

**9.** Verbindung gemäß Anspruch 8, worin $R^{14}$ steht für ein Phenyl, das substituiert ist durch ein oder zwei von Fluor; Chlor; Cyano; $(C_1-C_4)$-Alkyl; Trifluormethyl und $(CH_2)_zX^3R^{15}$, worin z steht für 0 oder 2, $X^3$ steht für $NR^{16}$, CO, COO, $CONR^{17}$, $NR^{18}CO$, $NHSO_2$, $SO_2NH$, $R^{15}$ steht für Wasserstoff, $(C_1-C_4)$-Alkyl, Phenyl$(C_1-C_4)$-alkyl oder Halogen$(C_1-C_4)$-alkyl, und $R^{16}$, $R^{17}$, $R^{18}$ und $R^{19}$ jeweils unabhängig stehen für Wasserstoff oder $(C_1-C_4)$-Alkyl.

**10.** Verbindung gemäß Anspruch 1, die ausgewählt ist aus der Gruppe, bestehend aus:

[2-(4-Hydroxyphenyl)cyclohex-2-enyl][(methylethyl)sulfonyl]amin;
(2-{4-[(3,5-Difluorphenyl)methoxy]phenyl}cyclohex-2-enyl)[(methylethyl)sulfonyl]amin;
2-{[4-(6-{[(Methylethyl)sulfonyl]amino}cyclohex-1-enyl)phenoxy]methyl}benzolcarbonitril;

[(Methylethyl)sulfonyl]{2-[4-(phenylmethoxy)phenyl]cyclopent-2-enyl}amin;
(2-{4-[(3,5-Difluorphenyl)methoxy]phenyl}cyclopent-2-enyl)[(methylethyl)sulfonyl]amin;
[2-(4-Chlorphenyl)cyclopent-2-enyl][(methylethyl)sulfonyl]amin; und
[2-(3,5-Difluorphenyl)cyclopent-2-enyl][(methylethyl)sulfonyl]amin;

oder ein pharmazeutisch annehmbares Salz davon.

**11.** Pharmazeutische Zusammensetzung, die eine Verbindung wie in einem der Ansprüche 1 bis 10 beansprucht und ein pharmazeutisch annehmbares Verdünnungsmittel oder einen pharmazeutisch annehmbaren Träger umfast.

**12.** Verbindung gemäß einem der Ansprüche 1 bis 10, oder ein pharmazeutisch annehmbares Salz davon, zur Verwendung als Arzneimittel.

**13.** Verwendung einer Verbindung gemäß einem der Ansprüche 1 bis 10, oder eines pharmazeutisch annehmbaren Salzes davon zur Herstellung eines Arzneimittels zur Potenzierung der Glutamatrezeptorfunktion.

**14.** Verwendung einer Verbindung gemäß einem der Ansprüche 1 bis 10 zur Herstellung eines Arzneimittels zur Behandlung einer kognitiven Störung; Alzheimer Krankheit; altersabhängigen Demenz; altersinduzierten Gedächtnisbeeinträchtigung; Depression, Aufmerksamkeitsdefizitstörung; Aufmerksamkeitsdefizithyperaktivitätsstörung; Psychose; kognitiver Defizite, die in Verbindung stehen mit einer Psychose; einer Arzneimittel-induzierten Psychose, Parkinson Krankheit oder eines Schlaganfalls.

## Revendications

**1.** Composé répondant à la formule

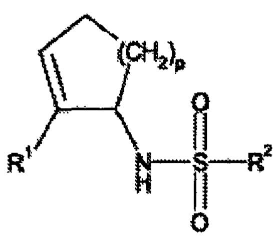

dans laquelle
p représente le nombre entier 1 ou 2 ;
$R^1$ représente un groupe aromatique non substitué ou substitué ou un groupe hétéroaromatique non substitué ou substitué ; et
$R^2$ représente un groupe alkyle en $C_1$-$C_6$ ; un groupe cycloalkyle en $C_3$-$C_6$ ; un groupe fluoroalkyle en $C_1$-$C_6$ ; un groupe chloroalkyle en $C_1$-$C_6$ ; un groupe alcényle en $C_2$-$C_6$ ; un groupe phényle qui est non substitué ou substitué par un atome d'halogène, par un groupe alkyle en $C_1$-$C_4$ ou par un groupe alcoxy en $C_1$-$C_4$ ; ou représente un groupe répondant à la formule $R^3R^4N$ dans laquelle $R^3$ et $R^4$ représentent, chacun indépendamment l'un de l'autre, un groupe alkyle en $C_1$-$C_4$ ou bien forment ensemble avec l'atome d'azote auquel ils sont fixés un groupe azétidinyle, un groupe pyrrolidinyle, un groupe pipéridinyle, un groupe morpholino, un groupe pipérazinyle, un groupe hexahydroazépinyle ou un groupe octahydroazocinyle ;
ou un de ses sels pharmaceutiquement acceptables.

**2.** Composé selon la revendication 1, dans lequel $R^2$ représente un groupe alkyle en $C_1$-$C_6$.

**3.** Composé selon la revendication 2, dans lequel $R^2$ représente un groupe 2-propyle.

**4.** Composé selon l'une quelconque des revendications 1 à 3, dans lequel $R^1$ représente un groupe :

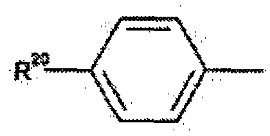

dans lequel

$R^{20}$ représente un atome d'halogène ; un groupe nitro ; un groupe cyano ; un groupe hydroxyimino ; un groupe alkyle en $C_1$-$C_{10}$ ; un groupe alcényle en $C_2$-$C_{10}$ ; un groupe alcynyle en $C_2$-$C_{10}$ ; un groupe cycloalkyle en $C_3$-$C_8$ ; un groupe hydroxycycloalkyle en $C_3$-$C_8$ ; un groupe oxocycloalkyle en $C_3$-$C_8$ ; un groupe halogénoalkyle en $C_1$-$C_{10}$ ; un groupe $(CH_2)_yX^1R^9$ dans lequel y est égal à 0 ou représente un entier de 1 à 4, $X^1$ représente un atome d'oxygène, un atome de soufre, un groupe $NHSO_2$, un groupe $SO_2NH$, un groupe $NR^{10}$, un groupe CO, un groupe COO, un groupe OCO, un groupe $CONR^{11}$, un groupe $NR^{12}CO$, un groupe $NR^{12}COCOO$, un groupe $OCONR^{13}$, $R^9$ représente un atome d'hydrogène, un groupe alkyle en $C_1$-$C_{10}$, un groupe alcényle en $C_3$-$C_{10}$, un groupe alcynyle en $C_3$-$C_{10}$, un groupe pyrrolidinyle, un groupe tétrahydrofuryle, un groupe morpholino ou un groupe cycloalkyle en $C_3$-$C_8$ et $R^{10}$, $R^{11}$, $R^{12}$ et $R^{13}$ représentent, chacun indépendamment l'un de l'autre, un atome d'hydrogène ou un groupe alkyle en $C_1$-$C_{10}$, ou bien $R^9$ et $R^{10}$, $R^{11}$, $R^{12}$ ou $R^{13}$, forment ensemble avec l'atome d'azote auquel ils sont fixés, un groupe azétidinyle, un groupe pyrrolidinyle, un groupe pipéridinyle ou un groupe morpholino ; un groupe N-alkyl(en $C_1$-$C_4$)pipérazinyle; un groupe N-phénylalkyl(en $C_1$-$C_4$)pipérazinyle ; un groupe thiényle ; un groupe furyle ; un groupe oxazolyle ; un groupe isoxazolyle ; un groupe pyrazolyle ; un groupe imidazolyle ; un groupe thiazolyle ; un groupe tétrazolyle ; un groupe pyridyle ; un groupe pyridazinyle ; un groupe pyrimidinyle ; un groupe dihydrothiényle ; un groupe dihydrofuryle ; un groupe dihydrothiopyranyle ; un groupe dihydropyranyle ; un groupe dihydrothiazolyle ; un groupe alcoxy(en $C_1$-$C_4$)carbonyldihydrothiazolyle ; un groupe alcoxy(en $C_1$-$C_4$)carbonyldiméthyldihydrothiazolyle ; un groupe tétrahydrothiényle ; un groupe tétrahydrofuryle ; un groupe tétrahydrothiopyranyle ; un groupe tétrahydropyranyle ; un groupe indolyle ; un groupe benzofuryle ; un groupe benzothiényle ; un groupe benzimidazolyle ; un groupe benzothiazolyle ; et un groupe répondant à la formule $R^{14}$-$(L^a)_n$-$X_2$-$(L^b)_m$ dans laquelle $X^2$ représente une liaison, un atome d'oxygène, un groupe NH, un atome de soufre, un groupe SO, un groupe $SO_2$, un groupe CO, un groupe CH(OH), un groupe CONH, un groupe NHCONH, un groupe NHCOO, un groupe COCONH, un groupe $OCH_2CONH$ ou un groupe CH=CH, un groupe NHCO, $L^a$ et $L^b$ représentent chacun un groupe alkylène en $C_1$-$C_4$, un des indices n et m est égal à 0 ou 1, l'autre étant égal à 0, et $R^{14}$ représente un groupe phényle ou un groupe hétéroaromatique qui est non substitué ou substitué par un ou deux membres choisis parmi le groupe comprenant un atome d'halogène ; un groupe nitro ; un groupe cyano ; un groupe alkyle en $C_1$-$C_{10}$; un groupe alcényle en $C_2$-$C_{10}$; un groupe alcynyle en $C_2$-$C_{10}$; un groupe cycloalkyle en $C_3$-$C_8$; un groupe 4-(1,1-dioxotétrahydro-1,2-thiazinyle) ; un groupe halogénoalkyle en $C_1$-$C_{10}$; un groupe cyanoalcényle en $C_2$-$C_{10}$; un groupe phényle ; un groupe $(CH_2)_zX^3R^{15}$ dans lequel z est égal à 0 ou représente un entier de 1 à 4, $X^3$ représente un atome d'oxygène, un atome de soufre, un groupe $NR^{16}$, un groupe CO, un groupe CH(OH), un groupe COO, un groupe OCO, un groupe $CONR^{17}$, un groupe $NR^{18}CO$, un groupe $NHSO_2$, un groupe $SO_2NH$, un groupe $NHSO_2NR^{17}$, un groupe NHCONH, un groupe $OCONR^{19}$, un groupe N(CO (alkyl en $C_1$-$C_4$)CO, ou un groupe $NR^{19}COO$, $R^{15}$ représente un atome d'hydrogène, un groupe alkyle en $C_1$-$C_{10}$, un groupe phénylalkyle en $C_1$-$C_4$, un groupe halogénoalkyle en $C_1$-$C_{10}$, un groupe alcoxy(en $C_1$-$C_4$)carbonylalkyle en $C_1$-$C_4$, un groupe alkyl(en $C_1$-$C_4$)sulfonylaminoalkyle en $C_1$-$C_4$, un groupe alkyl(en $C_1$-$C_4$)aminosulfonylalkyle en $C_1$-$C_4$, un groupe (N-alcoxy(en $C_1$-$C_4$)carbonyl)alkyl(en $C_1$-$C_4$)sulfonylaminoalkyle en $C_1$-$C_4$, un groupe alcényle en $C_3$-$C_{10}$, un groupe alcynyle en $C_3$-$C_{10}$, un groupe cycloalkyle en $C_3$-$C_8$, un groupe camphoryle ou encore un groupe aromatique ou un groupe hétéroaromatique qui est non substitué ou substitué par un ou deux membres choisis parmi le groupe comprenant un atome d'halogène, un groupe alkyle en $C_1$-$C_4$, un groupe halogénoalkyle en $C_1$-$C_4$, un groupe di(alkyl en $C_1$-$C_4$)amino et un groupe alcoxy en $C_1$-$C_4$ et $R^{16}$, $R^{17}$, $R^{18}$ et $R^{19}$ représentent, chacun indépendamment l'un de l'autre, un atome d'hydrogène ou un groupe alkyle en $C_1$-$C_{10}$, ou bien $R^{15}$ et $R^{16}$, $R^{17}$, $R^{18}$ ou $R^{19}$, forment ensemble avec l'atome d'azote auquel ils sont fixés, un groupe azétidinyle, un groupe pyrrolidinyle, un groupe pipéridinyle ou un groupe morpholino.

**5.** Composé selon la revendication 4, dans lequel $R^{20}$ représente un atome d'halogène ; un groupe alkyle en $C_1$-$C_{10}$ ; un groupe halogénoalkyle en $C_1$-$C_{10}$ ; un groupe $(CH_2)_yX^1R^9$ dans lequel y est égal à 0 ou représente un entier de 1 à 4, $X^1$ représente un atome d'oxygène, un atome de soufre, un groupe $NHSO_2$, un groupe $SO_2NH$, un groupe $NR^{10}$, un groupe CO, un groupe COO, un groupe OCO, un groupe $CONR^{11}$, un groupe $NR^{12}CO$, un groupe $NR^{12}COCOO$, un groupe $OCONR^{13}$, $R^9$ représente un atome d'hydrogène, un groupe alkyle en $C_1$-$C_{10}$, un groupe alcényle en $C_3$-$C_{10}$, un groupe alcynyle en $C_3$-$C_{10}$, un groupe pyrrolidinyle, un groupe tétrahydrofuryle, un groupe morpholino ou un groupe cycloalkyle en $C_3$-$C_8$ et $R^{10}$, $R^{11}$, $R^{12}$ et $R^{13}$ représentent, chacun indépendamment l'un de l'autre, un atome d'hydrogène ou un groupe alkyle en $C_1$-$C_{10}$, ou bien $R^9$ et $R^{10}$, $R^{11}$, $R^{12}$ ou $R^{13}$, forment

ensemble avec l'atome d'azote auquel ils sont fixés, un groupe pyrrolidinyle, un groupe pipéridinyle ou un groupe morpholino ; et un groupe répondant à la formule $R^{14}$-$(L^a)_n$-$X_2$-$(L^b)_m$ dans laquelle $X^2$ représente une liaison, un atome d'oxygène, un groupe NH, un atome de soufre, un groupe SO, un groupe $SO_2$, un groupe CO, un groupe CH(OH), un groupe CONH, un groupe NHCONH, un groupe NHCOO, un groupe COCONH, un groupe $OCH_2CONH$ ou un groupe CH=CH, un groupe NHCO, $L^a$ et $L^b$ représentent chacun un groupe alkylène en $C_1$-$C_4$, un des indices n et m est égal à 0 ou 1, l'autre étant égal à 0, et $R^{14}$ représente un groupe phényle qui est non substitué ou substitué par un ou deux membres choisis parmi le groupe comprenant un atome d'halogène ; un groupe nitro ; un groupe cyano ; un groupe alkyle en $C_1$-$C_{10}$; un groupe halogénoalkyle en $C_1$-$C_{10}$ ; un groupe phényle ; un groupe $(CH_2)_zX^3R^{15}$ dans lequel z est égal à 0 ou représente un entier de 1 à 4, $X^3$ représente un atome d'oxygène, un atome de soufre, un groupe $NR^{16}$, un groupe CO, un groupe CH(OH), un groupe COO, un groupe OCO, un groupe $CONR^{17}$, un groupe $NR^{18}CO$, un groupe $NHSO_2$, un groupe $SO_2NH$, un groupe $NHSO_2NR^{17}$, un groupe NHCONH, un groupe $OCONR^{19}$, un groupe N(CO(alkyl en $C_1$-$C_4$)CO, ou un groupe $NR^{19}COO$, $R^{15}$ représente un atome d'hydrogène, un groupe alkyle en $C_1$-$C_{10}$, un groupe phénylalkyle en $C_1$-$C_4$, un groupe halogénoalkyle en $C_1$-$C_{10}$, un groupe alcoxy(en $C_1$-$C_4$)carbonylalkyle en $C_1$-$C_4$, un groupe alkyl(en $C_1$-$C_4$)sulfonylaminoalkyle en $C_1$-$C_4$, un groupe alkyl(en $C_1$-$C_4$)aminosulfonylalkyle en $C_1$-$C_4$, un groupe (N-alcoxy (en $C_1$-$C_4$)carbonyl)alkyl(en $C_1$-$C_4$)sulfonylaminoalkyle en $C_1$-$C_4$, ou encore un groupe aromatique ou un groupe hétéroaromatique qui est non substitué ou substitué par un ou deux membres choisis parmi le groupe comprenant un atome d'halogène, un groupe alkyle en $C_1$-$C_4$, un groupe halogénoalkyle en $C_1$-$C_4$, un groupe di(alkyl en $C_1$-$C_4$) amino et un groupe alcoxy en $C_1$-$C_4$ et $R^{16}$, $R^{17}$, $R^{18}$ et $R^{19}$ représentent, chacun indépendamment l'un de l'autre, un atome d'hydrogène ou un groupe alkyle en $C_1$-$C_{10}$, ou bien $R^{15}$ et $R^{16}$, $R^{17}$, $R^{18}$ ou $R^{19}$, forment ensemble avec l'atome d'azote auquel ils sont fixés, un groupe pyrrolidinyle, un groupe pipéridinyle ou un groupe morpholino.

6. Composé selon la revendication 5, dans lequel $R^{20}$ représente un groupe $(CH_2)_yX^1R^9$ dans lequel y est égal à 0 ou représente un entier de 1 à 4, $X^1$ représente un atome d'oxygène, un atome de soufre, un groupe $NHSO_2$, un groupe $SO_2NH$, un groupe $NR^{10}$, un groupe CO, un groupe COO, un groupe OCO, un groupe $CONR^{11}$, un groupe $NR^{12}CO$, un groupe $NR^{12}COCOO$, un groupe $OCONR^{13}$, $R^9$ représente un atome d'hydrogène, un groupe alkyle en $C_1$-$C_{10}$, un groupe pyrrolidinyle, un groupe tétrahydrofuryle, un groupe morpholino ou un groupe cycloalkyle en $C_3$-$C_8$ et $R^{10}$, $R^{11}$, $R^{12}$ et $R^{13}$ représentent, chacun indépendamment l'un de l'autre, un atome d'hydrogène ou un groupe alkyle en $C_1$-$C_{10}$, ou bien $R^9$ et $R^{10}$, $R^{11}$, $R^{12}$ ou $R^{13}$, forment ensemble avec l'atome d'azote auquel ils sont fixés, un groupe pyrrolidinyle, un groupe pipéridinyle ou un groupe morpholino ; et un groupe répondant à la formule $R^{14}$-$(L^a)_n$-$X_2$-$(L^b)_m$ dans laquelle $X^2$ représente une liaison, un atome d'oxygène, un groupe NH, un atome de soufre, un groupe SO, un groupe $SO_2$, un groupe CO, un groupe CONH, un groupe NHCOO ou un groupe NHCO, $L^a$ et $L^b$ représentent chacun un groupe alkylène en $C_1$-$C_4$, un des indices n et m est égal à 0 ou 1, l'autre étant égal à 0, et $R^{14}$ représente un groupe phényle qui est non substitué ou substitué par un ou deux membres choisis parmi le groupe comprenant un atome d'halogène ; un groupe alkyle en $C_1$-$C_{10}$; un groupe halogénoalkyle en $C_1$-$C_{10}$; un groupe phényle ; un groupe $(CH_2)_zX^3R^{15}$ dans lequel z est égal à 0 ou représente un entier de 1 à 4, $X^3$ représente un atome d'oxygène, un atome de soufre, un groupe $NR^{16}$, un groupe CO, un groupe COO, un groupe OCO, un groupe $CONR^{17}$, un groupe $NR^{18}CO$, un groupe $NHSO_2$, un groupe $SO_2NH$, un groupe $NHSO_2NR^{17}$, un groupe NHCONH, un groupe $OCONR^{19}$, un groupe N(CO(alkyl en $C_1$-$C_4$)CO ou un groupe $NR^{19}COO$, $R^{15}$ représente un atome d'hydrogène, un groupe alkyle en $C_1$-$C_{10}$, un groupe phénylalkyle en $C_1$-$C_4$, un groupe halogénoalkyle en $C_1$-$C_{10}$, un groupe alcoxy(en $C_1$-$C_4$)carbonylalkyle en $C_1$-$C_4$, un groupe alkyl (en $C_1$-$C_4$)sulfonylaminoalkyle en $C_1$-$C_4$, un groupe alkyl(en $C_1$-$C_4$)aminosulfonylalkyle en $C_1$-$C_4$, un groupe (N-alcoxy(en $C_1$-$C_4$)carbonyl)alkyl(en $C_1$-$C_4$)sulfonylaminoalkyle en $C_1$-$C_4$, ou encore un groupe aromatique ou un groupe hétéroaromatique qui est non substitué ou substitué par un ou deux membres choisis parmi le groupe comprenant un atome d'halogène, un groupe alkyle en $C_1$-$C_4$, un groupe halogénoalkyle en $C_1$-$C_4$, un groupe di (alkyl en $C_1$-$C_4$)amino et un groupe alcoxy en $C_1$-$C_4$, et $R^{16}$, $R^{17}$, $R^{18}$ et $R^{19}$ représentent, chacun indépendamment l'un de l'autre, un atome d'hydrogène ou un groupe alkyle en $C_1$-$C_{10}$, ou bien $R^{15}$ et $R^{16}$, $R^{17}$, $R^{18}$ ou $R^{19}$, forment ensemble avec l'atome d'azote auquel ils sont fixés, un groupe pyrrolidinyle, un groupe pipéridinyle ou un groupe morpholino.

7. Composé selon la revendication 6, dans lequel $R^{20}$ représente un groupe $R^{14}$-$(L^a)_n$-$X_2$-$(L^b)_m$.

8. Composé selon la revendication 7, dans lequel le groupe $(L^a)_n$-$X_2$-$(L^b)_m$ représente une liaison, un groupe CONH ou un groupe $CH_2O$.

9. Composé selon la revendication 8, dans lequel $R^{14}$ représente un groupe phényle qui est substitué par un ou deux membres choisis parmi le groupe comprenant un groupe fluoro ; un groupe chloro ; un groupe cyano ; un groupe alkyle en $C_1$-$C_4$; un groupe trifluorométhyle ; et un groupe $(CH_2)_zX^3R^{15}$ dans lequel z est égal à 0 ou 2, $X^3$ repré-

sente un groupe $NR^{16}$, un groupe CO, un groupe COO, un groupe $CONR^{17}$, un groupe $NR^{18}CO$, un groupe $NHSO_2$, un groupe $SO_2NH$, $R^{15}$ représente un atome d'hydrogène, un groupe alkyle en $C_1$-$C_4$, un groupe phénylalkyle en $C_1$-$C_4$ ou un groupe halogénoalkyle en $C_1$-$C_4$, et $R^{16}$, $R^{17}$, $R^{18}$ et $R^{19}$ représentent, chacun indépendamment l'un de l'autre, un atome d'hydrogène ou un groupe alkyle en $C_1$-$C_4$.

**10.** Composé selon la revendication 1, qui est choisi parmi le groupe constitué par :

la [2-(4-hydroxyphényl)cyclohex-2-ényl][(méthyléthyl)sulfonyl]amine ;
la (2-{4-[(3,5-difluorophényl)méthoxy]phényl}cyclohex-2-ényl)[(méthyléthyl)sulfonyl]amine ;
le 2-{[4-(6-{[(méthyléthyl)sulfonyl]amino}cyclohex-1-ényl)phénoxy]méthyl}benzènecarbonitrile ;
la [(méthyléthyl)sulfonyl]{2-[4-(phénylméthoxy)phényl]cyclopent-2-ényl}amine ;
la (2-{4-[(3,5-difluorophényl)méthoxy]phényl}cyclopent-2-ényl)[(méthyléthyl)sulfonyl]amine ;
la [2-(4-chlorophényl)cyclopent-2-ényl][(méthyléthyl)sulfonyl]amine ; et
la [2-(3,5-difluorophényl)cyclopent-2-ényl][(méthyléthyl)sulfonyl]amine ;

ou un de ses sels pharmaceutiquement acceptables.

**11.** Composition pharmaceutique qui comprend un composé tel que revendiqué dans l'une quelconque des revendications 1 à 10 et un diluant ou un support pharmaceutiquement acceptable.

**12.** Composé selon l'une quelconque des revendications 1 à 10, ou un de ses sels pharmaceutiquement acceptables, à utiliser comme produit pharmaceutique.

**13.** Utilisation d'un composé selon l'une quelconque des revendications 1 à 10 ou d'un de ses sels pharmaceutiquement acceptables pour la préparation d'un médicament destiné à la potentialisation de la fonction du récepteur du glutamate.

**14.** Utilisation d'un composé selon l'une quelconque des revendications 1 à 10 pour la préparation d'un médicament destiné à traiter un trouble cognitif ; la maladie d'Alzheimer ; la démence liée à l'âge ; un déficit de la mémoire induit par l'âge ; la dépression ; des troubles déficitaires de l'attention ; des troubles d'hyperactivité liés à un déficit de l'attention ; des psychoses ; des déficits cognitifs associés à des psychoses ; des psychoses provoquées par des médicaments ; la maladie de Parkinson ; ou un accident vasculaire cérébral.